# EUROPEAN PATENT APPLICATION

(11) **EP 4 410 241 A1**
(43) Date of publication of application: **07.08.2024**
(21) Application number: 22874920.6
(22) Date of filing: 27.09.2022
(51) Int. Cl.: A61F 2/24

(54) **ARTIFICAL HEART VALVE STENT FOR ATRIOVENTRICULAR VALVE, INTERVENTIONAL INSTRUMENT, CONTROL HANDLE, CONVEYING SYSTEM, LOADING METHOD AND RELEASE METHOD**

(30) Priority: 29.09.2021 CN 202111150318
(71) Applicant: Venus MedTech (HangZhou) Inc., Binjiang District Hangzhou Zhejiang 310052 (CN)
(72) Inventor: ZHU, Lingke, Hangzhou, Zhejiang 310052 (CN); ZHANG, Jiaojiao, Hangzhou, Zhejiang 310052 (CN); CHEN, Yixun, Hangzhou, Zhejiang 310052 (CN)
(74) Representative: Zenz Patentanwälte Partnerschaft mbB
(86) International application number: PCT/CN2022/121769
(87) International publication number: WO 2023/051535

(57) **Abstract**

A prosthetic heart valve stent for atrioventricular valve, an interventional device (900), a control handle (100), a delivery system, a loading method and a release method are disclosed. The interventional device (900) includes an inner frame (300) and an outer frame (400) around the inner frame (300), the inner frame (300) and the outer frame (400) both have radially deformable and cylindrical mesh structures, the inner frame (300) encloses and defines a blood flow channel (301), and the prosthetic heart valve stent has an inflow side (910) and an outflow side (920) opposite to each other according to a direction of blood flow; the prosthetic heart valve stent further includes an anchoring arm (500), one end of the anchoring arm (500) being connected to the inner frame (300), the other end extending outward beyond the outer frame (400), and the part extending beyond the outer frame (400) being bent towards the inflow side (910) and used for positioning and engaging with a surrounding tissue. The interventional device (900) is engaged with the surrounding tissue by using the inner frame (300) and outer frame (400), increasing use stability of the interventional device (900).

## Description

### TECHNICAL FIELD

The present disclosure relates to the field of medical devices, and in particular to prosthetic heart valve stents for atrioventricular valve, interventional devices, control handles, delivery systems, loading methods and release methods.

### DESCRIPTION OF THE PRIOR ART

Human heart valves, including aortic valve, pulmonary valve, mitral valve, and tricuspid valve, essentially function as one-way valves that operate in synchrony with the beating heart. Mechanical and tissue-based heart valve prostheses can be used to replace damaged native heart valves. These replacement valves need to replace the native valves as perfectly as possible, and need to at least allow one-way blood flow, avoid perivalvular leakage, and be fixedly engaged with the surrounding tissue.

Some existing interventional devices (prosthetic valve devices) are positioned through barbs. In other words, the stent of the prosthetic valve device is connected with barbs, and the ends of the barbs will pierce the surrounding tissue for positioning, which results in relatively high safety risks.

Due to the limited atrial space and the irregular movement of the heart during systole and diastole, especially the irregular cross section of the heart where the atrioventricular valve is located, while most of the blood flow channels defined by the leaflets of the prosthetic valve devices have circular cross sections, it is necessary for the prosthetic valve devices to be able to deform to adapt to the surrounding tissue while maintaining the shape of the blood flow channels in the prosthetic valve devices, so as to control the blood flow effectively.

During the intervention process, the spatial positions and special structures of the mitral and tricuspid valves also make it more difficult to position and adjust the posture of the interventional devices, and the control operations become more complicated.

### SUMMARY OF THE DISCLOSURE

In order to solve the above technical problems, the present disclosure discloses a control handle for operating an interventional assembly, the interventional assembly including a delivery tube for loading an interventional device and a bending component for changing an orientation of a distal end of the delivery tube, the control handle having an axial direction in space, two ends of the control handle in the axial direction being distal and proximal ends respectively. The control handle includes:
two support bodies arranged sequentially in the axial direction, and the two support bodies being slidably engaged with each other in the axial direction;
a plurality of mounting seats installed on the corresponding support bodies fixedly or movably, wherein a mounting seat(s) on one support body is configured to connect the bending component, and a mounting seat(s) on the other support body is configured to connect the delivery tube; and
a plurality of driving members movably installed on the corresponding support bodies, which are engaged with and configured to drive the corresponding mounting seats on the support bodies.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

Optionally, the two support bodies include a first support body located at the distal end of the control handle and a second support body located at the proximal end of the control handle.

Optionally, a third support body is movably connected to the distal end of the first support body.

Optionally, the first support body and the third support body are movably connected to each other in a slide fit in the axial direction of the control handle or by a detachable connection.

Optionally, the mounting seats on the support bodies are configured as a fixed seat(s) and a movable seat(s) depending on movement relationships of the mounting seats relative to the support bodies, and all movable seats on the two support bodies are respectively provided with corresponding driving members.

Optionally, the mounting seats on the support bodies include at least one fixed seat and at least one movable seat.

Optionally, the mounting seats on the support bodies include a fixed seat and two movable seats.

Optionally, a fixed seat and a movable seat are provided on the third support body, and the movable seat is provided with a driving member.

Optionally, the bending component includes a tube(s) or a pull wire(s).

Optionally, one or more groups of bending components are provided, with each group consisting of two bending components, and wherein distal ends of the two bending components are relatively fixed, and proximal ends of the two bending components are respectively coupled with corresponding mounting seats and movable relative to each other.

Optionally, two groups of bending components are provided, with each group consisting of two bending components, and wherein the bending components in different groups are inserted one in the other, and distal ends thereof are movable relative to each other.

Optionally, two groups of bending components are provided, with one group connected to the first support body, and the other group connected to the third support body.

Optionally, the bending component includes an outer bending tube, a middle bending tube and an inner bending tube arranged sequentially from outside to inside, which are relatively fixed adjacent to a distal portion of the bending component, and proximal ends of which are respectively coupled to the corresponding mounting seats and movable relative to each other.

Optionally, the delivery tube includes an outer sheath, a support tube and an inner core tube arranged sequentially from outside to inside, proximal ends of which are respectively coupled with the corresponding mounting seats and movable relative to each other.

Optionally, the bending component includes two bending tubes arranged sequentially from outside to inside, the mounting seats on the first support body includes a fixed seat and a movable seat, distal ends of the two bending tubes are relatively fixed, and proximal ends thereof are respectively connected to the fixed seat and the movable seat.

Optionally, the bending component includes a pull wire and three bending tubes arranged sequentially from outside to inside.

The mounting seats on the first support body includes a fixed seat and a movable seat, wherein one of two inner bending tubes of the three bending tubes is connected to the fixed seat and the other is connected to the movable seat.

The mounting seats on the third support body includes a fixed seat and a movable seat, wherein an outermost bending tube of the three bending tubes is connected to the fixed seat, and the pull wire is connected to the movable seat.

Optionally, the bending component includes four bending tubes arranged sequentially from outside to inside, with two outer bending tubes defined as one group and two inner bending tubes defined as another group, the mounting seats on the first support body include two fixed seats and two movable seats, and wherein one of the two bending tubes in the same group is connected to a corresponding fixed seat and the other is connected to a corresponding movable seat.

Optionally, the mounting seats on the first support body include a first fixed seat, a first movable seat and a second movable seat arranged from the distal end to the proximal end, which are respectively connected to the outer bending tube, the middle bending tube and the inner bending tube.

Optionally, the mounting seats on the second support body include a third movable seat, a second fixed seat and a fourth movable seat arranged from the distal end to the proximal end, which are respectively connected to the outer sheath, the support tube and the inner core tube.

Optionally, a transmission member is connected to the third movable seat, the transmission member extends distally until exceeding the first support body, the exceeding part is provided with a coupling seat, and a proximal end of the outer sheath is fixed to the coupling seat.

Optionally, a connection slot is opened on the transmission member, and at least a part of the third movable seat passes through the connection slot so that the transmission member is allowed to move with the second support body.

Optionally, in the axial direction of the control handle, a coupling area is remained between the first support body and the third support body, a transmission member is connected to the third movable seat, the transmission member extends distally beyond the first support body until the coupling area, a distal end of the transmission member is provided with a coupling seat located in the coupling area, and a proximal end of the outer sheath is fixed to the coupling seat.

Optionally, outer peripheries of the support bodies are fixed with respective housings, and the transmission member extends in a radial gap between the support bodies and the housings.

Optionally, the transmission member extends on two sides of the support bodies opposite to each other in a radial direction.

Optionally, a transmission member is connected to a mounting seat on the second support body, the transmission member extends distally until exceeding the first support body, and the exceeding part is provided with a coupling seat for connecting the delivery tube.

Optionally, the driving members are cylindrical and are rotatably arranged around outer peripheries of the corresponding support bodies, the driving members are threadedly engaged with the corresponding mounting seats, and the support bodies are provided with slide grooves for guiding the corresponding mounting seats to move linearly.

Optionally, at least parts of the mounting seats are exposed to the corresponding support bodies, the exposed parts are provided with male threads, and inner peripheries of the driving members are provided with female threads engaging with the corresponding male threads.

Optionally, a rack and gear transmission mechanism(s) is arranged between the driving members and the mounting seats. For example, the rack is fixed on the mounting seat and extends in the axial direction of the support body, and the gear is fixed on the driving member or is engaged with and configured to be driven by the driving member. For another example, the driving member is configured to directly drive the mounting seat. That is, the driving member is directly fixed to the mounting seat, and when the operator applies force to the driving member, the driving member directly acts on the mounting seat.

Optionally, a connecting sleeve is rotatably mounted on the proximal end of the first support body, and a distal end of the second support body extends into the connecting sleeve and is threadedly engaged with the connecting sleeve.

Optionally, a distal periphery of the second support body is provided with transmission teeth, and the transmission teeth are threadedly engaged with a female thread of the connecting sleeve.

Optionally, a limiting mechanism is provided between the first support body and the second support body to prevent them from rotating relative to each other.

Optionally, the limiting mechanism is connected to one of the first support body and the second support body, and extends to and acts on the other.

Optionally, both the first support body and the second support body are slidably engaged with the limiting mechanism.

Optionally, the limiting mechanism is the transmission member, and the transmission member and the first support body are prevented from rotating relative to each other by:
a transmission groove being provided on the transmission member, the support body being partially or entirely located in the transmission groove, and in a radial direction of the support body, groove walls of the transmission groove clamping the support body from two opposite sides;

The transmission member and the second support body are prevented from rotating relative to each other by:
in a circumferential direction of the support body, two opposite side groove walls of the connection slot abutting against the third movable seat.

Optionally, all mounting seats on the second support body are located at the proximal end of the transmission tooth.

Optionally, the control handle for operating an interventional assembly further includes a synchronization mechanism acting between two adjacent driving members, wherein the synchronization mechanism includes:
first synchronization teeth on the driving members; and
a synchronization lock ring being movable in the axial direction of the control handle, the synchronization lock ring being provided with second synchronization teeth, and the synchronization lock ring having relative synchronization and release positions in the axial direction of the control handle.

At the synchronization position, the second synchronization teeth mesh with the first synchronization teeth on two driving members to link the two driving members; and

At the release position, the second synchronizing teeth mesh with the first synchronizing tooth on at most one driving member to release the linkage.

Optionally, the first synchronization teeth are distributed on outer peripheries of the corresponding driving members.

Optionally, the synchronization lock ring is slidably arranged around one of the driving members, with a guide mechanism provided therebetween for guiding the synchronization lock ring to move in the axial direction of the control handle.

Optionally, the control handle for operating an interventional assembly further includes an anti-rotation mechanism, and the anti-rotation mechanism includes:
a first latching tooth provided on a corresponding driving member; and
an anti-rotation lock ring being movable in the axial direction of the control handle, the anti-rotation lock ring being provided with second latching teeth, and the anti-rotation lock ring having relative locked and unlocked positions in the axial direction of the control handle

At the locked position, the first latching tooth is engaged with the second latching teeth to prevent the corresponding driving member from rotating.

At the unlocked position, the first latching tooth is disengaged from the second latching teeth to allow the corresponding driving member to rotate.

Optionally, the first latching tooth is distributed on an inner periphery of the corresponding driving member.

Optionally, the anti-rotation lock ring is slidably arranged around a corresponding support body, with a guide mechanism provided therebetween for guiding the anti-rotation lock ring to move in the axial direction of the control handle.

Optionally, the mounting seat includes a sealing member connected to a proximal end of the delivery tube or the bending component, and a housing that engages with a corresponding support body, and wherein the sealing member is disposed inside the housing.

Optionally, the housing includes a cover plate and a hollow body, and an outer periphery of the hollow body is provided with a male thread that is engaged with a female thread of a corresponding driving member.

Optionally, a limiting mechanism is provided between the cover plate and the hollow body to prevent them from moving relative to each other, and the limiting mechanism includes a positioning block provided on one of the cover plate and the hollow body and a positioning groove provided on the other and matching the positioning block.

Optionally, the cover plate is provided with a mounting base for installing the sealing member, the hollow body is provided with a mounting groove for accommodating the sealing member, and in an assembled state, the mounting base and the mounting groove clamp and fix the sealing member, and prevent the sealing member from moving in its own axial and radial directions.

Optionally, a middle part of the sealing member is provided with an annular step, the mounting base and the mounting groove have an annular protrusion that matches the annular step in the assembled state, and a side wall of the annular protrusion abuts against the sealing member.

Optionally, the sealing member includes a connecting tube for connecting the delivery tube or the bending component, and sealing plugs respectively provided at two ends of the connecting tube for sealing.

Optionally, the control handle includes an exhaust joint connected to one of the mounting seats on the first support body.

Optionally, the exhaust joint is connected to the fixed seat.

The present disclosure further discloses a control handle for operating an interventional assembly, the interventional assembly being configured to control an interventional device, the control handle having an axial direction in space, two ends of the control handle in the axial direction being distal and proximal ends respectively, wherein in the axial direction, the control handle includes a distal part, a main part and a proximal part arranged sequentially from the distal end to the proximal end.

The components of the interventional assembly are divided into three groups which are respectively connected to and controlled by corresponding parts of the control handle.

Two adjacent parts of the control handle are configured to be coupled to each other through a locking mechanism, and to drive the connected components of the interventional assembly to move relative to each other after being decoupled.

The present disclosure further discloses a delivery system, including an interventional assembly and the control handle for operating the interventional assembly.

Optionally, the interventional assembly includes a plurality of tubes distributed sequentially from outside to inside, and wherein one of two tubes is an outer tube and the other is an inner tube, the outer tube is connected to the second support body, and the inner tube is connected to the first support body.

Optionally, the interventional assembly includes at least three tubes distributed sequentially from outside to inside, and wherein outermost and innermost tubes are both connected to the second support body, and at least one middle tube is connected to the first support body.

Optionally, the interventional assembly includes a plurality of tubes distributed sequentially from outside to inside, at least one tube of the delivery tube is located outside the bending component, and the proximal end of the delivery tube is located at a distal end of the first support body and is connected to the second support body after bypassing the first support body through a transmission member.

Optionally, the interventional assembly includes six tubes distributed sequentially from outside to inside: an outer sheath, an outer bending tube, a middle bending tube, an inner bending tube, a support tube and an inner core tube; the delivery tube includes the outer sheath, the support tube and the inner core tube; the bending component includes the outer bending tube, the middle bending tube and the inner bending tube; and wherein the bending component is connected to the first support body, and the delivery tube is connected to the second support body.

Optionally, the interventional assembly includes an outer sheath, a first bending tube, a second bending tube, a third bending tube, a fourth bending tube, a support tube and an inner core tube distributed sequentially from outside to inside; the delivery tube includes the outer sheath, the support tube and the inner core tube; the bending component includes the first bending tube, the second bending tube, the third bending tube and the fourth bending tube; and wherein the bending component is connected to the first support body, and the delivery tube is connected to the second support body.

Optionally, the interventional assembly includes an outer sheath, a first bending mechanism, a second bending mechanism, a support tube and an inner core tube distributed sequentially from outside to inside; the delivery tube includes the outer sheath, the support tube and the inner core tube; the bending component includes the bending mechanisms; and wherein the bending component is connected to the first support body, and the delivery tube is connected to the second support body.

Optionally, the interventional assembly includes a third bending mechanism, an outer sheath, a fourth bending mechanism, a support tube and an inner core tube distributed sequentially from outside to inside; the delivery tube includes the outer sheath, the support tube and the inner core tube; the bending component includes the bending mechanisms; and wherein the third bending mechanism is connected to the third support body, the fourth bending mechanism is connected to the first support body, and the delivery tube is connected to the second support body.

Optional, each bending mechanism includes:
two bending tubes inserted one in the other, or
a bending tube and a pull wire, the pull wire being located inside or outside the corresponding bending tube.

Optionally, distal ends of the bending mechanisms are movable relative to each other, and distal ends of the bending mechanisms and the delivery tube are movable relative to each other.

Optionally, the support tube is provided with a mounting head for engaging with the interventional device, and the mounting head is provided with a chamfered structure at a distal edge thereof.

Optionally, a distal end of the outer sheath is provided with a first loading section with an increased diameter.

The distal end of the inner core tube exceeds the distal end of the outer sheath, and the exceeding part is provided with a second loading section with an increased diameter (relative to the other part of itself); the first loading section and the second loading section are opened towards each other and contact with each other to form a loading space for accommodating the interventional device.

Optional, the distal ends of the outer bending tube, middle bending tube and inner bending tube are fixed by:
the distal ends of the middle bending tube and the inner bending tube being fixedly connected to each other and movably engaged with the support tube;
the distal end of the outer bending tube being fixedly connected adjacent to the distal end of the middle bending tube.

The delivery system and control handle disclosed in the present disclosure can control the bending and position adjustment of the interventional assembly in the human body and the release of the interventional device.

The present disclosure further discloses a prosthetic heart valve stent for atrioventricular valve, which has opposite inflow and outflow sides depending on a direction of blood flow, wherein the prosthetic heart valve stent includes an inner frame and an outer frame located around the inner frame, the inner frame and the outer frame both have radially deformable and cylindrical mesh structures, and the inner frame encloses and defines a blood flow channel.

The inner frame or the outer frame is further connected with anchor arms, and an opening is formed by the anchor arms or between the anchor arms and the outer frame to accommodate a surrounding tissue for positioning and engaging with the surrounding tissue.

Optionally, the opening is opened radially outward or toward the inflow side.

Optionally, the anchor arm has a fixed end connected to the inner frame or outer frame and an opposing free end, and the free end has a rounded structure and/or is wrapped with a cushioning.

The present disclosure further discloses a prosthetic heart valve stent for atrioventricular valve, which has opposite inflow and outflow sides depending on a direction of blood flow, wherein the prosthetic heart valve stent includes an inner frame and an outer frame located around the inner frame, the inner frame and the outer frame both have radially deformable and cylindrical mesh structures, and the inner frame encloses and defines a blood flow channel.

The prosthetic heart valve stent further includes anchor arms for positioning and engaging with a surrounding tissue, each anchor arm has a fixed end connected to the inner frame or the outer frame and an opposing free end, the anchor arms are distributed in a circumference direction, all the anchor arms are connected to each other at the fixed ends thereof to form an annular structure as a whole and are connected to the inner frame or the outer frame through the annular structure.

The present disclosure further discloses a prosthetic heart valve stent for atrioventricular valve, which includes an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further includes anchor arms, one end of each anchor arm is connected to the inner frame, the other end extends outward and exceeds the outer frame, and the exceeding part beyond the outer frame is bent toward the inflow side for positioning and engaging with a surrounding tissue.

Optionally, each anchor arm extends outward and exceeds the outer frame by extending outward through a structural gap of the outer frame, and/or by extending outward from the outflow side of the outer frame and bypassing the outer frame.

The outflow side of the outer frame is provided with a plurality of semi-closed structural gaps which are opened toward the outflow side, and each anchor arm extends outward and exceeds the outer frame through a corresponding semi-closed structural gap.

The present disclosure further discloses a prosthetic heart valve stent for atrioventricular valve, which includes an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further includes anchor arms which are arranged in pairs and are connected with the outer frame, the anchor arms in the same pair are relatively arranged in an axial direction of the outer frame, and a clamping opening is defined between the anchor arms in the same pair that faces radially outward for positioning and engaging with a surrounding tissue.

Optionally, one of the anchor arms in the same pair on the inflow side is an upper arm, the other on the outflow side is a lower arm, and the upper arm and the lower arm each have a single strut or multi-strut structure.

Optionally, the inflow side of the outer frame has positioning structures extending radially outward, and the positioning structures also serve as the upper arms.

Optionally, each anchor arm has a fixed end connected to the inner frame or the outer frame and an opposing free end, and the fixed ends of the upper arm and the lower arm are adjacent to or axially spaced apart from each other.

Optionally, the clamping opening has an opening angle Σ at least in a range of 60 degrees < Σ < 120 degrees.

Optionally, the clamping opening has an axial span M2 in a range of 10 < M2 < 30 mm.

The present disclosure further discloses a prosthetic heart valve stent for atrioventricular valve, which includes an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further includes anchor arms, one end of each anchor arm is connected to the outflow side of the outer frame, and the other end extends outside the outer frame and toward the inflow side for positioning and engaging with a surrounding tissue.

The present disclosure further discloses a prosthetic heart valve stent for atrioventricular valve, which has opposite inflow and outflow sides depending on a direction of blood flow, wherein the prosthetic heart valve stent includes an inner frame and an outer frame located around the inner frame, the inner frame and the outer frame both have radially deformable and cylindrical mesh structures, and the inner frame and the outer frame are formed in separate pieces which are connected to each other at a connection portion(s), and the connection portion(s) is flexible.

The present disclosure further provides a prosthetic heart valve stent for atrioventricular valve, which has opposite inflow and outflow sides depending on a direction of blood flow, wherein the prosthetic heart valve stent includes an inner frame and an outer frame located around the inner frame, the inner frame and the outer frame both have radially deformable and cylindrical mesh structures, the inner frame encloses and defines a blood flow channel, and the outer frame is configured to support an inner side of a native valve annulus and native valve leaflets.

A radial spacing is defined between the inner frame and the outer frame which are connected to each other through a fixing ear(s) spanning the radial spacing.

Optionally, the inner frame defines a first circumferential surface, the outer frame defines a second circumferential surface, and the first circumferential surface and the second circumferential surface do not intersect with each other.

Optionally, the inflow side of the outer frame has positioning structures extending radially outward.

Optionally, an inflow edge of the positioning structure extends radially inward.

Optionally, an inflow edge of the positioning structure has a connecting ear for engaging with an interventional assembly.

Optionally, the angle between the connecting ear and the axis of the outer frame is α, and the angle α satisfies 0 degree ≤ α < 30 degrees. Preferably, 0 degree ≤ α < 15 degrees.

Optionally, the length of the connecting ear is L9, and L9 satisfies 3.5 mm < L9 < 6.5 mm.

Optionally, the positioning structures include a plurality of V-shaped struts arranged in a circumferential direction of the outer frame, a vertex of each V-shaped strut faces the inflow side, and two arms of each V-shaped strut are connected to a remaining part of the outer frame.

Optionally, the quantity of V-shaped struts is in a range of 6 to 12.

Optionally, adjacent V-shaped struts are connected to each other in the circumferential direction of the outer frame.

Optionally, in the circumferential direction of the outer frame, adjacent V-shaped struts are arranged at intervals, and wherein each interval has a circumferential width of M1 with a corresponding central angle of β, and the central angle β is in a range of 30 degrees to 60 degrees.

Optionally, a sum of central angles of all M1 in the circumferential direction of the outer frame is 360 degrees.

Optionally, in the circumferential direction of the outer frame, a release opening is defined between adjacent V-shaped struts for releasing connection therebetween, and wherein in a compressed state, the outer frame has a length of L1, the release opening has a length of L2, and L1 : L2 = X, wherein X is in a range of 2.0 to 3.0.

Optionally, in the circumferential direction of the outer frame, a release opening is defined between adjacent V-shaped struts for releasing connection therebetween, and wherein in a released state, the outer frame has a length of L3, the release opening has a length of L4, and L3 : L4 = Y, wherein Y is in a range of 1.5 to 2.5.

Optionally, the anchor arm and the inner frame or the outer frame are formed in one piece, or are formed in separate pieces that are fixedly connected to each other.

Optionally, the anchor arm and the inner frame or the outer frame are formed in separate pieces that are fixedly connected to each other, and the connection portion is rigid or flexible.

Optionally, the anchor arm and the inner frame or outer frame are fixed with a binding wire(s).

Optionally, a plurality of anchor arms are distributed in the circumferential direction, and the anchor arms are independent of each other or connected together to form an annular structure.

Optionally, each anchor arm has an open structure or encloses a closed space.

Optionally, each anchor arm is provided with one or more eyelets.

Optionally, at least one eyelet is provided at an end of each anchor arm.

Optionally, the periphery of the eyelet has a closed or semi-open structure.

Optionally, the anchor arm has a single-strut structure.

Optionally, the anchor arm has fixed ends connected to the inner frame or the outer frame and an opposing free end. The anchor arm has a multi-strut structure, and the multi-strut structure includes at least two struts. The fixed ends of the struts diverge from each other and the free ends thereof converge and fix each other.

Optionally, each anchor arm has a V shape and a vertex of the V shape is a free end.

Optionally, in a compressed state, the anchor arms are located on an axial side of the inner frame or the outer frame.

Optionally, during a switching process from a compressed state to a released state, a release timing of the inflow side and the outflow side is T, and a release timing of fixed ends and free ends of the anchor arms is T or -T.

Optionally, in a compressed state, the anchor arms contact a radial side of the inner frame or the outer frame.

Optionally, a connection portion(s) of the inner frame and the outer frame is located on the outflow side.

Optionally, the inner frame and the outer frame are formed in separate pieces that are fixedly connected to each other, and the connection portion is rigid or flexible.

Optionally, the outer frame has a radial stiffness less than that of the inner frame.

Optionally, the inner frame and the outer frame are bound and fixed by a flexible element(s).

Optionally, the inner frame and the outer frame are fixed with a binding wire(s).

Optionally, the inner frame and the outer frame are fixed to each other with a plurality of fixing ears distributed in a circumferential direction and arranged by at least one of:
the fixing ears and the inner frame being formed in one piece and the fixing ears being bent radially outward until being connected to the outer frame;
the fixing ears and the outer frame being formed in one piece and the fixing ears being bent radially inward until being connected to the inner frame; and
the fixing ears being divided into two groups which are formed in separate pieces with the inner frame and the outer frame respectively and are bent radially toward each other and connected to each other.

Optionally, the fixing ears are rod-shaped.

Optionally, the end of the fixing ear has a threading hole.

Optionally, the outflow side of the outer frame is radially flared.

Optionally, the outflow side of the outer frame is axially aligned with the outflow side of the inner frame.

The present disclosure further discloses an interventional device, including:
the prosthetic heart valve stent as mentioned above;
leaflets configured in the inner frame for control an opening degree of a blood flow channel; and
a sealing film connected in a radial gap between the inner and outer frames.

Optionally, the sealing film is located on the inflow side.

Optionally, the sealing film is loosely connected in the radial gap between the inner frame and the outer frame.

Optionally, the radial gap between the inner frame and the outer frame is open toward the outflow side.

Optionally, a first covering film is further included, which is connected to an inner side and/or an outer side of the inner frame.

Optionally, a second covering film is further included, which is connected to an inner side and/or an outer side of the outer frame.

Optionally, at least one of the first covering film and the second covering film is connected to the sealing film.

Optionally, both the inner frame and the outer frame have mesh structures.

Optionally, the outer frame includes one or more circles of cells in the axial direction, and the quantity of cells in each circle is in the range of 9 to 12.

Optionally, a circle of cells on the outflow side is a semi-closed structure, that is, the cells are open toward the outflow side.

The present disclosure further discloses a loading method for an interventional device, for fixing the interventional device to the above-mentioned interventional assembly. The loading method includes:
arranging the interventional device in an expanded state around an outer periphery of the support tube of the interventional assembly;
compressing the interventional device radially around the outer periphery of the support tube; and
driving the inner core tube and outer sheath to move until the interventional device is covered.

The present disclosure further discloses a release method of an interventional device, which is pre-fixed on the above-mentioned interventional assembly. The release method includes:
driving one of the inner core tube or the outer sheath to move so that at least part of anchor arms of the interventional device is exposed and released;
adjusting the anchor arms to the proper position to engage with a surrounding tissue; and
driving the other of the inner core tube and the outer sheath to move, so that the interventional device is completely exposed and switches to a released state.

The technical solution disclosed in the present disclosure can improve the stability of the interventional device in the human body.

Specific benefits will be further explained in combination with specific structures or steps in the specific embodiments.

### BRIEF DESCRIPTION OF DRAWINGS

FIG. 1 is a schematic view showing the state when a control handle according to the present disclosure is extended into the heart and an interventional device is covered;
FIG. 2 is a schematic view showing the interventional device in FIG. 1 after being exposed and expanded;
FIG. 3 is a schematic view of an interventional assembly in a control handle according to an embodiment of the present disclosure;
FIG. 4 is a schematic view showing the delivery tube in FIG. 3 being withdrawn as a whole;
FIG. 5 is a schematic view of the distal end of the delivery tube in the control handle according to an embodiment of the present disclosure;
FIG. 6 is a schematic view showing the inner core tube in FIG. 5 moving distally in the axial direction;
FIG. 7 is a schematic view showing the outer sheath in FIG. 6 moving proximally in the axial direction;
FIG. 8 is a perspective view of a control handle according to an embodiment of the present disclosure;
FIG. 9a is a cross-sectional view of a control handle according to an embodiment of the present disclosure;
FIG. 9b is a cross-sectional view showing a fourth driving member engaged with a transmission tooth in the control handle according to another embodiment of the present disclosure;
FIG. 9c is a schematic view showing the fourth driving member disengaged from the transmission tooth in FIG. 9b;
FIG. 10 is a schematic view showing the connection between the bending component and the support bodies in the control handle of FIG. 9a;
FIG. 11 is a schematic view showing the connection between the delivery tube and the support bodies in the control handle of FIG. 9a;
FIG. 12 is a schematic view showing the connection between the bending component and the support bodies in the control handle according to another embodiment of the present disclosure;
FIG. 13 is a schematic view showing the connection between the bending component and the support bodies in the control handle according to another embodiment of the present disclosure;
FIG. 14 is an exploded view of the driving member in FIG. 8;
FIG. 15 is a further exploded view of the support bodies in FIG. 14;
FIG. 16 is a partial view showing the connection among the first support body, the second support body and the transmission member in FIG. 14;
FIG. 17 is a schematic view showing the transmission member and the second support body moving axially relative to the first support body in FIG. 16;
FIG. 18 is a partial cross-sectional view showing the connection between the second support body and the transmission member;
FIG. 19 is a perspective view of the first support body in FIG. 14;
FIG. 20 is a perspective view of the second support body in FIG. 14;
FIG. 21 is a perspective view of the transmission member in FIG. 14;
FIG. 22 is a partial enlarged view showing the synchronization lock ring and anti-rotation lock ring in FIG. 14;
FIG. 23a is a structural view of the first driving member in FIG. 22;
FIG. 23b is a structural view of the synchronization lock ring in FIG. 22;
FIG. 23c is a structural view of the second driving member in FIG. 22;
FIG. 24a is a partial structural view of the first support body in FIG. 22;
FIG. 24b is a structural view of the anti-rotation lock ring in FIG. 22;
FIG. 24c is a structural view of the first driving member in FIG. 22 from another perspective;

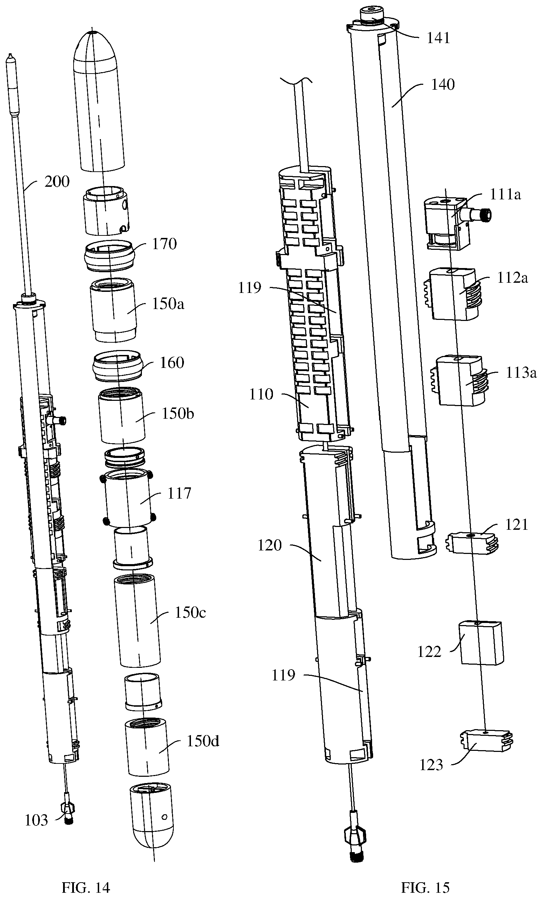

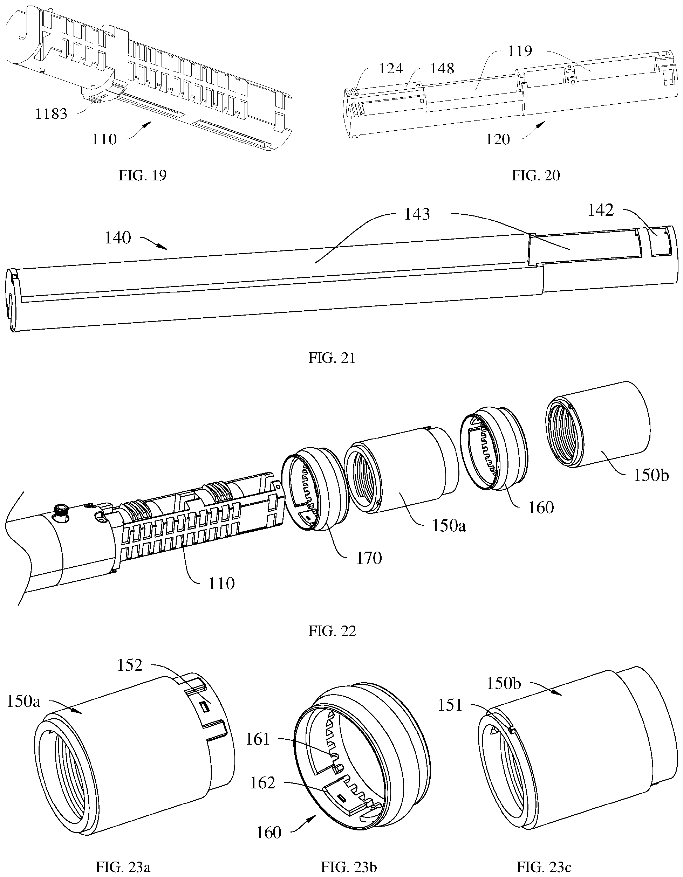

FIG. 23 is a perspective view of the driving member engaged with the synchronization lock ring in FIG. 14;

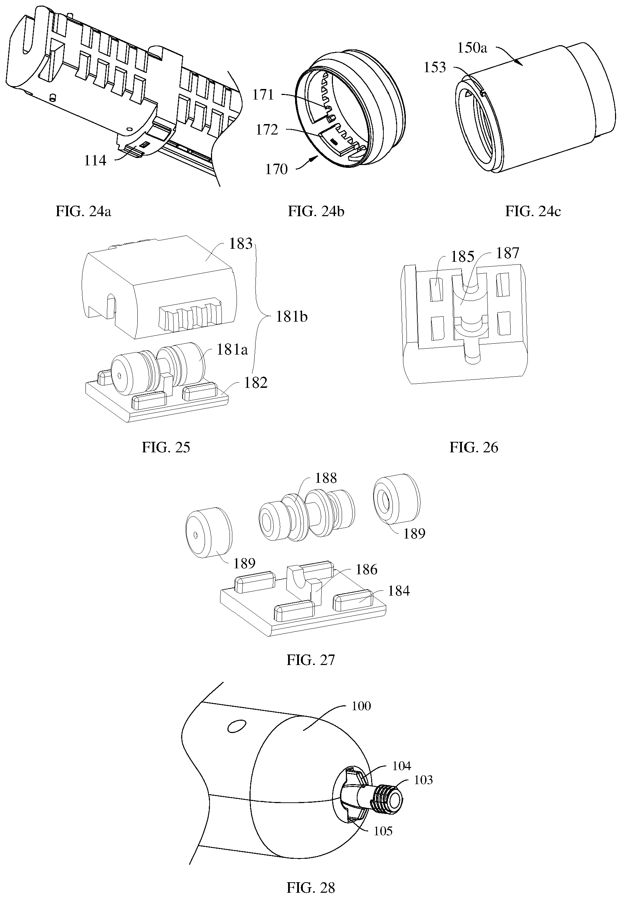

FIG. 24 is a perspective view of FIG. 23 from another perspective;
FIG. 25 is an exploded view of the mounting seat in FIG. 15;
FIG. 26 is a perspective view of the hollow body in FIG. 25;
FIG. 27 is a further exploded view of the mounting seat in FIG. 25;
FIG. 28 is a structural view showing the Luer connector in FIG. 14;

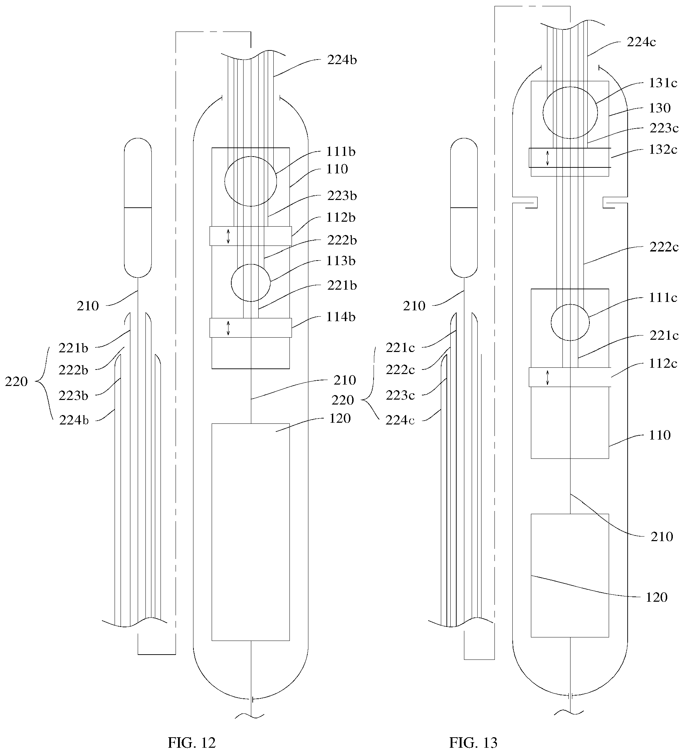

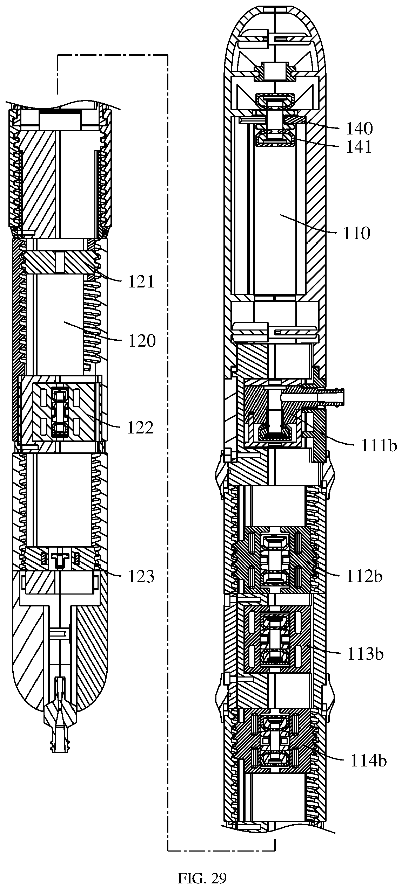

FIG. 29 is a structural cross-sectional view of the control handle shown in FIG. 12;

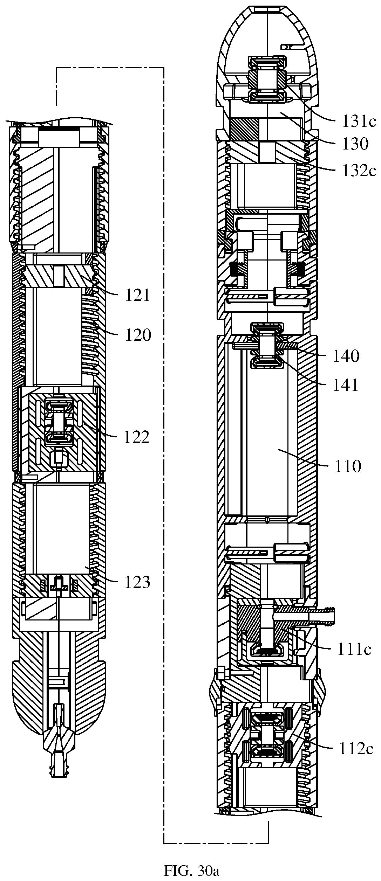

FIG. 30a is a structural cross-sectional view of the control handle shown in FIG. 13;

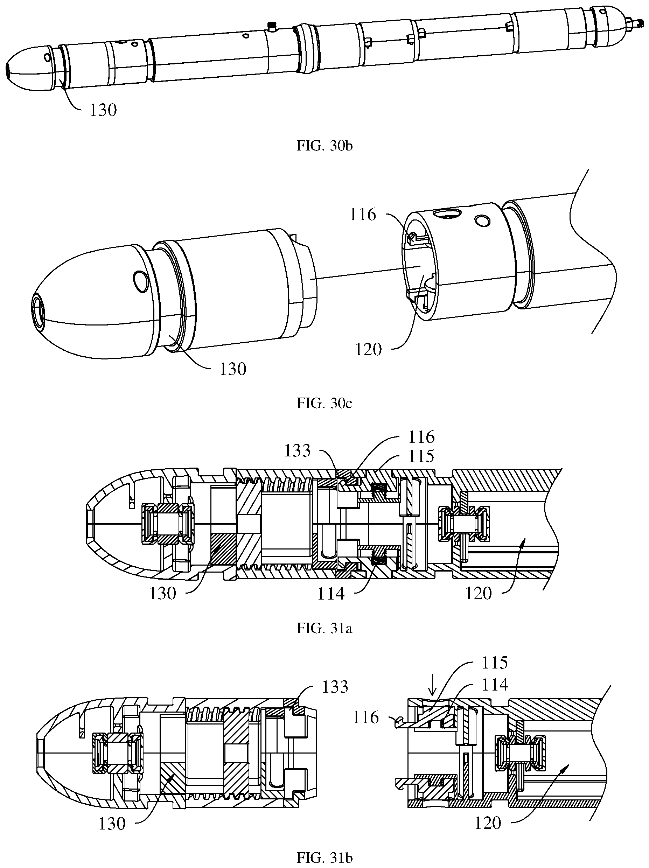

FIG. 30b is a perspective view of the control handle shown in FIG. 13;
FIG. 30c is a partial perspective view showing the first support body and the third support body in FIG. 30a separated from each other;
FIG. 31a is a partial perspective view of the third support body in FIG. 30b in the disassembled state;
FIG. 31a is a cross-sectional view of the third support body in FIG. 30b in the assembled state;
FIG. 31b is a cross-sectional view of the third support body in FIG. 30b in the disassembled state;

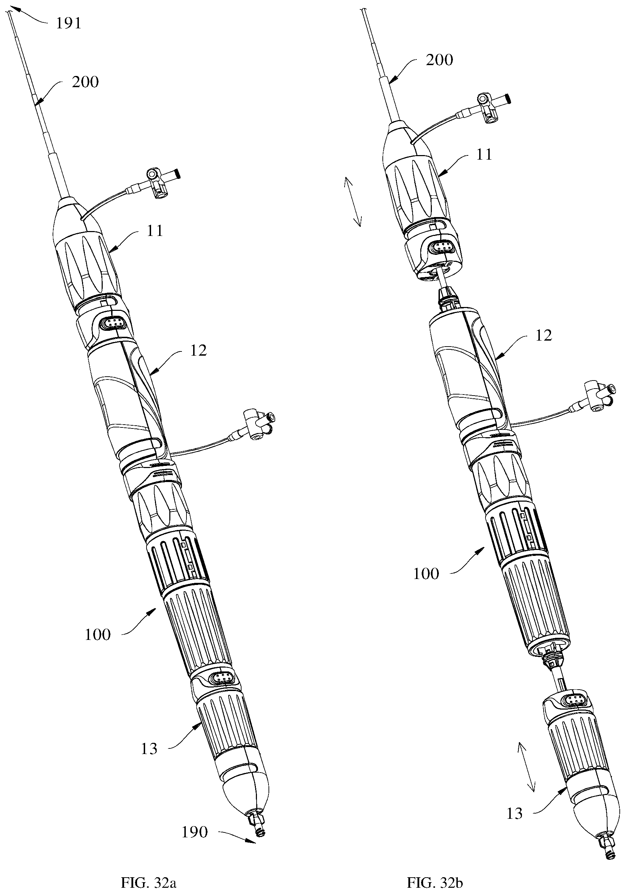

FIG. 32a is a perspective view of a control handle according to another embodiment of the present disclosure;
FIG. 32b is a perspective view showing the distal part and the proximal part sliding relative to the main part in FIG. 32a;

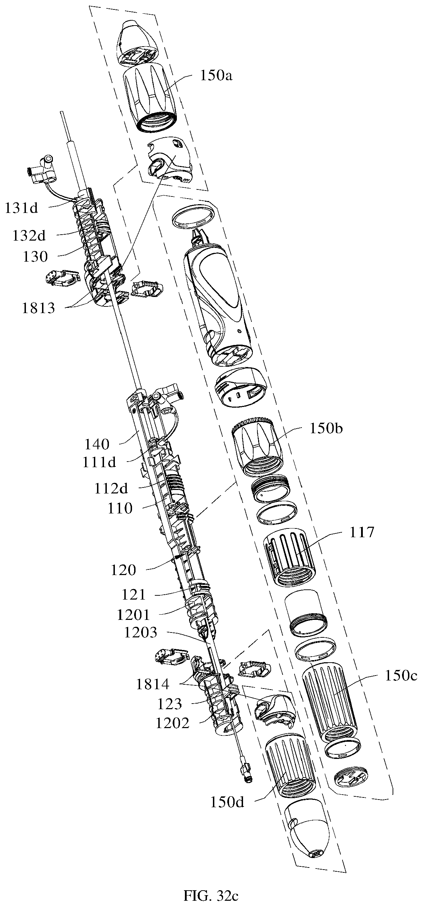

FIG. 32c is an exploded view of the control handle in FIG. 32b;

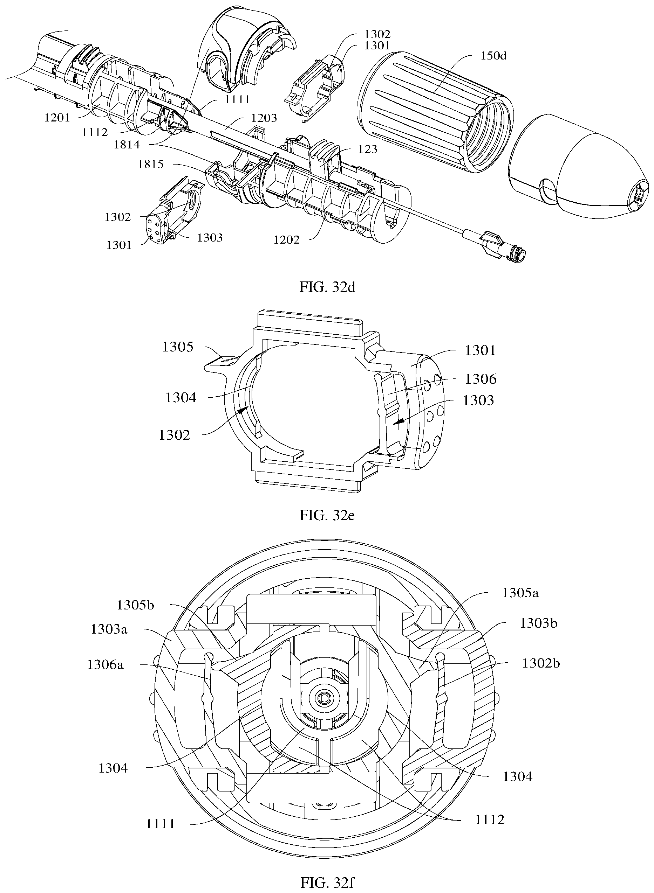

FIG. 32d is a partial enlarged view of the proximal part in FIG. 32c;
FIG. 32e is a perspective view of the limiting member and the control button in the control handle according to an embodiment of the present disclosure;
FIG. 32f is a cross-sectional view of the control handle at the limiting member according to an embodiment of the present disclosure;

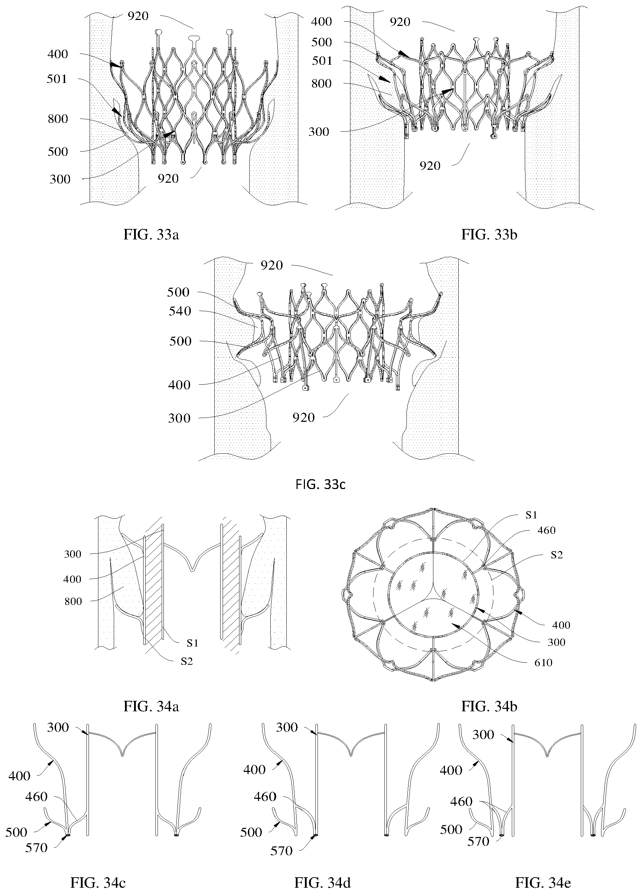

FIG. 33a is a schematic view showing an interventional device according to an embodiment of the present disclosure adapted to the surrounding tissue in the human body;
FIG. 33b is a schematic view showing an interventional device according to another embodiment of the present disclosure adapted to the surrounding tissue in the human body;
FIG. 33c is a schematic view showing an interventional device according to another embodiment of the present disclosure adapted to the surrounding tissue in the human body;
FIG. 34a is a schematic view showing an interventional device according to an embodiment of the present disclosure adapted to the surrounding tissue in the human body;
FIG. 34b is a schematic view of the interventional device in FIG. 34a in the human body from a top view;
FIG. 34c is a schematic view of an interventional device according to an embodiment of the present disclosure;
FIG. 34d is a schematic view of an interventional device according to another embodiment of the present disclosure;
FIG. 34e is a schematic view of an interventional device according to another embodiment of the present disclosure;

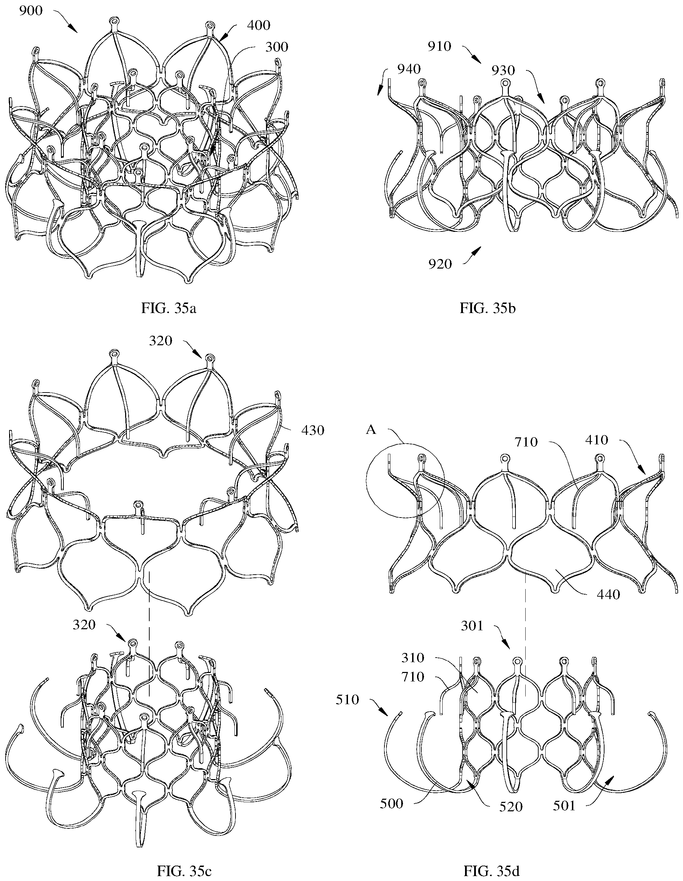

FIG. 35a is a structural view of an interventional device according to an embodiment of the present disclosure;
FIG. 35b is a front view of the interventional device in FIG. 35 a;
FIG. 35c is an exploded view of the interventional device in FIG. 35a;
FIG. 35d is an exploded view of the interventional device in FIG. 35b;

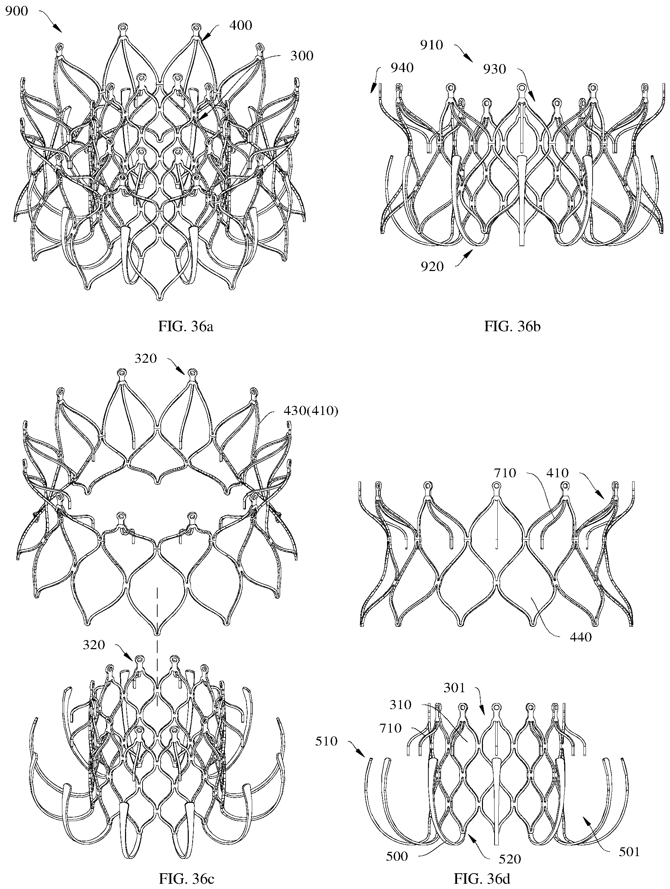

FIG. 36a is a structural view of an interventional device according to another embodiment of the present disclosure;
FIG. 36b is a front view of the interventional device in FIG. 36a;
FIG. 36c is an exploded view of the interventional device in FIG. 36a;
FIG. 36d is an exploded view of the interventional device in FIG. 36b;

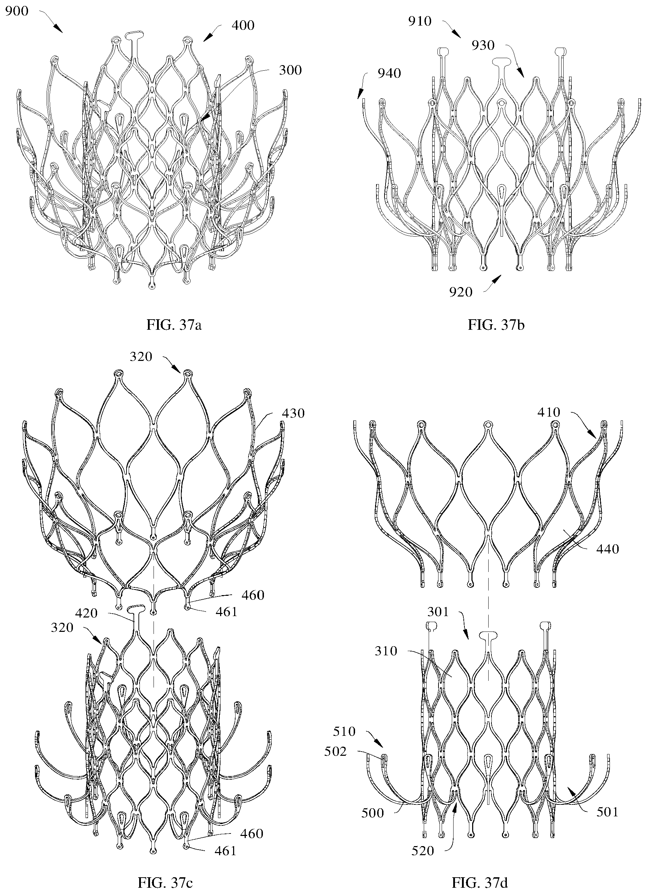

FIG. 37a is a structural view of an interventional device according to another embodiment of the present disclosure;
FIG. 37b is a front view of the interventional device in FIG. 37a;
FIG. 37c is an exploded view of the interventional device in FIG. 37a;
FIG. 37d is an exploded view of the interventional device in FIG. 37b;

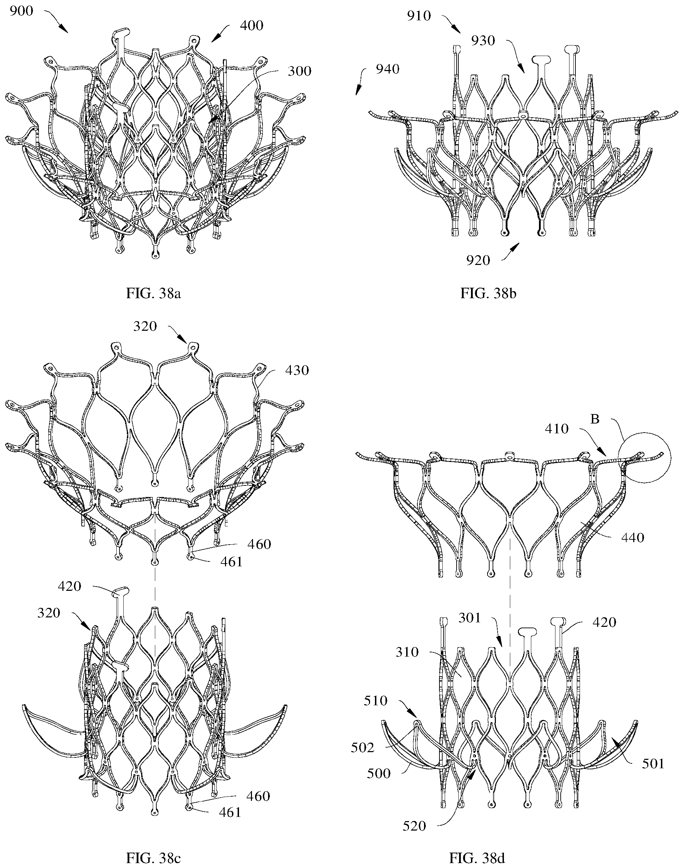

FIG. 38a is a structural view of an interventional device according to another embodiment of the present disclosure;
FIG. 38b is a front view of the interventional device in FIG. 38a;
FIG. 38c is an exploded view of the interventional device in FIG. 38a;
FIG. 38d is an exploded view of the interventional device in FIG. 38b;

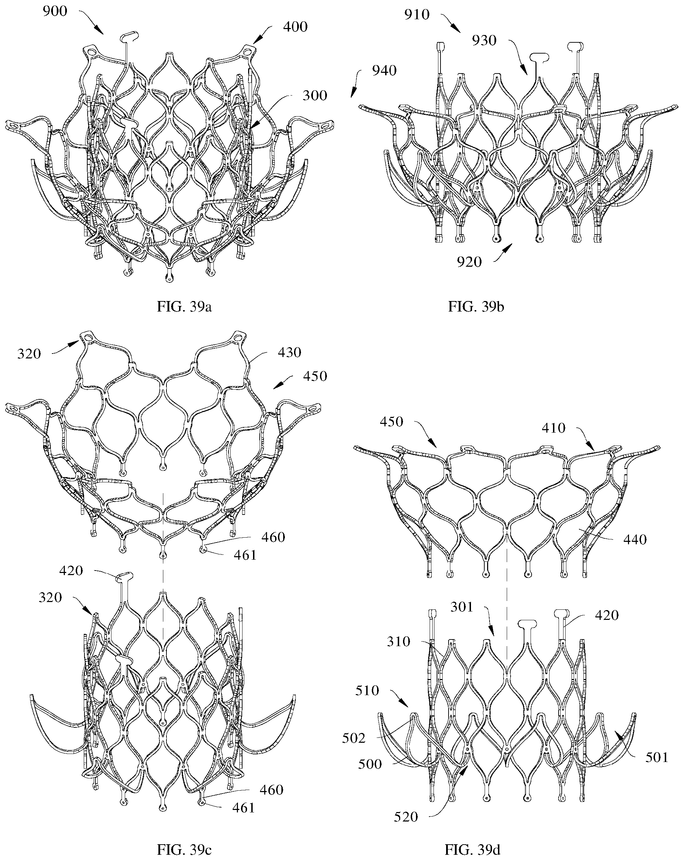

FIG. 39a is a structural view of an interventional device according to another embodiment of the present disclosure;
FIG. 39b is a front view of the interventional device in FIG. 39a;
FIG. 39c is an exploded view of the interventional device in FIG. 39a;
FIG. 39d is an exploded view of the interventional device in FIG. 39b;

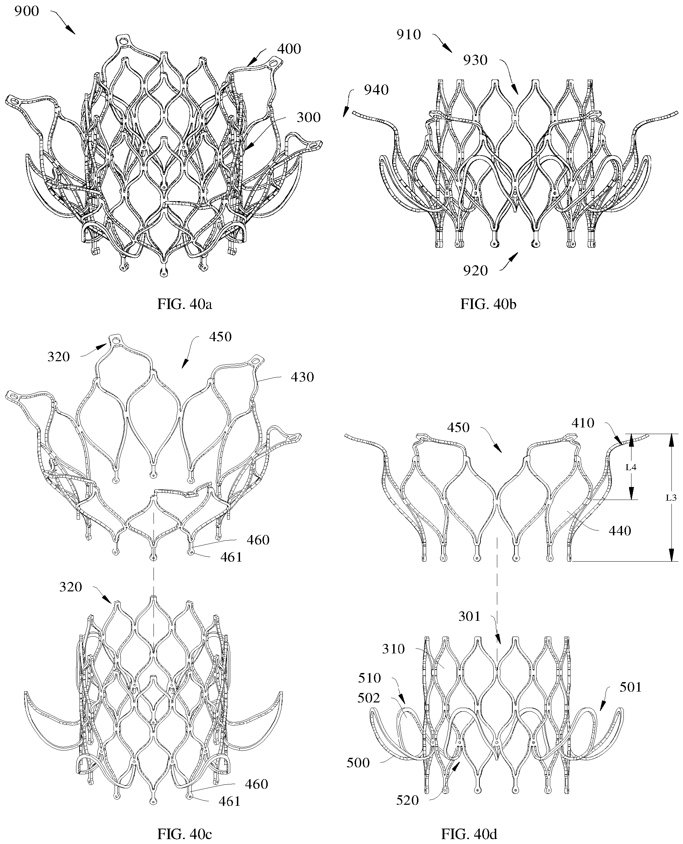

FIG. 40a is a structural view of an interventional device according to another embodiment of the present disclosure;
FIG. 40b is a front view of the interventional device in FIG. 40a;
FIG. 40c is an exploded view of the interventional device in FIG. 40a;
FIG. 40d is an exploded view of the interventional device in FIG. 40b;

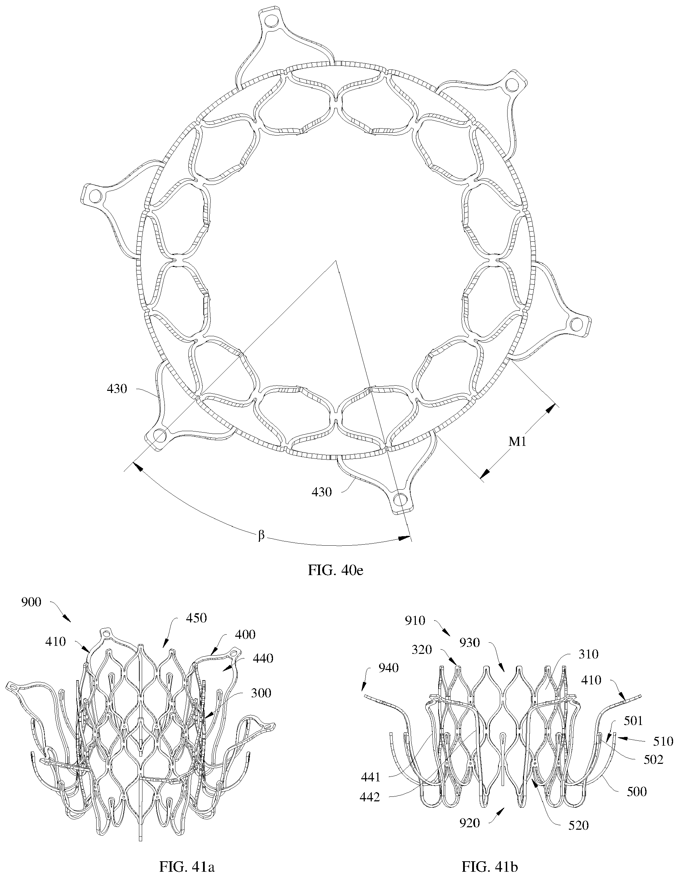

FIG. 40e is a top view of the interventional device in FIG. 40d;
FIG. 41a is a structural view of an interventional device according to another embodiment of the present disclosure.
FIG. 41b is the front view of the interventional device in FIG. 41a;

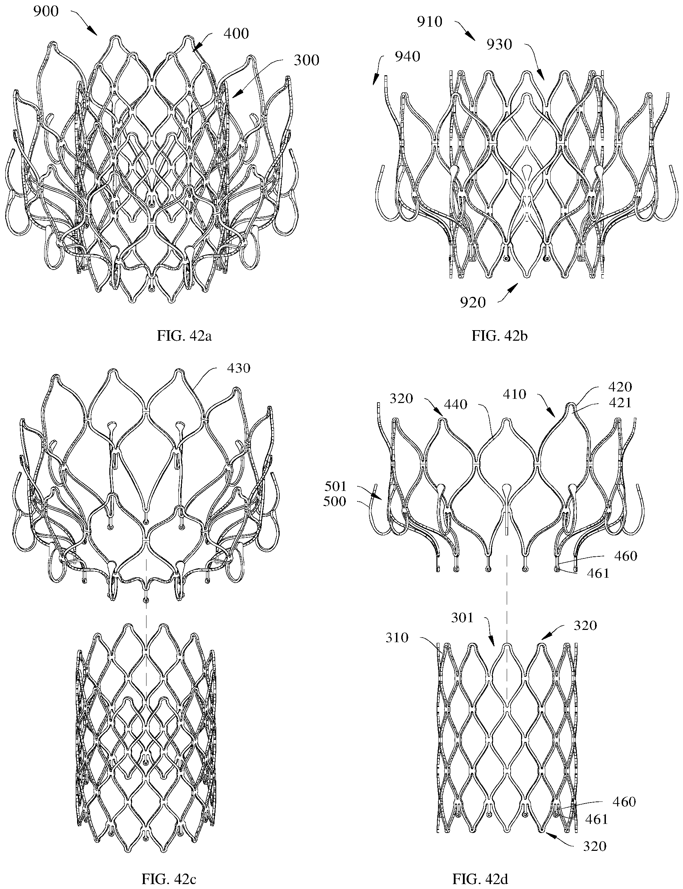

FIG. 42a is a structural view of an interventional device according to another embodiment of the present disclosure;
FIG. 42b is a front view of the interventional device in FIG. 42a;
FIG. 42c is an exploded view of the interventional device in FIG. 42a;
FIG. 42d is an exploded view of the interventional device in FIG. 42b;

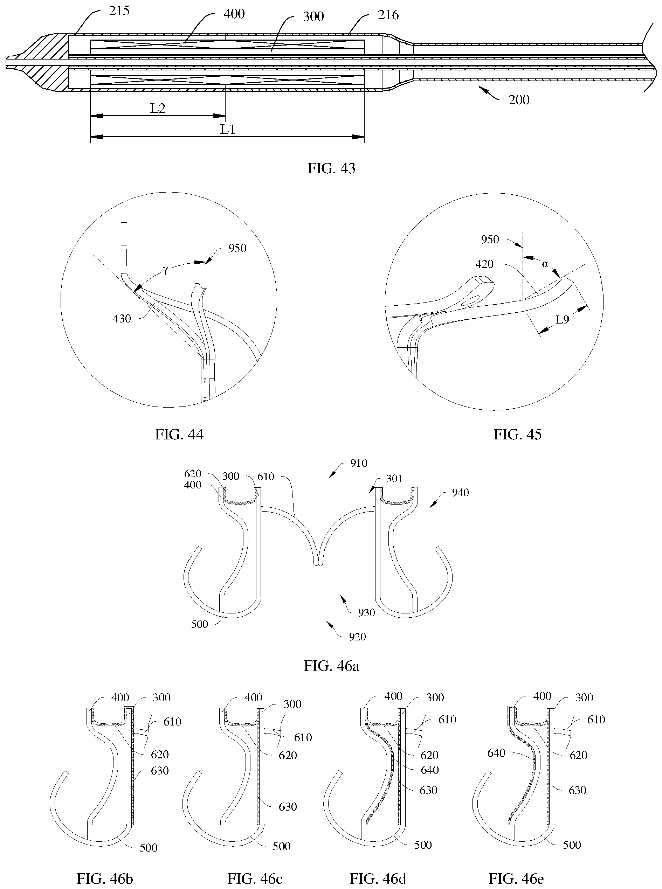

FIG. 43 is a structural view showing an interventional device according to an embodiment of the present disclosure engaged with an interventional assembly;
FIG. 44 is an enlarged view of part A in FIG. 35d;
FIG. 45 is an enlarged view of part B in FIG. 38d;
FIG. 46a is a schematic view of an interventional device according to an embodiment of the present disclosure sewn with valve leaflets and a sealing film;
FIG. 46b is a schematic view of a first covering film connected to the inside of the inner frame in FIG. 46 a;
FIG. 46c is a schematic view of a first covering film connected to the outside of the inner frame in FIG. 46a;
FIG. 46d is a schematic view of a second covering film connected to the inside of the outer frame in FIG. 46c;
FIG. 46e is a schematic view of a second covering film connected to the outside of the outer frame in FIG. 46c;

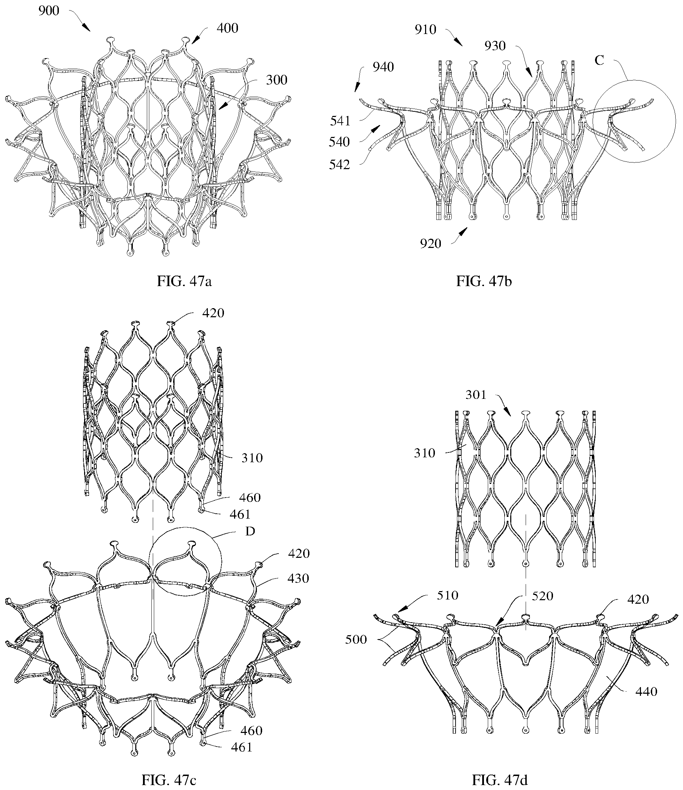

FIG. 47a is a structural view of an interventional device according to another embodiment of the present disclosure;
FIG. 47b is a front view of the interventional device in FIG. 47a;
FIG. 47c is an exploded view of the interventional device in FIG. 47a;
FIG. 47d is an exploded view of the interventional device in FIG. 47b;

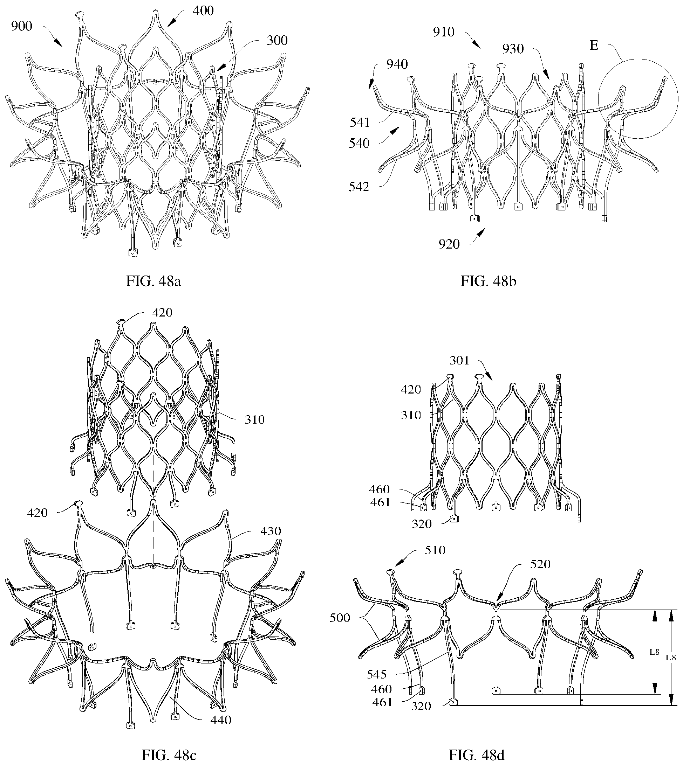

FIG. 48a is a structural view of an interventional device according to another embodiment of the present disclosure;
FIG. 48b is a front view of the interventional device in FIG. 48a;
FIG. 48c is an exploded view of the interventional device in FIG. 48a;
FIG. 48d is an exploded view of the interventional device in FIG. 48b;

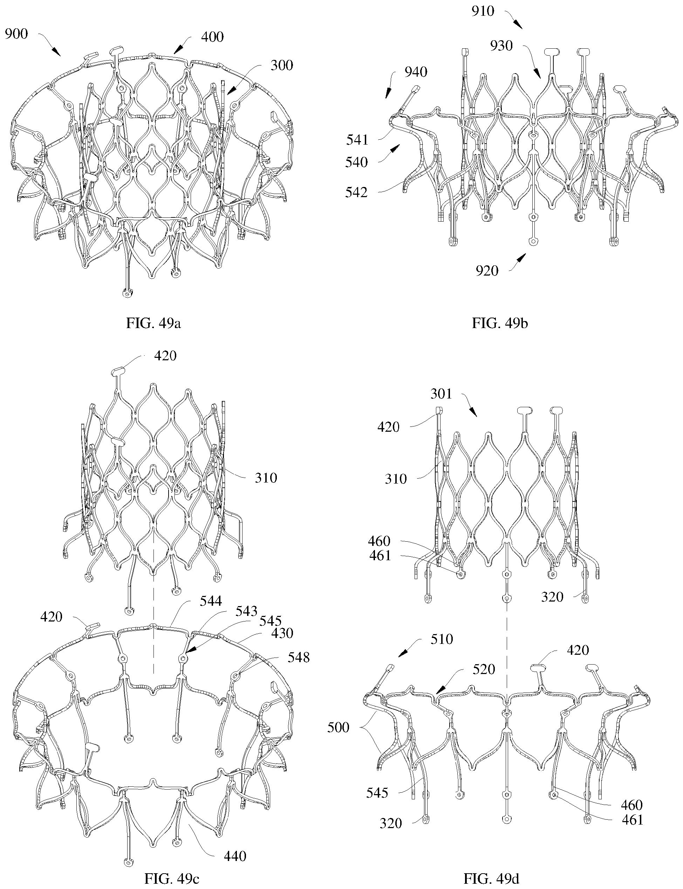

FIG. 49a is a structural view of an interventional device according to another embodiment of the present disclosure;
FIG. 49b is a front view of the interventional device in FIG. 49a;
FIG. 49c is an exploded view of the interventional device in FIG. 49a;
FIG. 49d is an exploded view of the interventional device in FIG. 49b;

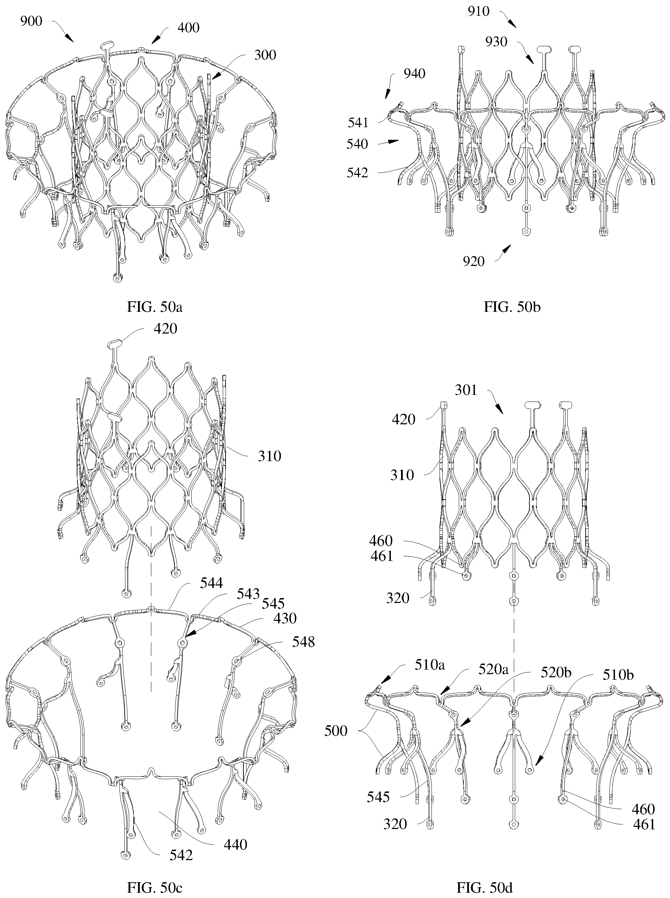

FIG. 50a is a structural view of an interventional device according to another embodiment of the present disclosure;
FIG. 50b is a front view of the interventional device in FIG. 50a;
FIG. 50c is an exploded view of the interventional device in FIG. 50a;
FIG. 50d is an exploded view of the interventional device in FIG. 50b;

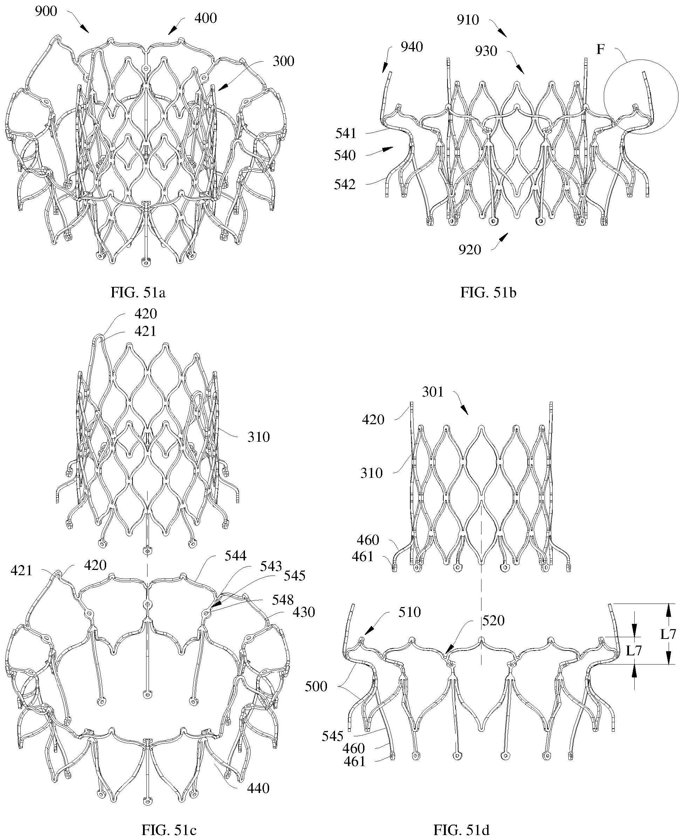

FIG. 51a is a structural view of an interventional device according to another embodiment of the present disclosure;
FIG. 51b is a front view of the interventional device in FIG. 51a;
FIG. 51c is an exploded view of the interventional device in FIG. 51a;
FIG. 51d is an exploded view of the interventional device in FIG. 51b;

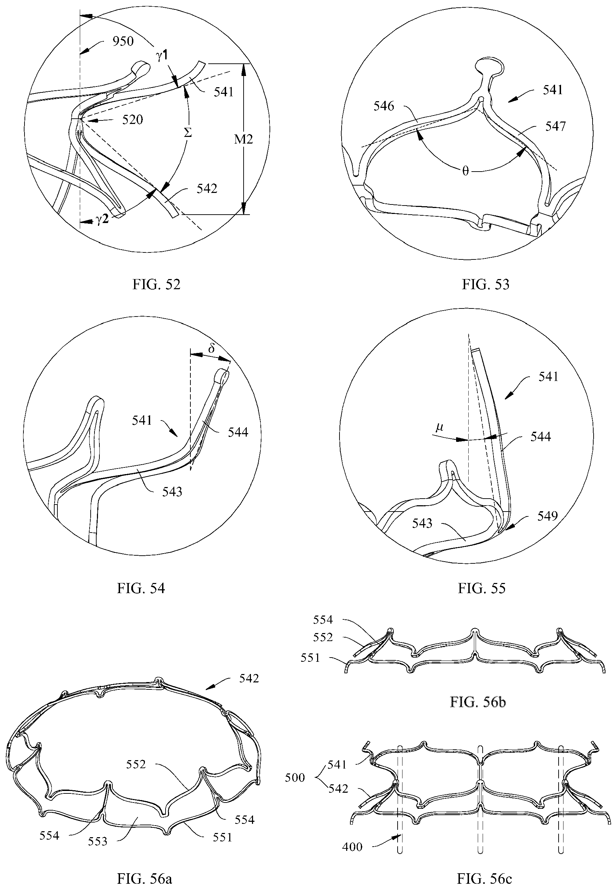

FIG. 52 is an enlarged view of part C in FIG. 47b;
FIG. 53 is an enlarged view of part D in FIG. 47c;
FIG. 54 is an enlarged view of part E in FIG. 48b;
FIG. 55 is an enlarged view of part F in FIG. 51b;
FIG. 56a is a structural view of positioning structures of an interventional device according to another embodiment of the present disclosure;
FIG. 56b is the front view of the positioning structures in FIG. 55a;
FIG. 56c is a front view showing an outer frame according to an embodiment of the present disclosure provided with the positioning structures in FIG. 56b;

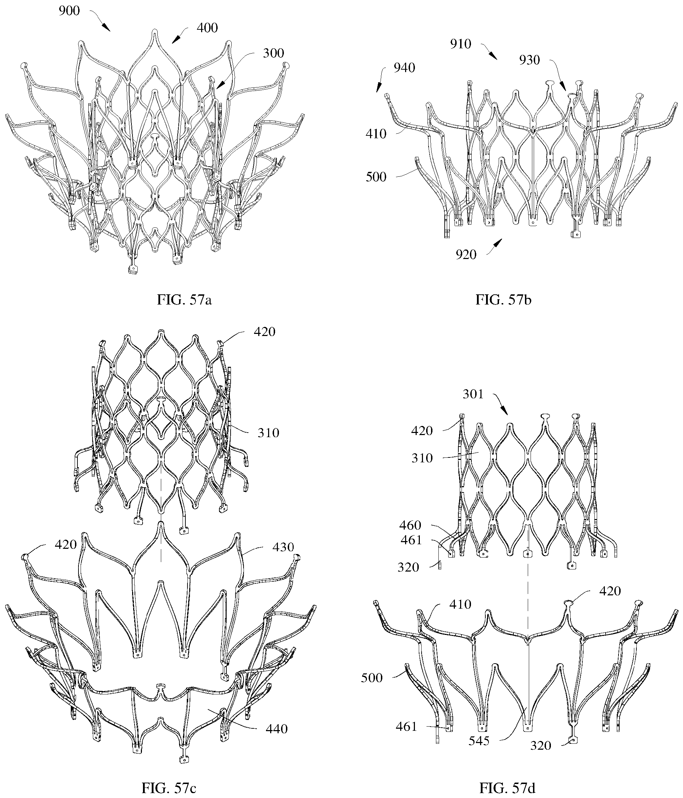

FIG. 57a is a structural view of an interventional device according to another embodiment of the present disclosure;
FIG. 57b is a front view of the interventional device in FIG. 57a;
FIG. 57c is an exploded view of the interventional device in FIG. 57a;
FIG. 57d is an exploded view of the interventional device in FIG. 57b;

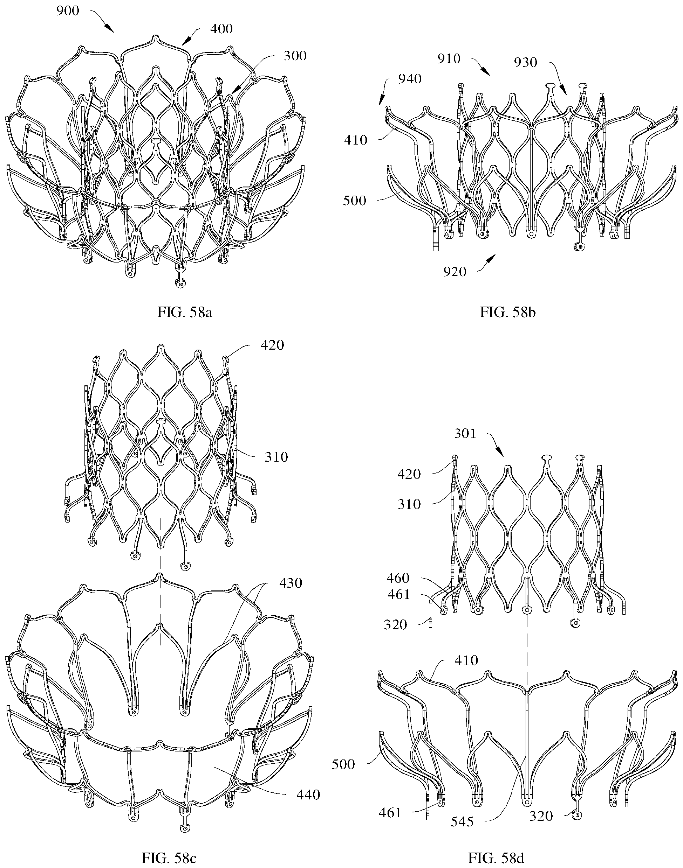

FIG. 58a is a structural view of an interventional device according to another embodiment of the present disclosure;
FIG. 58b is a front view of the interventional device in FIG. 58a;
FIG. 58c is an exploded view of the interventional device in FIG. 58a;
FIG. 58d is an exploded view of the interventional device in FIG. 58b;

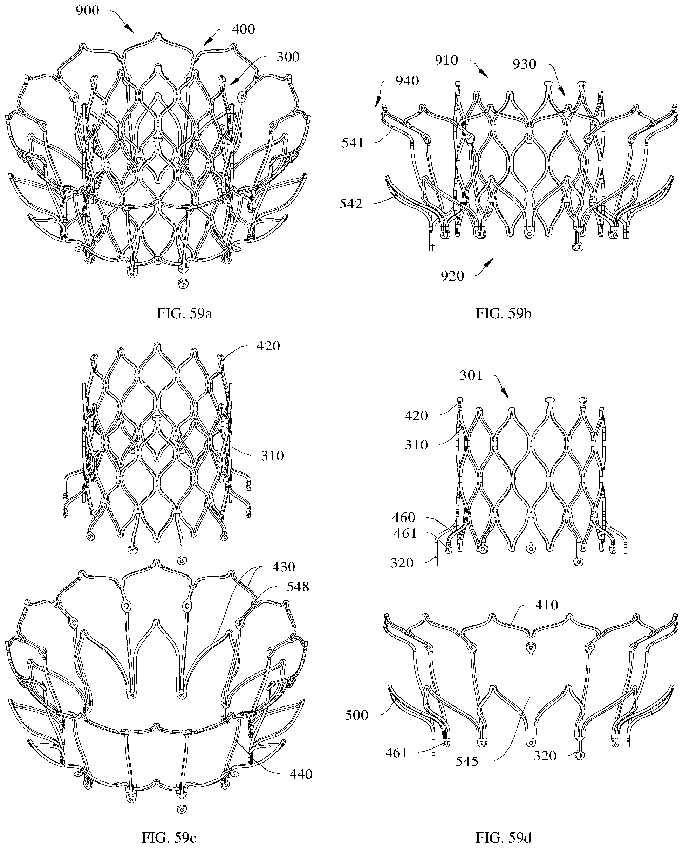

FIG. 59a is a structural view of an interventional device according to another embodiment of the present disclosure;
FIG. 59b is a front view of the interventional device in FIG. 59a;
FIG. 59c is an exploded view of the interventional device in FIG. 59a;
FIG. 59d is an exploded view of the interventional device in FIG. 59b;

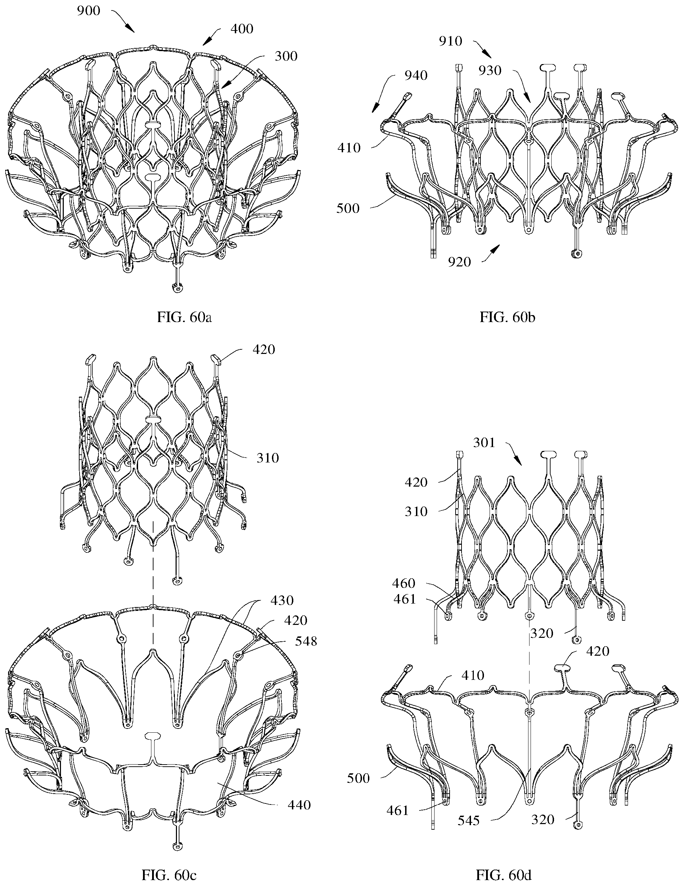

FIG. 60a is a structural view of an interventional device according to another embodiment of the present disclosure;
FIG. 60b is a front view of the interventional device in FIG. 60a;
FIG. 60c is an exploded view of the interventional device in FIG. 60a;
FIG. 60d is an exploded view of the interventional device in FIG. 60b;

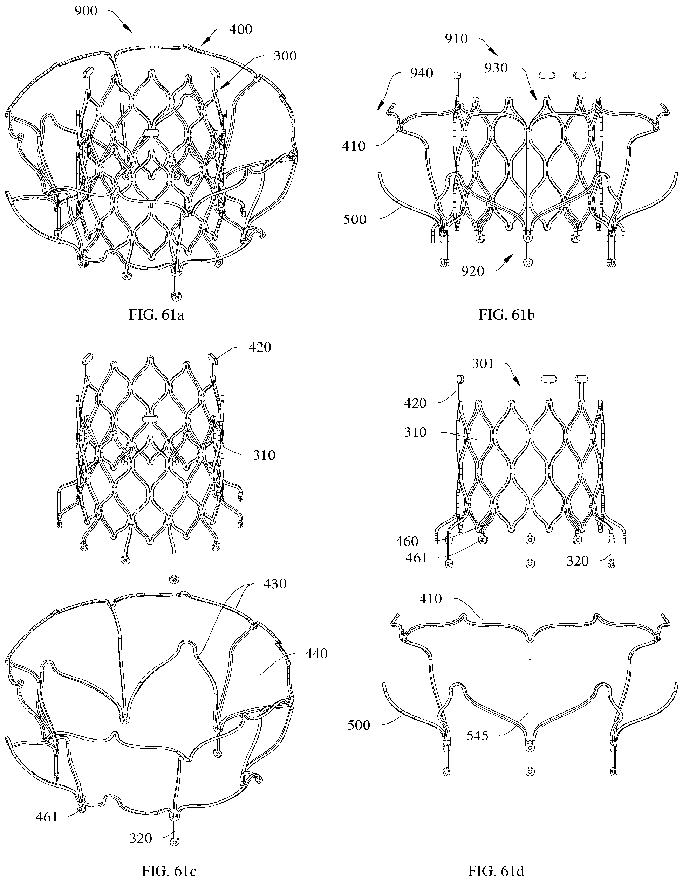

FIG. 61a is a structural view of an interventional device according to another embodiment of the present disclosure;
FIG. 61b is a front view of the interventional device in FIG. 61a;
FIG. 61c is an exploded view of the interventional device in FIG. 61a;
FIG. 61d is an exploded view of the interventional device in FIG. 61b;

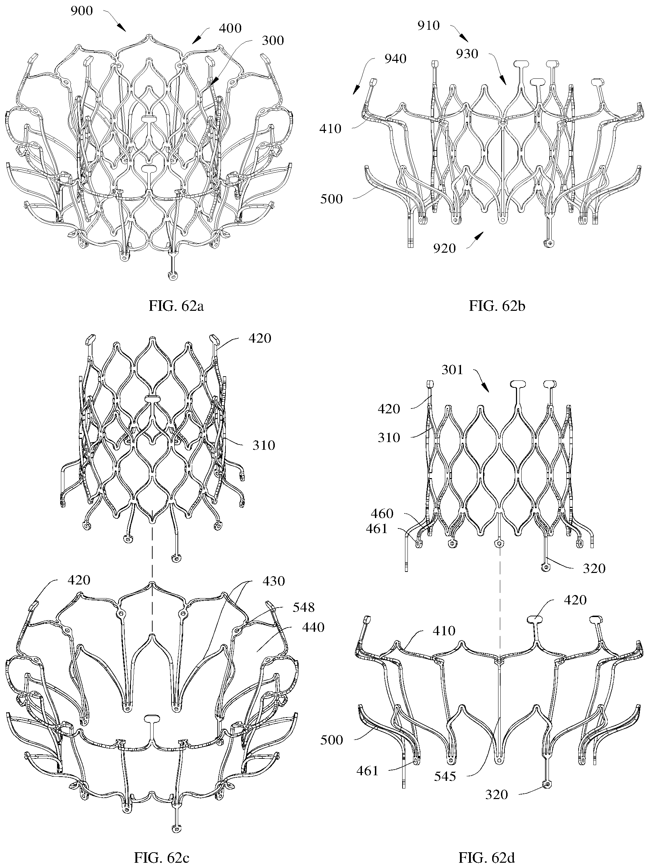

FIG. 62a is a structural view of an interventional device according to another embodiment of the present disclosure;
FIG. 62b is a front view of the interventional device in FIG. 62a;
FIG. 62c is an exploded view of the interventional device in FIG. 62a;
FIG. 62d is an exploded view of the interventional device in FIG. 62b;

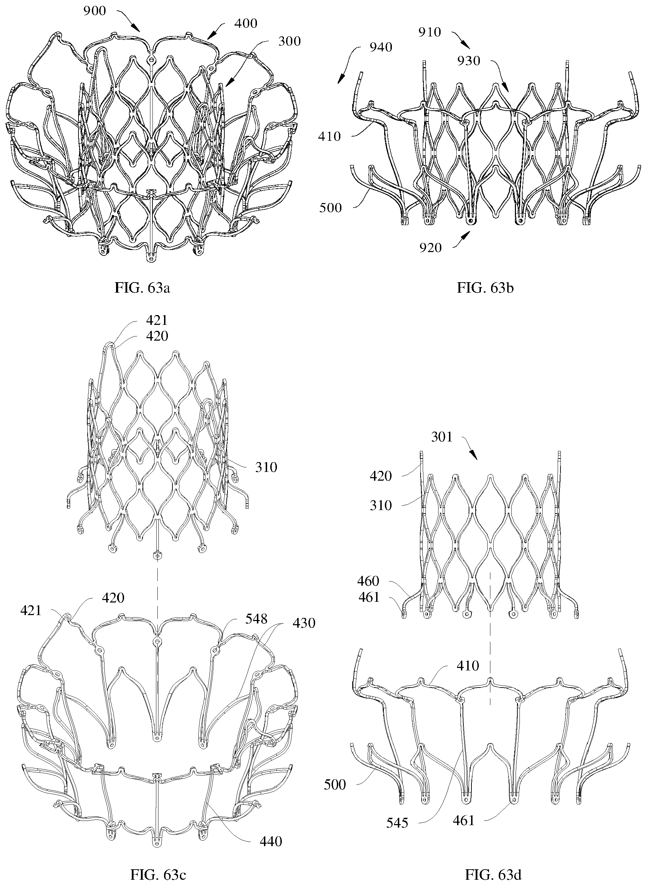

FIG. 63a is a structural view of an interventional device according to another embodiment of the present disclosure;
FIG. 63b is a front view of the interventional device in FIG. 63a;
FIG. 63c is an exploded view of the interventional device in FIG. 63a;
FIG. 63d is an exploded view of the interventional device in FIG. 63b;

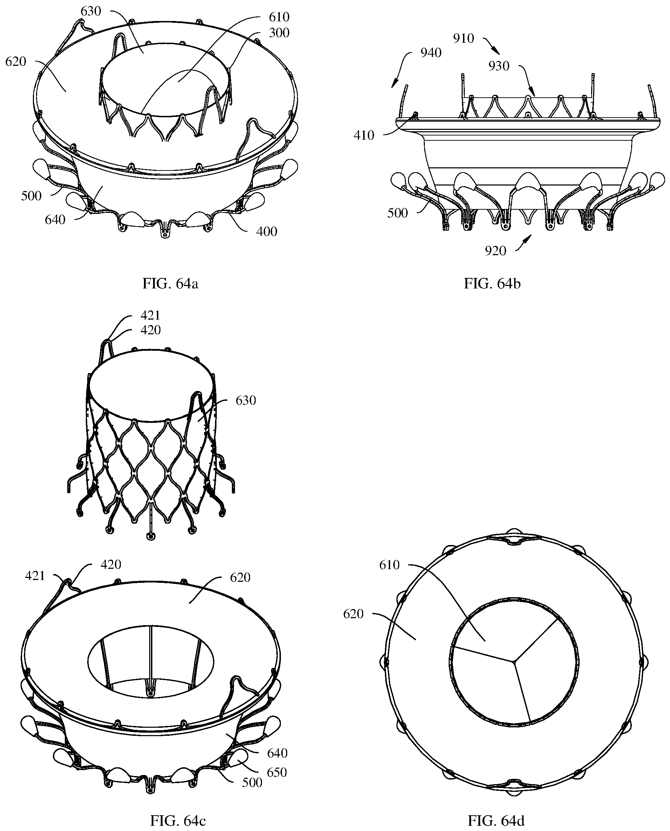

FIG. 64a is a structural view of an interventional device according to another embodiment of the present disclosure;
FIG. 64b is a front view of the interventional device in FIG. 64a;
FIG. 64c is an exploded view of the interventional device in FIG. 64a;
FIG. 64d is a top view of the interventional device in FIG. 64b;

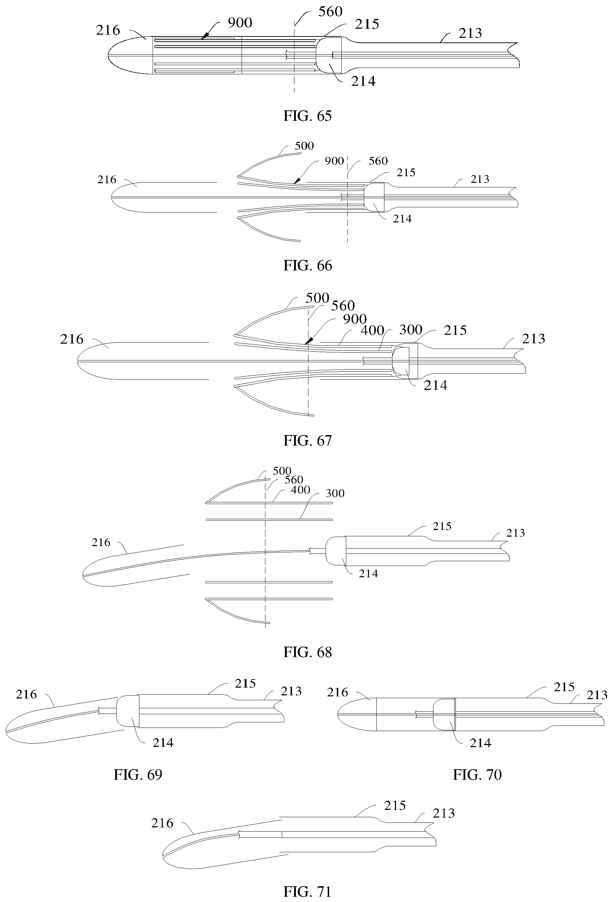

FIGS. 65 to 68 illustrate the release process of an interventional device according to an embodiment of the present disclosure;
FIG. 69 is a schematic view showing the connection of the first loading section and the second loading section according to an embodiment of the present disclosure;
FIG. 70 is a schematic view showing the successful connection of the first loading section and the second loading section according to an embodiment of the present disclosure;
FIG. 71 is a schematic view showing the failed connection of the first loading section and the second loading section according to an embodiment of the present disclosure.

List of reference signs:
11, distal part; 12, main part; 13, proximal part; 100, control handle; 101, exhaust joint; 102, annular groove; 103, Luer connector; 104, guide piece; 105, guide groove; 110, first support body; 111a, first fixed seat; 112a, first movable seat; 113a, second movable seat; 111b, first fixed seat; 112b, second movable seat; 113b, third fixed seat; 114b, fourth movable seat; 111c, third fixed seat; 112c, fourth movable seat; 111d, third fixed seat; 112d, fourth movable seat; 1111, connecting section; 1112, lock groove; 114, spring member; 115, snap button; 116, snap; 117, connecting sleeve; 1181, annular groove; 1182, annular protrusion; 1183, positioning groove; 119, slide groove; 120, second support body; 121, third movable seat; 122, second fixed seat; 123, fourth movable seat; 124, transmission tooth; 125, transmission tooth; 126, triggering member; 127, spring member; 128, mounting groove; 129, avoidance slot; 1201, first section; 1202, second section; 1203, support member; 130, third support body; 131c, first fixed seat; 132c, second movable seat; 133, snap groove; 131d, first fixed seat; 132d, second movable seat; 1301, control button; 1301a, control button; 1301b, control button; 1302, limiting member; 1302a, limiting member; 1302b, limiting member; 1303, reset member; 1303a, reset member; 1303b, reset member; 1304, locking tooth; 1305, abutting portion; 1305a, abutting portion; 1305b, abutting portion; 1306, elastic piece; 140, transmission member; 141, coupling seat; 142, connection slot; 143, transmission groove; 150, driving member; 150a, first driving member; 150b, second driving member; 150d, fourth driving member; 150c, fifth driving member; 151, first synchronization tooth; 153, first latching tooth; 160, synchronization lock ring; 161, second synchronization tooth; 170, anti-rotation lock ring; 171, second latching tooth; 172, positioning protrusion; 180, mounting seat; 181a, sealing member; 181b, housing; 182, cover plate; 183, hollow body; 184, positioning block; 185, positioning groove; 186, mounting base; 187, mounting groove; 188, connecting tube; 189, sealing plug; 1811, first housing; 1813, third housing; 1814, fourth housing; 1815, guide groove; 190, proximal end; 191, distal end;
200, interventional assembly; 210, delivery tube; 211, inner core tube; 212, support tube; 213, outer sheath; 214, mounting head; 215, first loading section; 216, section loading section; 217, guide wire; 220, bending component; 221a, inner bending tube; 222a, middle bending tube; 223a, outer bending tube; 221b, first bending tube; 222b, second bending tube; 223b, third bending tube; 224b, fourth bending tube; 221c, first bending tube; 222c, second bending tube; 223c, third bending tube; 224c, fourth pull wire;
300, inner frame; 301, blood flow channel; 310, cell; 320, connection portion;
400, outer frame; 410, positioning structure; 420, connecting ear; 421, eyelet; 430, v-shaped strut; 440, cell; 441, strut; 442, strut; 450, release opening; 460, fixing ear; 461, eyelet;
500, anchor arm; 501, opening; 502, eyelet; 510, free end; 520, fixed end; 540, clamping opening; 541, upper arm; 542, lower arm; 543, first section; 544, second section; 545, connecting strut; 546, first strut; 547, second strut; 548, eyelet; 549, turning point; 551, first layer; 552, second layer; 553, closed space; 554, connecting strut; 560, annulus line; 570, flexible element;
610, leaflet; 620, sealing film; 630, first covering film; 640, second covering film; 650, cushioning fabric;
710, limiting strut; 800, native valve leaflet;
900, interventional device; 910, inflow side; 920, outflow side; 930, inner side; 940, outer side; 950, axis.

### DESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of the present disclosure.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

The interventional device 900 may be any type of medical device implantable in the human body. The embodiments of the present disclosure will be described referring to a prosthetic atrioventricular valve. During surgery, the interventional device 900 is loaded and delivered through an interventional assembly. The interventional assembly usually includes a tube(s), a balloon(s), or a wire(s) among others, the proximal end of which is usually provided with a control handle, and the components of the interventional assembly are driven to move relative to each other through mounting seats and driving members on the control handle so as to release or retrieve the interventional device.

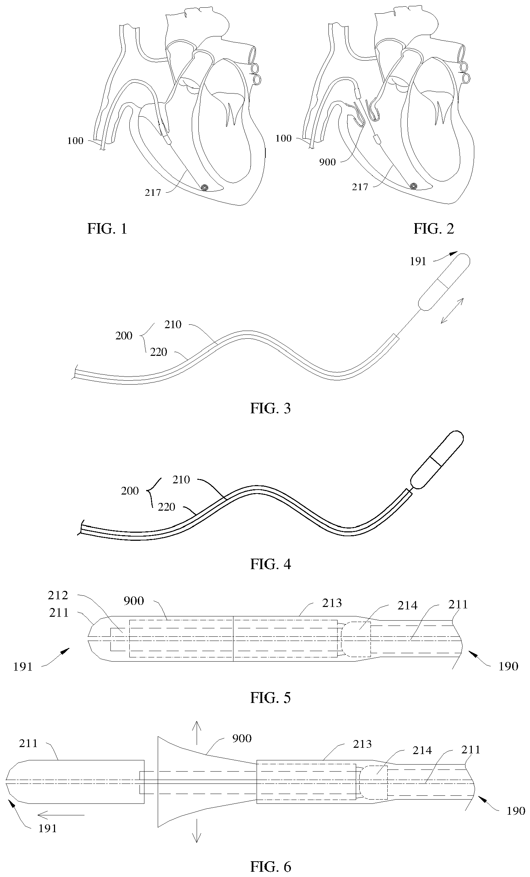

Referring to FIGS. 1 to 9, an embodiment of the present disclosure discloses a control handle 100 for operating an interventional assembly. The interventional assembly 200 includes a delivery tube 210 for loading an interventional device, and a bending component 220 for changing the orientation of the distal end of the delivery tube. The control handle has an axial direction in space, and the two ends of the control handle in the axial direction are the distal end 191 and the proximal end 190 respectively. The control handle includes:
two support bodies arranged sequentially in the axial direction and slidably engaged with each other in the axial direction;
a plurality of mounting seats 180 which are installed on the corresponding support bodies fixedly or movably, wherein the mounting seat(s) on one of the support bodies is configured to connect the bending component, and the mounting seat(s) on the other support body is configured to connect the delivery tube; and
a plurality of driving members 150 which are movably installed on the corresponding support bodies, and are engaged with and configured to drive the corresponding mounting seats on the supporting bodies.

Unless otherwise specified in this embodiment and the following embodiments, the distal end refers to the end of the control handle facing the interventional device, and the opposite end to the distal end is the proximal end. The same applies to the interventional assembly 200.

The interventional device 900 has a compressed state and an expanded state, and can be loaded in the distal end of the delivery tube 210 in the compressed state. The operator can operate the control handle 100 to drive the interventional assembly 200 to move into and out of the human body. The delivery tube 210 functions to deliver the interventional device to a predetermined position, and the posture of the delivery tube 210 is controlled by the bending component 220 during the delivery and withdrawal process.

The support bodies are used to support or install the delivery tube, the bending component, the mounting seats, and the driving members, etc. Other components of the control handle can also be installed on the support bodies. The two support bodies slidably engaged with each other may be that one support body is fixed relative to the control handle, and the other support body is axially slidable in the control handle, or that the two support bodies are both axially slidable. The driving force for sliding can be provided by the driving member or other components on the control handle. The delivery tube 210 includes at least one tube for loading the interventional device. The bending component 220 includes at least one bending tube/wire, which acts on and bends the delivery tube. If it is necessary to achieve multi-directional bending, more bending tubes/wires can be provided accordingly.

When installing the mounting seats on the support bodies (fixedly or movably), the two support bodies can each use the two connection modes (fixed connection and movable connection), or the two support bodies each use only one connection mode (fixed connection or movable connection), or one of the support bodies uses only one connection mode (fixed connection or movable connection) and the other support body uses the two connection modes (fixed connection and movable connection). A rack and gear transmission mechanism is provided between the driving member and the mounting seat. For example, the rack is fixed on the mounting seat and extends in the axial direction of the support body, and the gear is fixed on the driving member or is engaged with and configured to be driven by the driving member. For another example, the driving member is configured to directly drive the mounting seat. That is, the driving member is directly fixed to the mounting seat, and when the operator applies force to the driving member, the driving member directly acts on the mounting seat.

The driving member 150 can be operated by the operator or by a stabilizer connected to the control handle. For example, referring to FIG. 3, in one embodiment, the driving member is provided with an interface (for example, an annular groove 102) for coupling to the stabilizer.

In this embodiment, the mounting seats are divided into two groups according to the functions thereof and are arranged on corresponding support bodies. Since the two support bodies are slidably engaged with each other, by combination, the functions of the control handle are multiplied.

In one embodiment, the two support bodies include a first support body 110 located at the distal end of the control handle and a second support body 120 located at the proximal end of the control handle.

The mounting seats on the support bodies are divided into a fixed seat(s) and a movable seat(s) according to the movement relationships thereof with the corresponding support bodies. The movable seats on the two support bodies are respectively configured with driving members. That is, the movable seats can move independently. In some other embodiments, a synchronization mechanism can be provided for the movable seats, which can simplify the operation process or achieve synchronous control.

The fixed seat is fixedly connected in the corresponding support body, and the movable seat is movable relative to the corresponding support body and is driven by the corresponding driving member. The specific movement of the movable seat is not strictly limited and can be designed in various ways, depending on the desired effects in practice. For example, the movable seat may be slidable in the axial direction of the support body or rotatable around the axis of the support body. In various embodiments, the quantity and type of the mounting seats 180 on the support bodies can be set differently. As an example, the support bodies may be provided with at least one fixed seat and at least one movable seat. For example, the mounting seats on the support bodies may include one fixed seat and one movable seat. For another example, the mounting seats on the support bodies may include one fixed seat and two movable seats. For a further example, the mounting seats on the support bodies may include two fixed seats and two movable seats.

The following will describes the specific structures of the bending component and the delivery tube as well as the arrangement of the corresponding mounting seats.

In one embodiment, the bending component 220 includes a tube(s) or a pull wire(s). One or more groups of bending components 220 can be provided, and each group includes two bending components. The distal portions of the two bending components are relatively fixed, and the proximal ends thereof are coupled to the corresponding mounting seats 180 respectively and are movable relative to each other (i.e., the proximal ends of the tubes or wires can move relative to each other).

In one embodiment, the bending component 220 includes two bending tubes arranged sequentially from outside to inside. The mounting seats on the first support body 110 include a fixed seat and a movable seat. The distal portions of the two bending tubes are relatively fixed, and the proximal ends thereof are respectively connected to the fixed seat and the movable seat. The movable seat is configured to move to drive one of the bending tubes to move so as to bend the distal end of the bending component.

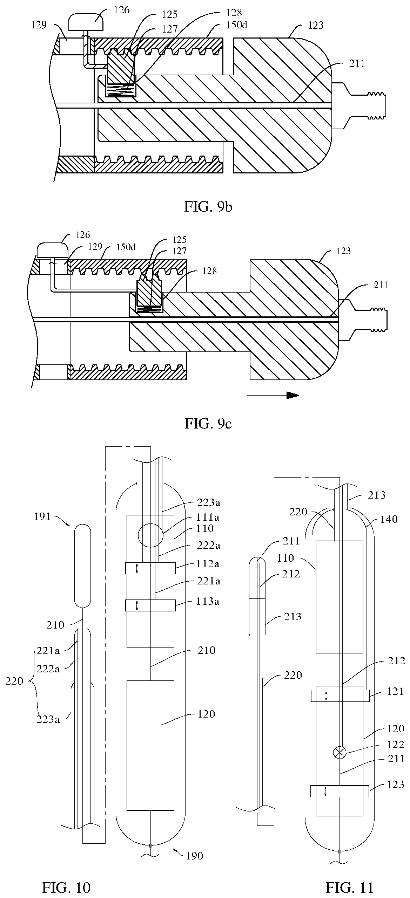

Referring to FIG. 10, in another embodiment, the bending component 220 includes an outer bending tube 223a, a middle bending tube 222a and an inner bending tube 221a arranged sequentially from outside to inside. The portions of the three tubes near the distal end of the bending component are relatively fixed, and the proximal ends of the three tubes are respectively coupled to the corresponding mounting seats and are movable relative to each other. At the distal end of the bending component, one of the three bending tubes is fixedly connected to the other two, and one of them is connected to a fixed seat, and the other two are connected to two movable seats respectively, so as to achieve at least two-directional bending.

Referring to FIGS. 5 to 7, in one embodiment, the delivery tube 210 includes an outer sheath 213, a support tube 212 and an inner core tube 211 arranged sequentially from outside to inside. The proximal ends of the three are respectively coupled to the corresponding mounting seats and are movable relative to each other.

The distal end of the outer sheath 213 is provided with a first loading section 215 with an increased diameter, and the proximal end thereof is connected to the movable seat on the second support body 120.

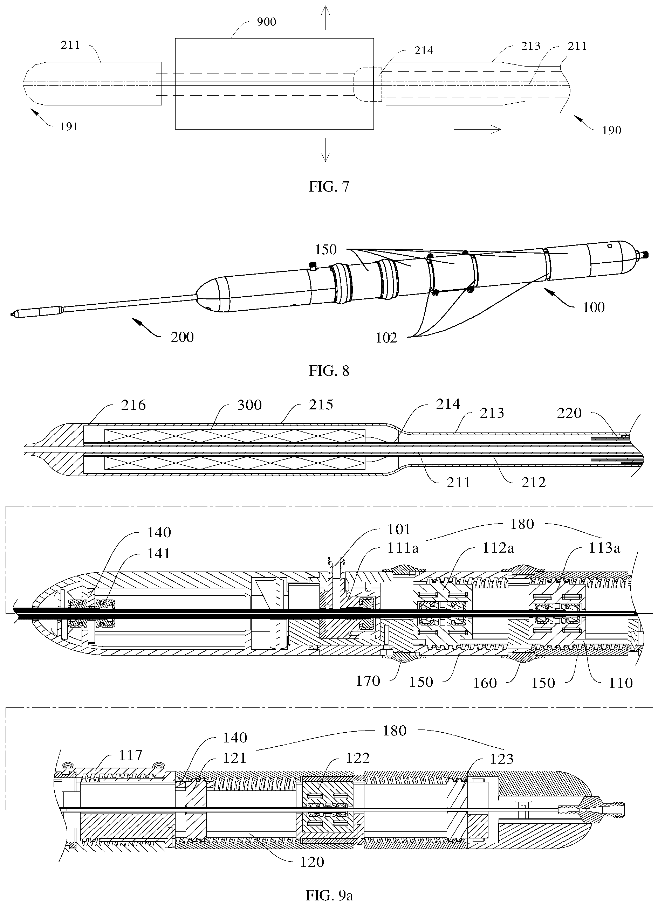

Referring to FIG. 9a, the support tube 212 is provided with a mounting head 214 near the distal end thereof for engaging with the interventional device. The end of the interventional device is provided with a connecting part (such as a connecting ear). In the compressed state, the interventional device is loaded around the support tube, and the mounting head 214 is engaged with the connecting part of the interventional device, thereby restricting the movement of the interventional device in the axial direction of the support tube. The proximal end of the support tube 212 is connected to the fixed seat on the second support body 120.

The inner core tube 211 is a hollow tube, and a guide wire 217 for establishing an intervention path can be inserted therein. The distal end of the inner core tube 211 exceeds the distal end of the outer sheath 213 and the exceeding part is provided with a second loading section 216 with an increased diameter. The first loading section 215 and the second loading section 216 are opened towards each other and contact with each other to form a loading space for accommodating the interventional device. The contact portions of the two loading sections can abut against each other in the axial direction, or overlap with each other slightly in the radial direction. The proximal end of the inner core tube 211 is connected to the movable seat on the second support body 120.

The distal end of the mounting head 214 has a chamfered structure. When the inner core tube 211 is retracted, the opening of the second loading section 216 will abut against the chamfered structure and be guided to contact with the opening of the first loading section 215.

In this embodiment, different parts of the interventional device are covered by the first loading section 215 and the second loading section 216 respectively. During release, the first loading section 215 and the second loading section 216 move in opposite directions to expose the interventional device, so that the interventional device can adapt to the physiological structure of the atrioventricular valve better. Specifically, after the interventional device is approximately positioned in place, one of the first loading section 215 and the second loading section 216 moves toward the atrium side, and the other moves toward the ventricular side. Therefore, the first loading section 215 and the second loading section 216 can adapt to various interventional devices with different sizes, eliminating the defects due to the limited release space.

Referring to FIGS. 5 to 10, FIG. 14 and FIG. 15, in one embodiment, the driving member 150 is cylindrical and is rotatably arranged around the outer periphery of the corresponding support body. The driving member 150 and the corresponding mounting seat are threadedly engaged with each other. Each support body is provided with a slide groove 119 for guiding the mounting seat to move linearly. This linear motion can also be understood as an axial motion. At least parts of the mounting seats are exposed to the corresponding support bodies, and male threads are provided on the exposed parts. The inner peripheries of the driving member are provided with female threads that engage with the male threads. The mounting seats on the first support body 110 include a first fixed seat 111a, a first movable seat 112a and a second movable seat 113a arranged sequentially from the distal end to the proximal end, which are respectively connected to the outer bending tube 223a, the middle bending tube 222a and the inner bending tube 221a. The mounting seats on the second support body 120 include a third movable seat 121, a second fixed seat 122 and a fourth movable seat 123 arranged sequentially from the distal end to the proximal end, which are respectively connected to the outer sheath 213, the support tube 212 and the inner core tube 211. The driving members arranged on the first support body 110 includes a first driving member 150a, a second driving member 150b and a connecting sleeve 117 arranged sequentially from the distal end to the proximal end, and the driving members arranged on the second support body 120 include a fourth driving member 150d and a fifth driving member 150c arranged sequentially from the proximal end to the distal end.

Referring to FIG. 9b and FIG. 9c, in another embodiment, the inner core tube 211 can be threadedly driven by the fourth driving member 150d. Alternatively, the inner core tube 211 can be driven by other means. Specifically, the engagement between the transmission tooth 125 and the fourth driving member 150d can be disengaged by a triggering member 126, after that, the fourth movable seat 123 can be directly driven, so as to directly pull the inner core tube 211 axially, so that, for example, after the interventional device is released, the inner core tube 211 can be withdrawn quickly.

The fourth movable seat 123 can be opened with a mounting groove 128. Alternatively, a base can be additionally fixed to the inner core tube 211, and the mounting groove 128 can be defined in the base. The transmission tooth 125 is movably arranged in the mounting groove 128, and can be guided by the mounting groove 128 to change the positional relationship thereof with the fourth driving member 150d. A spring member 127 can be provided in the mounting groove 128 to drive the transmission tooth 125 to engage with the fourth driving member 150d, while the triggering member 126 functions to disengage the engagement between the transmission tooth 125 and the fourth driving member 150d.

The transmission tooth 125 may slide radially to change the positional relationship thereof with the fourth driving member 150d, or swing around an axis based on a lever principle to change the positional relationship thereof with the fourth driving member 150d.

To avoid interference, the corresponding support body is provided with an avoidance slot 129 for the triggering member 126 to move with the inner core tube 211. Preferably, the triggering member 126 is offset from the fourth driving member 150d in the axial direction, so as to avoid interfering with the rotation of the fourth driving member 150d. Referring to FIG. 14, in one embodiment, a Luer connector 103 is connected to the proximal end of the inner core tube 211.

Referring to FIG. 28, a guide mechanism for sliding is provided between the Luer connector 103 and the control handle 100. The guide mechanism for sliding includes a guide piece 104 provided on the Luer connector and a guide groove 105 provided on the control handle that engages with the guide piece.

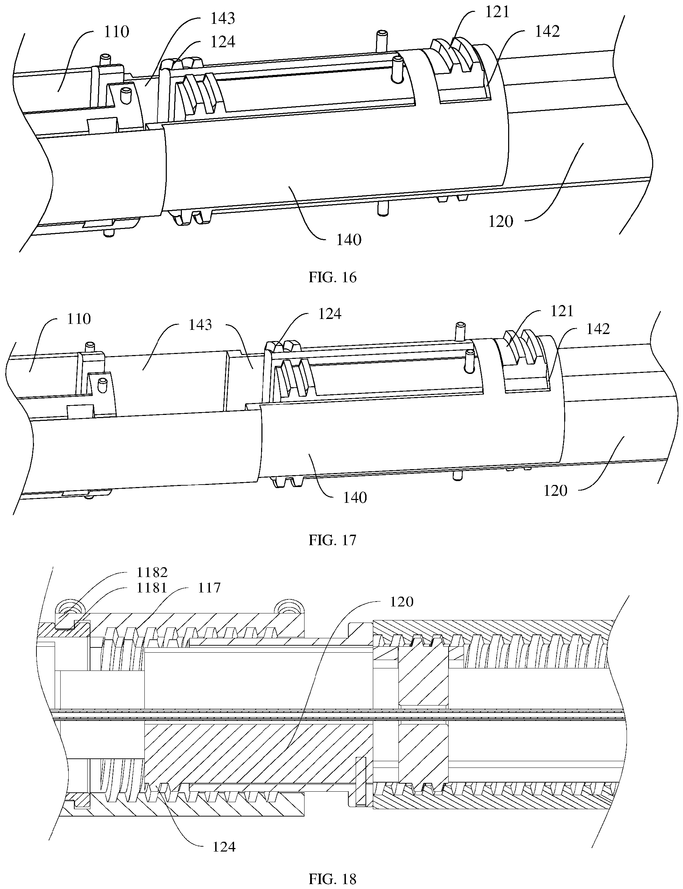

Referring to FIG. 11 and FIG. 14 to FIG. 18, in one embodiment, a transmission member 140 is connected to the third movable seat 121. The transmission member extends distally until it exceeds the first support body 110, and the exceeding part is provided with a coupling seat 141. The proximal end of the outer sheath 213 is fixed to the coupling seat 141.

In general, the control handle involves tubes arranged in the radial direction and mounting seats connected to the tubes and arranged in the axial direction in a certain order. For convenience of assembly, the tubes are usually arranged from outside to inside and the mounting seats connected to the tubes are arranged from the distal end to the proximal end.

Accordingly, the outer sheath 213 is connected to the mounting seat in the second support body, and is located inside the bending component 220 in the radial direction. The transmission member 140 is arranged so that the outer sheath 213 is located outside all of the tubes and covers all of the tubes. The transmission member 140 can move together with the second support body 120 and slide in the axial direction relative to the first support body 110, so that the delivery tube 210 can move as a whole. Now, at least the bending component 220 remains stationary so that the spatial posture of the distal end of the bending component 220 is fixed, that is, the orientation of the distal end of the bending component 220 is fixed, and the distal end of the delivery tube 210 extending beyond the bending component 220 can move as a whole in this orientation, for example, being withdrawn or advancing as a whole, so as to ensure an optimal release position of the interventional device 900 at the diseased site.

In order to realize the synchronous movement of the transmission member 140 and the second support body 120, specifically, a connection slot 142 is provided on the transmission member 140, and at least part of the third movable seat 121 passes through the connection slot 142, so that the transmission member 140 can move following the second support body 120. In the axial direction, the walls of the connection slot 142 abut against the third movable seat 121.

The fifth driving member 150c can only rotate relative to the second support body 120 around the axis. When the fifth driving member 150c stops rotating, the corresponding third movable seat 121 is fixed relative to the second support body 120, so that the transmission member 140 and the second support body are fixed relative to each other and can move synchronously. When the fifth driving member 150c rotates, it drives the third movable seat 121 to slide relative to the second support body 120 in the axial direction, and the corresponding transmission member 140 moves following the third movable seat 121. The transmission member 140 has a fixed state in which it can move synchronously with the second support body 120, and a movable state in which it is fixed with the third movable seat 121 and slides relative to the second support body 120.

The transmission member 140 moving following the second support body 120 means that the transmission member 140 can move synchronously with the second support body 120. To achieve this, the fifth driving member 150c needs to stop rotating.

Referring to FIG. 9a and FIG. 14 to FIG. 18, in one embodiment, the proximal end of the first support body 110 is mounted with a rotatable connecting sleeve 117, and the distal end of the second support body 120 extends into and is threadedly engaged with the connecting sleeve 117. A snap-fit mechanism is provided between the connecting sleeve 117 and the first support body 110 to prevent them from separating from each other in the axial direction. The snap-fit mechanism includes an annular groove 1181 provided on the first support body 110, and an annular protrusion 1182 provided on the connecting sleeve 117 that matches the annular groove 1181. The snap-fit mechanism allows the connecting sleeve 117 to rotate around the axis to drive the second support body 120 so that the delivery tube 210 can move as a whole.

In one embodiment, the outer periphery of the distal end of the second support body 120 is provided with transmission teeth 124, and the transmission teeth 124 are threadedly engaged with the female thread provided in the connecting sleeve 117. All the mounting seats on the second support body 120 are located at the proximal side of the transmission teeth 124. Accordingly, all driving members that engage with these mounting seats are located at the proximal side of the connecting sleeve 117.

In one embodiment, a limiting mechanism is provided between the first support body 110 and the second support body 120 to prevent them from rotating relative to each other, and both the first support body 110 and the second support body 120 are slidably engaged with the limiting mechanism.

In one embodiment, the limiting mechanism is the transmission member 140. The transmission member 140 and the first support body 110 are prevented from rotating relative to each other. Specifically, a transmission groove 143 is defined in the transmission member 140, part or the entire of the support body is located in the transmission groove 143, and the groove walls of the transmission groove 143 clamp the support body from two opposite sides in the radial direction of the support body. The transmission member 140 and the second support body 120 are prevented from rotating relative to each other. Specifically, the two opposite side groove walls of the connection slot 142 abuts against the third movable seat 121 in the circumferential direction of the support body.

In one embodiment, the control handle 100 further includes a synchronization mechanism, which acts between two adjacent driving members. The synchronization mechanism includes:
first synchronization teeth 151 on the driving members 150; and
a synchronization lock ring 160 being movable in the axial direction of the control handle. The synchronization lock ring is provided with second synchronization teeth 161. In the axial direction of the control handle, the synchronization lock ring has a synchronization position and a relative release position.

At the synchronization position, the second synchronization teeth 161 mesh with the first synchronization teeth 151 on the two driving members so as to link the two driving members. At the release position, the second synchronization teeth 161 at most mesh with the first synchronization tooth 151 on one driving member so as to release the linkage. The first synchronization teeth 151 on the two driving members corresponding to the synchronization lock ring 160 may have different structures, and the corresponding second synchronization teeth may also have different structures, provided that the above functions can be achieved. At the synchronization position, the two driving members are linked, and thus the corresponding tubes connected to them will also be linked. At the release position, the tubes connected to them can move separately to achieve different functions as exemplarily explained below.

Referring to FIG. 10, FIG. 14 and FIG. 21 to FIG. 23c as well as the aforementioned bending component 220, the synchronization lock ring 160 acts on the first driving member 150a and the second driving member 150b connected to the middle bending tube 222a and the inner bending tube 221a. The synchronization lock ring 160 is always meshed with the first driving member 150a, and the synchronization lock ring 160 is allowed to slide in the axial direction relative to the first driving member 150a to mesh with the second driving member 150b, wherein the synchronization teeth of the two meshing with each other guide the sliding process. The synchronization lock ring 160 has a plurality of second synchronization teeth 161 on the side close to the second driving member 150b, which are arranged at intervals on the inner periphery of the synchronization lock ring 160, and the second driving member 150b is provided with a first synchronization tooth 151, which facilitates re-meshing after the two rotate. When the synchronization mechanism is at the release position, the two driving members rotate separately to realize the corresponding bending operations. When the synchronization mechanism is at the synchronization position, the two driving members move synchronously or are locked synchronously.

In one embodiment, the control handle 100 further includes an anti-rotation mechanism, and the anti-rotation mechanism includes:
a first latching tooth 153 provided on the driving member; and
an anti-rotation lock ring 170 slidably arranged around the support body. The anti-rotation lock ring 170 is provided with second latching teeth 171. In the axial direction of the control handle, the anti-rotation lock ring has relative locked and unlocked positions.

At the locked position, the first latching tooth 153 and the second latching teeth 171 are engaged with each other to prevent the driving member 150 from rotating.

At the unlocked position, the first latching tooth 153 and the second latching teeth 171 are disengaged, allowing the driving member 150 to rotate.

A positioning mechanism for preventing the rotation of the anti-rotation lock ring 170 is provided between the anti-rotation lock ring 170 and the support body, and the anti-rotation lock ring 170 and the synchronization lock ring 160 act on the same driving member, so that two driving members can be locked synchronously. The first latching teeth on the support body and the driving member corresponding to the anti-rotation lock ring 170 may have different structures, and the corresponding second latching teeth 171 may also have different structures, provided that the above functions can be achieved. Referring to FIG. 22 and FIG. 24a to FIG. 24c, the positioning mechanism includes a positioning protrusion provided on the first support body 110 and a positioning groove 1183 defined on the anti-rotation lock ring 170 and matching with the positioning protrusion 172 to prevent the anti-rotation lock ring 170 from rotating. The positioning protrusion 172 extends in the axial direction, and thus can also guide the sliding process of the anti-rotation lock ring 170, facilitating the engagement of the anti-rotation lock ring 170 with the first driving member 150a. The anti-rotation lock ring 170 has a plurality of second latching teeth 171 on the side close to the first driving member 150a, which are arranged at intervals on the inner periphery of the anti-rotation lock ring 170, and the first driving member 150a is provided with a first latching tooth 153, which facilitates re-meshing after the two rotate. When the anti-rotation lock ring 170 is engaged with the first driving member 150a, and the synchronization lock ring 160 is engaged with the second driving member 150b, the two driving members are locked synchronously.

The following will describe the arrangements with various bending components with different structures or quantities and corresponding support bodies, in combination with the aforementioned support body which is provided with two fixed seats and two movable seats.

Referring to FIG. 12 and FIG. 29, in one embodiment, two groups of bending components 220 are provided, and each group includes two bending components. The bending components in different groups are inserted one in the other, and the distal ends thereof are movable relative to each other.

The bending components 220 include four bending tubes arranged sequentially from outside to inside. The outer two bending tubes constitute one group, and the inner two bending tubes constitute another group. The mounting seats on the first support body 110 include two fixed seats and two movable seats. One of the two bending tubes in the same group is connected to the corresponding fixed seat and the other is connected to the corresponding movable seat.

Specifically, the four bending tubes include a first bending tube 221b, a second bending tube 222b, a third bending tube 223b and a fourth bending tube 224b arranged sequentially from inside to outside, and the corresponding mounting seats on the support body include a first fixed seat 111b, a second movable seat 112b, a third fixed seat 113b and a fourth movable seat 114b.

Referring to FIG. 13 and FIG. 30a to FIG. 31b, in another embodiment, a third support body 130 is further movably connected to the distal end of the first support body 110.

The driving member and the mounting seat(s) on the third support body 130 are installed in the same way as those on the other support bodies. For example, a fixed seat and a movable seat can be provided on the third support body 130, and the movable seat can be provided with a driving member. The fixed seat and the movable seat are connected to a group of bending components 220 consisting of two bending tubes/wires, or a bending tube and a bending wire for bending the distal end thereof. It can be derived from the arrangement of the tubes that said group of bending components is located at the outermost side of all tubes.

The first support body 110 and the third support body 130 are movably connected with each other in a slide fit in the axial direction of the control handle or by a detachable connection.

In this embodiment, a locking mechanism is provided between the third support body 130 and the first support body 110 to prevent them from separating from each other. The locking mechanism includes:
a snap groove 133 provided on the third support body;
a snap button 115 radially slidably installed on the first support body 110 and having a snap 116 that matches the snap groove 133; and
a spring member 114 arranged in the first support body and acting on the snap button so that the snap can extend into the snap groove.

The locking mechanism has:
a locked state, in which the snap extends into the snap groove to prevent the first support body 110 and the third support body 130 from separating from each other in the axial direction; and
an unlocked state, in which the snap button is pressed so that the snap moves out of the snap groove, allowing the third support body 130 to move axially away from the first support body 110.

In case with the third support body 130, two groups of bending components 220 can be provided, wherein one group is connected to the first support body 110 and the other is connected to the third support body 130.

The bending components 220 include a pull wire and three bending tubes arranged sequentially from outside to inside. The mounting seats on the first support body 110 include a fixed seat and a movable seat, and one of the two inner bending tubes is connected to the fixed seat and the other is connected to the movable seat. The mounting seats on the third support body 130 include a fixed seat and a movable seat, the outermost bending tube is connected to the fixed seat, and the pull wire is connected to the movable seat.

Referring to FIG. 13, specifically, the mounting seats in the third support body 130 and the first support body 110 include a first fixed seat 131c, a second movable seat 132c, a third fixed seat 111c and a fourth movable seat 112c arranged sequentially from the distal end to the proximal end, and the corresponding bending components 220 include a first bending tube 221c, a second bending tube 222c, a third bending tube 223c and a fourth pull wire 224c arranged from inside to outside.

In one embodiment, in the axial direction of the control handle, a coupling area is remained between the first support body 110 and the third support body 130. The third movable seat 121 is connected to the transmission member 140, and the transmission member extends distally and beyond the first support body 110 to the coupling area. The distal end of the transmission member 140 is provided with a coupling seat 141 located in the coupling area, and the proximal end of the outer sheath 213 is fixed to the coupling seat.

The transmission member 140 does not extend into the third support body 130 and is not connected with the third support body 130. The third support body 130 is movably connected to the first support body and does not affect the movement of the transmission member when it is detached from the first support body. Thus, the structure of the transmission member 140 and the connection thereof with other components can refer to the above embodiments.

For the two groups of bending components 220 connected to the first support body 110 and the third support body 130 respectively, the group of bending component 220 connected to the first support body 110 is located in the outer sheath 213, and the group of bending component 220 connected to the third support body 130 is located outside the outer sheath 213, which are movably engaged with the outer sheath 213 at the distal end and act on the outer sheath 213 for bending.

Referring to FIGS. 25 to 27, in one embodiment, the mounting seat 180 includes a sealing member 181a connected to the proximal end of the delivery tube 210 or the bending component 220, and a housing 181b that engages with the support body. The sealing member is arranged inside the housing.

In one embodiment, the housing 181b includes a cover plate 182 and a hollow body 183. The outer periphery of the hollow body 183 is provided with a male thread that engages with the female thread of the driving member.

In this embodiment, a limiting mechanism is provided between the cover plate 182 and the hollow body 183 to prevent them from moving relative to each other. The limiting mechanism includes a positioning block 184 provided on one of the cover plate 182 and the hollow body 183, and a positioning groove 185 defined on the other and matching the positioning block.

In one embodiment, the cover plate 182 is provided with a mounting base 186 for installing the sealing member, and the hollow body 183 is provided with a mounting groove 187 for accommodating the sealing member. In the assembled state, the mounting base 186 and the mounting groove 187 clamps and fixes the sealing member and prevents the sealing member from moving in its own axial and radial directions.

An annular step is provided at the middle of the sealing member 181a. The mounting base 186 and the mounting groove 187 in the assembled state have an annular protrusion that matches the annular step, and the side wall of the annular protrusion abuts the sealing member 181a. The two axial side walls of the mounting base are in clearance fit with the sealing member for positioning and installation.

The sealing member 181a includes a connecting tube 188 for connecting the delivery tube or the bending component, and sealing plugs 189 provided at two ends of the connecting tube 188 for sealing. The control handle 100 includes an exhaust joint 101 connected to one of the mounting seats in the first support body. Preferably, the exhaust joint 101 is connected to the fixed seat.

The exhaust joint is connected to an exhaust equipment for exhaust, for example, by using physiological saline. The physiological saline enters the fixed seat through the exhaust joint, and then enters the gap between adjacent tubes through communicated holes opened in the walls of the tubes to discharge the air.

An embodiment of the present disclosure discloses a delivery system, which includes an interventional assembly and a control handle for driving the interventional assembly. For the specific structure of the control handle, please refer to other embodiments.

In one embodiment, the interventional assembly includes a plurality of tubes arranged sequentially from outside to inside, one of two tubes of which is an outer tube and the other is an inner tube. The outer tube is connected to the second support body 120, and the inner tube is connected to the first support body 110.

The distal ends of the outer tube and the inner tube are movable relative to each other. The outer tube covers the inner tube. The outer tube can move axially to cover/expose the interventional device. The inner tube can be bent to change the advancing direction of the interventional assembly.

In one embodiment, the interventional assembly 200 includes at least three tubes arranged sequentially from outside to inside, wherein the outermost and innermost tubes are both connected to the second support body, and at least one middle tube is connected to the first support body.

The outermost and innermost tubes can move axially and are used to cover/expose the distal and proximal ends of the interventional device respectively. The middle tube is used for bending and changing the advancing direction of the interventional assembly.

In one embodiment, the interventional assembly includes a plurality of tubes distributed sequentially from outside to inside. At least one of the delivery tubes 210 is located outside all the bending components 220, the proximal end of which is located at the distal end of the first support body and is connected to the second support body after bypassing the first support body through the transmission member. That is, this delivery tube is the outer sheath 213, and the corresponding structure and effect refer to the above embodiments.

In one embodiment, the interventional assembly 200 includes six tubes: the outer sheath 213, the outer bending tube 223a, the middle bending tube 222a, the inner bending tube 221a, the support tube 212 and the inner core tube 211 arranged sequentially from outside to inside. The delivery tube 210 includes the outer sheath 213, the support tube 212 and the inner core tube 211. The bending component 220 includes the outer bending tube 223a, the middle bending tube 222a and the inner bending tube 221a. The bending component 220 is connected to the first support body 110 and the delivery tube 210 is connected to the first support body.

In another embodiment, the interventional assembly 200 includes the outer sheath 213, the first bending tube 221b, the second bending tube 222b, the third bending tube 223b, the fourth bending tube 224b, the support tube 212 and the inner core tube 211 arranged sequentially from outside to inside. The delivery tube 210 includes the outer sheath 213, the support tube 212 and the inner core tube 211. The bending component 220 includes the first bending tube 221b, the second bending tube 222b, the third bending tube 223b and the fourth bending tube 224b. The bending component 220 is connected to the first support body 110, and the delivery tube 210 is connected to the second support body 120.

In another embodiment, the interventional assembly 200 includes the outer sheath 213, a first bending mechanism 221c, a second bending mechanism 222c, the support tube 212 and the inner core tube 211 arranged sequentially from outside to inside. The delivery tube 210 includes the outer sheath 213, the support tube 212 and the inner core tube 211. The bending component 220 includes the bending mechanisms. The bending component 220 is connected to the first support body 110, and the delivery tube 210 is connected to the second support body 120.

In one embodiment, the interventional assembly 200 includes a third bending mechanism 223c, the outer sheath 213, a fourth bending mechanism 224c, the support tube 212 and the inner core tube 211. The delivery tube 210 includes the outer sheath 213, the support tube 212 and the inner core tube 211. The bending component 220 includes the bending mechanisms. The third bending mechanism 223c is connected to the third support body 130, the fourth bending mechanism 224c is connected to the first support body 110, and the delivery tube 210 is connected to the second support body 120.

In one embodiment, each bending mechanism includes:
two bending tubes inserted one in the other, or
a bending tube and a pull wire which is located inside or outside the bending tube.

The distal ends of the bending mechanisms are movable relative to each other, and the distal ends of the bending mechanism and the delivery tube are movable relative to each other.

In one embodiment, the distal ends of the middle bending tube 222a and the inner bending tube 221a are fixedly connected with each other. The middle bending tube 222a moves at the proximal end thereof for bending in one direction. The distal end of the outer bending tube 223a is fixedly connected adjacent to the distal end of the middle bending tube 222a. The outer bending tube 223a moves at the proximal end thereof for bending in another direction.

Referring to FIG. 32a and FIG. 32b, another embodiment of the present disclosure discloses a control handle for operating an interventional assembly to control an interventional device. The control handle 100 has an axial direction in space, and the two ends in the axial direction are respectively the distal end and the proximal end. In the axial direction, the control handle 100 includes a distal part 11, a main part 12 and a proximal part 13 arranged sequentially from the distal end to the proximal end.

The components of the interventional assembly are divided into three groups, and each group is connected to and controlled by a corresponding part of the control handle 100.

Two adjacent parts of the control handle 100 can be coupled to each other through a locking mechanism, and can drive the connected components of the interventional assembly to move relative to each other after being decoupled.

The distal part 11, the main part 12 and the proximal part 13 can move in the axial direction, at least eliminating the defects due to the limited movement distance and speed resulted from threaded driving structure and the like.

Referring to FIGS. 32a to 32c, in some embodiments, the control handle 100 includes a third support body 130, a first support body 110 and a second support body 120 sequentially arranged in the axial direction from the distal end to the proximal end, wherein the third support body 130 and the first support body 110 are detachably connected to each other.

The mounting seats in the third support body 130 include a first fixed seat 131d and a second movable seat 132d sequentially arranged from the distal end to the proximal end. The mounting seats in the first support body 110 include a third fixed seat 111d and a fourth movable seat 112d sequentially arranged from the distal end to the proximal end. Correspondingly, the first driving member 150a is arranged on the third support body 130, and the second driving member 150b is arranged on the first support body 110. The arrangement of the corresponding bending members can refer to the aforementioned embodiments.

The second support body 120 includes a first section 1201 coupled to the first support body 110, and a second section 1202 detachably connected to the first section 1201. The mounting seats on the first section 1201 include the third movable seat 121 and the second fixed seat (not shown in the figure, as being covered by the second support body). The mounting seats on the second section 1202 include the fourth movable seat 123. The three mounting seats are connected to the outer sheath 213, the support tube 212 and the inner core tube 211 respectively. The fifth driving member 150c and the connecting sleeve 117 are arranged on the first section 1201 from the proximal end to the distal end, and the fourth driving member 150d is arranged on the second section 1202.

The second section 1202 has a first position (refer to FIG. 32a) at which it is connected with the first section 1201, and a second position (refer to FIG. 32b) at which it slides a certain distance proximally, so as to rapidly retract the inner core tube while retaining the threaded driving.

A first locking mechanism is provided between the first section 1201 and the second section 1202, and a second locking mechanism is provided between the first support body 110 and the third support body 130. The structures of the first and second locking mechanisms can refer to the aforementioned embodiments.

Alternatively, referring to FIGS. 32d to 32f, in one embodiment, the first locking mechanism includes:
a connecting section 1111 provided at the proximal end of the first section 1201 for connecting with the second section 1202;
a control button 1301 movably inserted in the second section 1202 and having a limiting member 1302 for engaging with the connecting section 1111, the limiting member 1302 and the connecting section 1111 having a locked state in which they are engaged with each other and an unlocked state in which they are disengaged from each other; and
a reset member 1303 acting on the control button 1301 to reset it so that the first locking mechanism switches to the locked state.

The outer wall of the connecting section 1111 has a lock groove 1112 for engaging with the limiting member 1302. In the locked state, the limiting member 1302 snaps into the lock groove 1112 for axial positioning.

The reset member 1303 is configured so that the initial state of the limiting member 1302 is in the locked state when no external force is applied on the reset member 1303, which can improve the safety. When the operator switches the limiting member 1302 to the unlocked state, he/she only needs to overcome the resilient force of the reset member 1303.

The limiting member 1302 has an annular structure and surrounds the periphery of the connecting section 1111. The inner edge of the annular structure of the limiting member has a locking tooth 1304 for engaging with the lock groove 1112.

The second section 1202 is provided with a fourth housing 1814. The fourth housing 1814 is provided with a guide groove 1815. At least a part of the limiting member 1302 is slidably fitted into the guide groove 1815 to ensure the sliding stability of the limiting member 1302. The fourth housing 1814 consists of two parts snapped with each other and surrounds the periphery of the second section. Alternatively, in one embodiment, part of the fourth housing 1814 is an integral part of the distal end of the second section 1202.

The connecting section 1111 has two symmetrical lock grooves 1112. There are two groups of first locking mechanisms. The limiting members (limiting member 1302a and limiting member 1302b) in these two groups are overlapped side by side. The locking teeth 1304 on the limiting members engage with the respective lock grooves 1112 on the connecting section 1111. Two groups of limiting members overlapped side by side lock the connecting section 1111 from opposite sides, applying more uniformly force and ensuring the locking effect.

The reset members (reset member 1303a, reset member 1303b) and the control buttons 1301 (control button 1301a, control button 1301b) in the two groups are distributed symmetrically, corresponding to the respective limiting members (limiting member 1302a, limiting member 1302b).

The limiting member 1302 and the control button 1301 are formed in one piece. The reset member 1303 is an elastic piece 1306 protruding from the inner wall of the control button 1301, and the limiting member 1302 is provided with an abutting portion 1305a protruding from the outer wall thereof for abutting against the elastic piece 1306 of the other limiting member. Referring to the figure, the abutting portion 1305a abuts the free end of the reset member 1303b, and the abutting portion 1305b abuts the free end of the reset member 1303a.

During operation, the operator can press the control button 1301a and the control button 1301b simultaneously with one hand to move them toward each other. Accordingly, the limiting member 1302a and the limiting member 1302b are unlocked simultaneously, and the reset member 1303b and the reset member 1303a are deformed. When the two control buttons are released, the reset member 1303a and the reset member 1303b act on the limiting member 1302b and the limiting member 1302a respectively, driving the control buttons 1301 to reset.

In one embodiment, the proximal end of the second support body 120 is provided with a support member 1203 extending into the second section 1202, and the fourth movable seat 123 is slidably installed on the support tube 212, to provide support after the second section 1202 and the fourth housing 1814 are separated from each other. The support member 1203 has a tubular structure, and the inner core tube 211 is located in the support member 1203.

The second locking mechanism includes:
a connecting section 1111 provided at the distal end of the first support body 110 for connecting with the third support body 130;
a control button 1301 movably inserted in the third support body 130 and having a limiting member 1302 for engaging with the connecting section 1111, the limiting member 1302 and the connecting section 1111 having a locked state in which they are engaged with each other and an unlocked state in which they are disengaged from each other; and
a reset member 1303 acting on the control button 1301 to reset it so that the second locking mechanism switches to the locked state. The other structures of the second locking mechanism refer to the first locking mechanism.

In one embodiment, a third housing 1813 is arranged around the outer periphery of the third support body. The third housing 1813 is provided with a guide groove 1815. At least a part of the limiting member 1302 is slidably fitted into the guide groove 1815 to ensure that the limiting member 1302 slides stability.

In one embodiment, the first support body 110 is surrounded by a first housing 1811, and the connecting section 1111 can be disposed on the first housing 1811.

Other structures of the control handle refer to the aforementioned embodiments and will not be further described here.

The following embodiments disclose the specific structures of interventional devices or the stents thereof (taking prosthetic heart valve stents for atrioventricular valve as examples). The interventional devices can be operated by using the existing control handles. In order to fully take advantage of the structural characteristics of the interventional devices and meet the delivery requirements, it is preferable to use the control handles according to the above embodiments to operate the interventional devices.

An embodiment of the present disclosure discloses a prosthetic heart valve stent for atrioventricular valve, which has opposite inflow and outflow sides depending on the direction of blood flow. The prosthetic heart valve stent includes an inner frame 300 and an outer frame 400 located around the inner frame 300. The inner frame and the outer frame both have radially deformable and cylindrical mesh structures, and the inner frame 300 encloses and defines a blood flow channel.

The stent further includes anchor arms 500 connected to the inner frame 300 or the outer frame 400. An opening is defined by the anchor arms 500 or between the anchor arms 500 and the outer frame 400 for accommodating and positioning the surrounding tissue.

In use, depending on the lesion site, the prosthetic heart valve stent is provided with corresponding valve leaflets or a covering film(s), thereby forming the interventional device 900. The interventional device 900 can be assembled to any of the interventional assemblies 200 according to the aforementioned embodiments. The interventional device 900 has relative released and compressed states. The compressed state refers to the state in which the interventional device is radially compressed and loaded on the support tube 212 and covered by the outer sheath. The released state refers to the state after manufacturing and before loading, or the free state after being positioned in the human body (without considering the force from the surrounding tissue) or the expanded state as mentioned in the aforementioned embodiments. The process of increasing the radial size is a release process, and the process of reducing the radial size is a compression process. The following descriptions about shapes refer to the released state unless otherwise specified.

Different from the positioning method of piercing the surrounding tissue in the prior art, the present embodiment mainly uses the opening to accommodate part of the surrounding tissue for positioning. For example, the anchor arm 500 is connected to the inner frame 300 and is hook-shaped as a whole. The anchor arms 500 extend from the outflow side and turn outward to the outside of the outer frame 400, with an opening 501 facing the inflow side formed between the anchor arms 500 and the outer frame 400 (see FIG. 33a).

In another example, the anchor arms 500 are connected to the outflow side of the outer frame 400 and extend toward the inflow side and radially outward, with an opening toward the inflow side formed between the anchor arms 500 and the outer frame 400. Taking the tricuspid valve as an example, part of the native valve leaflet 800 is received in the opening to prevent the prosthetic heart valve from being displaced toward the atrium side due to reverse blood flow (FIG. 33b).

In another example, the anchor arms 500 are arranged in pairs. A radially outward opening (i.e., a clamping opening 540 as shown) is formed between the anchor arms 500 in pairs, and a portion of the surrounding native valve annulus is received in the opening for positioning (as shown in FIG. 33c). In order to further lower the safety risks, the end of the anchor arm 500 is improved. For example, the anchor arm 500 has a fixed end 520 connected to the inner or outer frame and an opposing free end 510. The free end 510 has a smooth structure and/or wrapped with a cushioning material. The smooth structure may have a curved edge or the like, and the cushioning material can use a cushioning fabric 650 for wrapping.

The cross section of the atrioventricular valve is irregular. For example, compared with the atrioventricular valve, the annular of the aortic valve is more like a circle, which can better adapt to the profile of the interventional device. For contacting the surrounding tissue more closely to avoid perivalvular leakage and ensuring the sealing effect of the blood flow channel, one embodiment of the present disclosure further provides a prosthetic heart valve stent for atrioventricular valve, which has opposite inflow and outflow sides depending on the blood flow direction. The prosthetic heart valve stent includes an inner frame 300 and an outer frame 400 located around the inner frame 300. Both the inner frame 300 and the outer frame 400 have radially deformable and cylindrical mesh structures. A radial spacing is defined between the inner frame 300 and the outer frame 400, which are connected to each other through fixing ears across the radial spacing. The inner frame 300 encloses and defines the blood flow channel, and the outer frame 400 is supported on the inside of the native valve annulus and native valve leaflets. Therefore, the native valve annulus and native valve leaflets do not directly press the inner frame 300, so the blood flow channel maintains its original preset shape as much as possible, reducing the risk of leaflets failure.

Optionally, in this embodiment, the anchor arms 500 can also be provided for positioning the surrounding tissue. The anchor arms can use existing anchor arms, or the anchor arms according to other embodiments of the present disclosure.

Since there is a radial distance between the inner frame 300 and the outer frame 400, it can also be understood that the inner frame 300 defines a first circumferential surface S1, the outer frame 400 defines a second circumferential surface S2, and the first circumferential surface S1 and the second circumferential surface S2 do not intersect with each other, which is only applicable to the actual product, and does not exclude the situation where the circumferential surfaces extend further in the axial direction and then intersects with each other. The circumferential surfaces (at least in the released state) are radially spaced (as shown in FIG. 34a), so that the outer frame 400 can absorb the deformation of the surrounding tissue better without affecting the shape of the inner frame 300.

The second circumferential surface S2 needs to contact the surrounding tissue as much as possible, so the radial stiffness of the outer frame 400 should be smaller than the radial stiffness of the inner frame 300. To reduce the radial stiffness, the meshes can be designed more sparsely, thinner struts for the mesh can be used, and/or softer materials for the struts can be used. The outer frame 400 has smaller radial stiffness and thus is more compliant and easier to deform than the inner frame 300 when subjected to radial force (as shown in FIG. 34b), which not only prevents the surrounding tissue from pressing the inner frame and can also adapt to the shape of the atrioventricular valve, thereby reducing perivalvular leakage.

The circumferential surfaces of both the inner frame 300 and the outer frame 400 have mesh structures with hollow areas. The circumferential surfaces of the two are defined by the struts of meshes, although some struts may be misaligned with the circumferential surfaces in the radial direction (extending outward or inward). The circumferential surfaces generally have the following functions. For example, the interior of the inner frame 300 is used as the blood flow channel, the inner frame 300 is generally cylindrical, and the first circumferential surface S1 is the outer circumferential surface of the cylinder. The axial middle part of the outer frame 400 mainly functions to abut the inner edge of the native annulus and native leaflets of the tricuspid valve. The second circumferential surface S2 is the inner circumferential surface of the axial middle part. The axial ends of the outer frame 400 may extend radially outward noticeably, in which case the distance between the axial ends and the first circumferential surface S1 becomes larger, so the first circumferential surface S1 and the second circumferential surface S2 do not intersect with each other either.

In order to connect the inner frame 300 and the outer frame 400, part of the struts of the outer frame 400 can serve as fixing ears which may be bent inward from the second circumferential surface S2 until being connected to the inner frame 300. Similarly, part of the struts of the inner frame 300 may also extend radially outward until being connected to the outer frame 400. However, these connection structures are not regarded as extensions or intersections of the first circumferential surface S1 and the second circumferential surface S2, and these sparse connection structures are not rigid connection structures, so the outer frame 400 and the inner frame 300 can still deform independently.

An embodiment of the present disclosure further discloses a prosthetic heart valve stent for atrioventricular valve, which has opposite inflow and outflow sides depending on the direction of blood flow. The prosthetic heart valve stent includes an inner frame 300 and an outer frame located around the inner frame 300. The inner frame 300 and the outer frame 400 both have radially deformable and cylindrical mesh structures. The inner frame 300 and the outer frame 400 are formed in separate pieces which are fixedly connected with each other at connection portions, and the connection portions are flexible. This embodiment may further include the anchor arms 500 for positioning the surrounding tissue. The anchor arms can use existing anchor arms, or the anchor arms according to other embodiments of the present disclosure.

The flexible connection portions between the inner frame 300 and the outer frame 400 can further absorb the deformation of the outer frame 400 and avoid affecting the blood flow channel defined by the inner frame 300. Specifically, flexible elements can be used for binding and fixing the connection portions. When the outer frame 400 is pressed by the surrounding tissue and is partially deformed or tilted relative to the inner frame 300, the flexible connection portion will absorb the stress to prevent the inner frame 300 from being affected.

Further, when there is a radial gap between the inner frame 300 and the outer frame 400 with the flexible connection portions, the fixing ears 460 can be bound together with flexible elements 570. For example, a plurality of fixing ears 460 can be provided in the circumferential direction between the inner frame 300 and the outer frame 400. The fixing ears 460 can be formed in at least one of the following ways:
the fixing ears 460 and the inner frame 300 being formed in one piece and bent radially outward until being connected to the outer frame 400 (as shown in FIG. 34c);
the fixing ears 460 and the outer frame 400 being formed in one piece and bent radially inward until being connected to the inner frame 300 (as shown in FIG. 34d); and
the fixing ears 460 being divided into two groups of fixing ears 460, which are formed with the inner frame 300 and the outer frame 400 in separate pieces respectively and are bent toward each other in the radial direction and connected to each other (as shown in FIG. 34e).

The fixing ear is rod-shaped and has a threading hole at the end thereof for threading the flexible element.

An embodiment of the present disclosure further discloses a prosthetic heart valve stent for atrioventricular valve, which includes an outer frame 400 and an inner frame 300 surrounded by the outer frame 400. Both the inner frame and the outer frame have radially deformable and cylindrical mesh structures. The inner frame encloses and defines the blood flow channel. The prosthetic heart valve stent has an inflow side 910 and an opposing outflow side 920 depending on the direction of blood flow. The interventional device further includes anchor arms 500. One end of the anchor arm is connected to the inner frame, the other end extends outward and exceeds the outer frame, and the exceeding part beyond the outer frame is bent toward the inflow side for positioning the surrounding tissue.

Refer to FIG. 35a to FIG. 35d and FIG. 43, the cylindrical mesh structure has an axial direction and a radial direction. The cylindrical mesh structures allow the inner frame and the outer frame to deform respectively. Taking the inner frame 300 as an example, the side radially close to the blood flow channel is the inner side 930 and the side away from the blood flow channel is the outer side 940. The outer frame 400 has inner and outer sides in the same direction as the inner frame. The cylindrical mesh structure is generally shaped as a solid of revolution, but the generatrix is not strictly limited to a straight line. It may alternatively have a diameter-reduced waist or a cone, for example.

The outer frame 400 and the inner frame 300 surrounded by the outer frame 400 can limit the movement between the two, for example, limit the axial and/or radial movement between the two. After the two engaged with each other or one of them is engaged with the surrounding tissue, the inner frame 300 would have a stable working environment, and the outer frame can absorb the deformation of the surrounding tissue to prevent the blood flow channel from being pressed and thus avoid affecting the sealing effect.

The anchor arm 500 generally has a hook shape, for partially or completely engaging with the surrounding tissue. The anchor arm hooks the native valve leaflet to avoid the displacement of the interventional device under the action of blood flow. When the anchor arms 500 are provided on the inner frame 300, the inner frame 300 can be directly engaged with the surrounding tissue, thereby more effectively preventing the inner frame from shifting. The other ends of the anchor arms extending outward beyond the outer frame 400 means that the radial size of that part of the anchor arms 500 is larger than the maximum radial size of the outer frame, or is larger than the radial size of part of the outer frame.

In addition, part of the outer periphery of the outer frame is also positioned against the surrounding tissue through friction or other means.

In this embodiment, the anchor arm 500 extends outward and exceeds the outer frame 400 by extending outward through a structural gap of the outer frame, and/or extending outward and bypassing the outer frame from the outflow side of the outer frame.

In the released state, the structural gaps are enlarged, and the anchor arms 500 gradually extend outward through the corresponding structural gaps. Alternatively, the anchor arms 500 can extend from the inner frame 300 toward the outflow side 920 until crossing the outer frame 400 and then being bent and extending toward the inflow side 910 to form hooks with an opening 501. The opening 501 faces the inflow side 910 for receiving the native valve leaflet or other surrounding tissue. In this embodiment, the anchor arm 500 extends from the edge of the outflow side 920. Preferably, the hook is arc-shaped to avoid scratching the surrounding tissue.

The inflow side 910 of the outer frame 400 has positioning structures 410 extending radially outward.

The positioning structures 410 are radially thicker than the remaining part of the outer frame 400 and can be positioned against the native valve annulus in the axial direction of the interventional device. In another embodiment, the outflow side 920 of the outer frame 400 is radially flared for engaging with the surrounding tissue to further ensure the positioning effect.

In one embodiment, the positioning structures 410 include a plurality of V-shaped struts 430 arranged in the circumferential direction of the outer frame. The vertex of the V shape faces the inflow side 910, and the two arms of the V shape are connected to the remaining part of the outer frame 400. Adjacent V-shaped struts 430 are connected to each other in the circumferential direction of the outer frame. That is, adjacent two arms of adjacent V-shaped struts are connected to each other. The quantity of V-shaped struts 430 is in the range of 6 to 12.

Referring to FIG. 44, the V-shaped strut 430 has an extension turning outward means that it has an extension extending radially outward. An angle γ is defined between the extension of the V-shaped strut 430 and the axis 950, and the angle γ ranges from 15 degrees to 90 degrees. For example, the angle γ ranges from 15 degrees to 60 degrees. For example, the angle γ can be 30 degrees as shown in the figure.

The inner and outer frames and thus the interventional device can be engaged with the interventional assembly 200, for example, through a connection portion(s) 320. For example, in one embodiment, the inflow edge of the positioning structure 410 can be provided with a connecting ear 420 for engaging with the interventional assembly 200. The connecting ear 420 can be a part of the outer frame or a separate piece connected to the outer frame by existing technologies, such as by welding. The connecting ear 420 is a specific implementation of the connection portion 320 and is used for assembling the interventional device and the interventional assembly 200.

The connecting ear 420 is configured to engage with the interventional assembly 200 to prevent the interventional device in the compressed state from moving axially, so as to facilitate loading and delivery. The connecting ear 420 can be inserted into the interventional assembly 200 or bound to the interventional assembly 200 by winding wire(s). For example, the connecting ear 420 in this embodiment can be provided with an eyelet 421, and the interventional assembly 200 is provided with a lock wire passing through the eyelet 421. The lock wire is operable. In release, the lock wire is operated to disengage from the eyelet 421. Alternatively, the interventional assembly can be provided with a boss for engaging with the eyelet 421. The eyelet 421 may have a closed structure formed by opening or a semi-closed structure formed by the inner frame or the outer frame itself.

Alternatively, the support tube 212 of the interventional assembly is provided with a mounting head, and the outer peripheral surface of the mounting head is provided with a positioning post to be inserted into the eyelet 421. Alternatively, the connecting ear 420 is configured as a T-shaped block, the support tube 212 of the interventional assembly is provided with a mounting head, and a T-shaped groove that matches the T-shaped block is defined on the outer peripheral surface of the mounting head. In the compressed state, the T-shaped block is received in the T-shaped groove. During the release process, the T-shaped block automatically moves radially outward until being disengaged from the T-shaped groove.

Alternatively, the inner frame 300 can also be provided with a connecting ear(s) 420, the specific structure and shape of which can refer to the connecting ear(s) 420 on the outer frame 400. Depending on the loading and release sequence, the connecting ear(s) 420 can be arranged on the inflow side 910 and/or the outflow side 920.

Referring to FIG. 38a to FIG. 3d and FIG. 45, the angle between the extension of the connecting ear 420 and the axis of the outer frame is α, the angle α satisfies 0 degree =5 α < 30 degrees, and the end of the connecting ear is inclined radially inward. Preferably, 0 degree ≤ α < 15 degrees.

The extension of the connecting ear 420 refers to an imaginary connection line connecting the end point of the connecting ear 420 and the connection point of the connecting ear 420 with the outer frame. The length of the connecting ear 420 is L9, and L9 satisfies 3.5 mm < L9 < 6.5 mm. The length of the connecting ear 420 refers to the length from the end point of the connecting ear 420 to the connection point of the connecting ear 420 with the outer frame, which may be a straight line distance or a curve distance.

The positioning structures of the outer frame are directly engaged with the surrounding tissue, while the inner frame is engaged with the surrounding tissue through the anchor arms. The specific structure of the anchor arm is explained in detail below.

First, the inner frame 300 and the outer frame 400 have mesh structures. The outer frame 400 includes cells 440, and the inner frame 300 includes cells 310. The anchor arm 500 has a fixed end connected to the inner frame 300 or the outer frame 400 and an opposing free end. In one embodiment, the anchor arm 500 has a single-strut structure or multi-strut structure.

The single-strut structure refers to FIGS. 35a to FIG. 37d. The anchor arms 500 are independent of each other and the fixed ends thereof are connected to the outflow side 920 of the inner frame 300. The fixed end can be connected in various manners. For example, as shown in FIG. 37a, the fixed end of the anchor arm 500 is connected to the connection of the adjacent cells 310. For example, as shown in FIG. 35a and FIG. 36a, the fixed end of the anchor arm 500 is connected to the outflow edge of the cell 310. In either case, the free end of the anchor arm 500 extends through the gap between adjacent cells 440 on the outer frame 400.

The multi-strut structure refers to FIGS. 38a to FIG. 40c. The anchor arm 500 includes at least two struts. The fixed ends of the struts diverge from each other and the free ends converge and fix each other, so that the anchor arm 500 is V-shaped. The vertex is the free end. The fixed end is connected to the connection between adjacent cells 310, and the two struts respectively extend through the gaps between adjacent cells 440 on the outer frame 400.

The anchor arm 500 and the inner frame 300 are formed in one piece or in separate pieces which are fixedly connected with each other, for example, by welding or binding, and the connection portions are flexible. In a preferred embodiment, the anchor arms 500 and the inner frame 300 are fixed together by binding wire(s), which provides certain flexibility for the connections of the anchor arms 500 and the inner frame 300.

In one embodiment, referring to FIGS. 37a to 37d, one or more eyelets are defined on the anchor arm 500, which can be used to engage with pull wire(s) to control the release process or for retrieval (if necessary). In this case, the anchor arm 500 also serves as the connecting ear 420, and is provided with a corresponding eyelet 421, in which a metal material can be embedded as a marker for surgery to assist in engagement with the native leaflets. Optionally, soft fabric can be sewn on the eyelet 502 to prevent scratching the surrounding tissue. Since a plurality of anchor arms 500 are arranged in the circumferential direction, at least one eyelet is provided at the end of the anchor arm 500.

The periphery of the eyelet has a closed structure or a semi-open structure. For example, referring to FIG. 37c, the single-strut structure of the anchor arm is provided with an eyelet with a closed structure. Referring to FIGS. 38a to 40d, the multi-strut structure formed by two struts the ends of which are converged is provided with a semi-open structure.

In FIGS. 35a to 40d, the anchor arm 500 in the compressed state is turned over and straightened on one axial side of the inner frame. For example, the anchor arm 500 is straightened at the inflow side 910 of the inner frame.

In one embodiment, an axial limiting structure is provided between the inner frame 300 and the outer frame 400, to prevent undesired misalignment between the inner frame and the outer frame and also connect the inner frame 300 and the outer frame 400. For example, the axial limiting structure can include limiting struts 710 provided on at least one of the inner frame 300 and the outer frame 400 or the fixing ears 460 in the following embodiment. The limiting struts extend from one of the inner frame 300 and the outer frame 400 toward the other of the inner frame 300 and the outer frame 400 to interfere with the other of the inner frame 300 and the outer frame 400, or interfere with the limiting struts on the other of the inner frame 300 and the outer frame 400.

The interference may be the limiting struts of the inner frame 300 and the outer frame 400 interfering with each other, or the limiting strut 710 interfering with the inner frame 300 and/or the outer frame 400, to provide restriction on the inner frame 300 and the outer frame 400 in the axial and/or radial directions. For example, the limiting strut 710 on the inner frame 300 can extend into the structural gap of the outer frame 400 and interacts with the edge of the structural gap. Optionally, the corresponding limiting struts of the inner and outer frames are fixedly connected to each other, for example, by welding, etc. It should be noted that the fixed connection of the limiting struts 710 to each other does not affect the compression of the inner and outer frames.

The limiting strut 710 on the inner frame extends outward relative to the first circumferential surface, and the limiting strut 710 on the outer frame extends inward relative to the second circumferential surface, to ensure the radial distance between the first circumferential surface and the second circumferential surface.

The inner frame 300 and the outer frame 400 are formed in separate pieces which are fixedly connected with each other, and the connection portions of them are located at the outflow side 920, which are rigid or flexible. Preferably, the inner frame 300 and the outer frame 400 are fixed together by binding.

In one embodiment, the outflow sides of the inner frame 300 and the outer frame 400 are provided with fixing ears 460 for fixing the two frames. The fixing ear 460 is also a specific implementation of the connection portion 320 and is used for fixed connection of the inner and outer frames. Referring to the connecting ear 420 mentioned above, the fixing ears 460 can be welded and fixed to each other, or may be provided with eyelets 461 and then bound and fixed together. The periphery of the eyelet 461 has a closed or semi-open structure. Alternatively, the fixing ears 460 can be provided on the inflow side 910.

The outflow side of the outer frame 400 is axially aligned with the outflow side of the inner frame 300, which facilitates the connection of the fixing ears on the outflow sides.

Both the inner frame 300 and the outer frame 400 have mesh structures. The outer frame 400 includes one or more circles of cells 440 in the axial direction, and the quantity of cells in each circle is in the range of 9 to 12. The inner frame 300 includes one or more circles of cells 310 in the axial direction. The circles of cells 310 have the same radial dimension, that is, the inner frame 300 is generally shaped as a straight cylinder. The circle quantity, cell quantity and cell shape directly affect the structural strength of the inner and outer frames. The cell 310 (440) is roughly diamond-shaped.

Regarding the circle quantity and the cell quantity, for example, as shown in FIG. 48d, the outer frame 400 includes two circles of cells 440 in the axial direction, and the quantity of cells in each circle is 9. The inner frame 300 includes three circles of cells 310 in the axial direction, and the quantity of cells in each circle is 9. As another example in FIG. 37d, the outer frame 400 includes two circles of cells 440 in the axial direction, and the quantity of cells in each circle is 12. The inner frame 300 includes four circles of cells 310 in the axial direction, and the quantity of cells in each circle is 12. For example, as shown in FIG. 42d, the outer frame 400 includes two circles of cells 440 in the axial direction, and the quantity of cells in each circle is 12. The inner frame 300 includes five circles of cells 310 in the axial direction, and the quantity of cells in each circle is 12.

For example, as shown in FIG. 38a to FIG. 40d and FIG. 44, in some embodiments, the angle γ of the V-shaped strut 430 on the inflow side 910 of the outer frame is in the range of 70 degrees to 80 degrees, preferably 72 degrees, which can improve the positioning effect of the outer frame 400 on the surrounding tissue.

In order to allow the V-shaped strut 430 to bend to a great extent, in one embodiment, referring to FIGS. 39a to 40e, in the circumferential direction of the outer frame, adjacent V-shaped struts 430 are arranged at intervals, the circumferential width of the interval is M1, the central angle corresponding to M1 is β, and the central angle β is in the range of 30 degrees to 60 degrees. In the circumferential direction of the outer frame, the sum of the central angles corresponding to all M1 is 360 degrees. Preferably, the V-shaped struts 430 are evenly spaced from each other in the circumferential direction of the outer frame. The quantity of V-shaped struts in this embodiment is 6, and the corresponding central angle β is 60 degrees. M1 is at least 15 mm, preferably not less than 20 mm.

In the circumferential direction of the outer frame, between adjacent V-shaped struts is a release opening 450 for releasing connection therebetween. Referring to FIG. 43, in the compressed state, the length of the outer frame is L1, the length of the release opening is L2, and L1 : L2 = X, wherein X is in the range of 2.0 to 3.0. In the circumferential direction of the outer frame, between adjacent V-shaped struts is a release opening 450 for releasing connection therebetween. In the released state, the length of the outer frame is L3, the length of the release opening is L4, L3 : L4 = Y, and Y is in the range of 1.5 to 2.5 (as shown in FIG. 40d). The release opening 450 can be understood as the gap between the two V-shaped struts 430, which eliminates or reduces the adverse effect due to deformation of adjacent V-shaped struts so that the V-shaped struts can adapt to the surrounding tissue better.

L1 is the maximum axial length of the outer frame in the compressed state, and L2 is the projection distance in the axial direction of an imaginary connection line connecting the outflow edge of the outer frame and the connection of the cells 440. L3 is the maximum axial length of the outer frame in the released state, and L4 is the projection distance in the axial direction of an imaginary connection line connecting the inflow edge of the outer frame and the connection of the cells 440.

As mentioned above, the inner frame 300 and the outer frame 400 are formed in separate pieces which are fixedly connected with each other. In another embodiment, as shown in FIG. 41a to FIG. 41b, the inner frame 300 and the outer frame 400 are formed in one piece, which are connected to each other on the outflow side, for example, by a plurality of arc-shaped struts arranged at intervals in the circumferential direction of the inner frame. One end of each arc-shaped strut is connected to the inner frame and the other end is connected to the outer frame.

The outer frame 400 can use other implementations. In this embodiment, the outer frame 400 is further improved, which does not use the circumferentially complete annular structure, but includes a plurality of units which are spaced apart in the circumferential direction. The specific structure of each unit is not strictly limited. For example, it may use various forms of meshes or struts. In this embodiment, each unit is simplified and consists of only one cell viewed in the axial direction of the outer frame, and the cell is open toward the outflow side, which can further release the restriction between adjacent cells to absorb the deformation of the surrounding tissue.

Both the interior of each cell and the area between two adjacent cells can be regarded as avoidance areas. The inner frame has a plurality of anchor arms, and each anchor arm extends to the outside of the outer frame through a corresponding avoidance area. The circumferential widths of the avoidance areas of the outer frame can be the same.

In the circumferential direction of the outer frame, the two sides of the cell 440 are respectively struts 441 and 442. Each strut extends roughly in the axial direction of the outer frame. The ends of the struts 441 and 442 facing the outflow side is connected to the corresponding arc-shaped struts, and the ends of the struts 441 and 442 facing the inflow side is close to each other and connected. To connect the ends of the struts 441 and 442 facing the inflow side, the struts 441 and 442 can each have a smooth turning (such as an S-shaped turning) for safety and loading.

The intersection of the strut 441 and the strut 442 is roughly V-shaped. The V-shaped portions extend outward in the radial direction of the outer frame and serve as the positioning structure 410 of the outer frame 400.

In this embodiment, in the radial direction of the outer frame, the outer edge of the positioning structure 410 exceeds the outer edge of the anchor arms, that is, the diameter of the circumscribed circle of the positioning structures 410 is greater than the diameter of the circumscribed circle of the anchor arms. In the axial direction of the outer frame, the anchor arms are located between the two ends of the outer frame.

An embodiment of the present disclosure further provides a prosthetic heart valve for atrioventricular valve, including the stent of the above embodiment and valve leaflets 610 (two or three leaflets 610) provided in the inner frame 300 for controlling the opening degree of the blood flow channel 301. The prosthetic heart valve for atrioventricular valve further includes a sealing film 620, which is arranged in the radial gap between the inner frame 300 and the outer frame 400 in a sealed connection. Referring to FIGS. 46a to 46e, the leaflets 610 are arranged inside the inner frame 300 to simulate the native leaflets. The sealing film 620 mainly functions to prevent blood from flowing back through the radial gap. The leaflets 610 and the sealing film 620 can be fixedly connected to the cells of the corresponding frame by sewing. The sealing film 620 can be located on the inflow and/or outflow side. As shown in the figure, it is located on the inflow side 910, and thus the radial gap between the inner frame 300 and the outer frame 400 is open toward the outflow side 920. Referring to FIGS. 46b to 46e, in some embodiments, the prosthetic heart valve further includes a first covering film 630, and the first covering film 630 is connected to the inner side 930 and/or the outer side 940 of the inner frame 300. The first covering film 630 can block or reduce blood flowing into the radial gap between the inner frame 300 and the outer frame 400 from the structural gap of the inner frame 300, and can also connect the sealing film 620 and the leaflets 610.

In some embodiments, the prosthetic heart valve further includes a second covering film 640 connected to the inner side 930 and/or the outer side 940 of the outer frame 400, which can block or reduce blood flowing to the outside 940 of the interventional device from the structural gap of the outer frame 400.

Depending on the location of the sealing film 620, the corresponding side edges of the first covering film 630 and the second covering film 640 and the sealing film 620 can be formed in one or separate pieces to achieve good sealing effect. The valve leaflets, sealing film and covering film can be made of the same or different materials (such as biological or synthetic materials, etc.). The sealing film 620 can be loosened to avoid tightening, so as to reduce the impact of the deformation of the outer frame 400 on the inner frame 300.

The release process of the interventional device after it is delivered into the human body through the interventional assembly 200 will be described below.

In one embodiment, in the compressed state, the anchor arms 500 are located on one axial side of the inner frame 300.

In one embodiment, during the switching process from the compressed state to the released state, the release timing of the inflow side 910 and the outflow side 920 is T, and the release timing of the fixed end 520 and the free end 510 of the anchor arm 500 is T or -T.

For example, if the inflow side 910 is released first, and the outflow side 920 is released later, the timing is considered to be T. If the fixed end 520 is released first, and the free end 510 is released later, the timing is also considered to be T. On the contrary, if the fixed end 520 is released later, and the free end 510 is released first, the timing is considered to be -T.

Depending on the arrangement of the anchor arms, an embodiment of the present disclosure further provides another prosthetic heart valve stent for atrioventricular valve, including an outer frame 400 and an inner frame 300 surrounded by the outer frame 400. Both the inner frame and the outer frame have radially deformable and cylindrical mesh structures. The inner frame encloses and defines the blood flow channel. The interventional device 900 has an inflow side 910 and an opposing outflow side 920 depending on the direction of blood flow. The prosthetic heart valve stent further includes anchor arms 500 which are arranged in pairs and connected to the outer frame 400. The anchor arms of the same pair are relatively arranged in the axial direction of the outer frame, and a clamping opening 540 is defined between the anchor arms of the same pair that is opened radially outward for positioning the surrounding tissue.

The differences of the following embodiments from the aforementioned embodiments will be explained.

Referring to FIG. 47a to FIG. 47d, the anchor arms 500 of the same pair extend generally radially outward, and can also extend in the axial direction. The anchor arms adjacent to the inflow side further extend axially toward the inflow side, and the anchor arms adjacent to the outflow side further extend axially toward the outflow side, thereby forming the clamping opening 540.

The clamping opening 540 acts on the surrounding tissue for positioning. Different from the aforementioned positioning method in which the anchor arms 500 hook the native valve leaflet, in this embodiment, after the anchor arms engage with the surrounding tissue, for example, the surrounding tissue is received in the clamping opening 540, or the anchor arms 500 of the same pair abut against the surrounding tissue, thereby preventing the outer frame 400 from being displaced under the action of blood flow.

The anchor arms 500 being arranged in pairs only means that the anchor arms in the same pair are arranged sequentially in the axial direction. The shapes of the two anchor arms in the same pair are not strictly required to be the same. Even if the structures of the two anchor arms in the same pair are the same, they are not strictly required to be symmetrical.

In the same pair of anchor arms 500, the upper arm 541 is located on the inflow side 910, and the lower arm 542 is located on the outflow side 920. The upper arm 541 and the lower arm 542 each have a single-strut or multi-strut structure. The inflow side 910 of the outer frame 400 has positioning structures 410 extending radially outward, which also serve as the upper arms 541. The quantity of anchor arms 500 is in the range of 9 to 12.

The anchor arm 500 has a fixed end 520 connected to the outer frame 400 and an opposing free end 510. The fixed ends 520 of the upper arm 541 and the lower arm 542 are adjacent to or axially spaced apart from each other.

Referring to FIG. 52, the upper arm 541 and the lower arm 542 extend radially outward. The angles formed between the extensions of the upper arm 541 and the lower arm 542 and the axial direction are angle γ₁ and angle γ₂ respectively, where angle γ₁ is in the range of 30 degrees to 90 degrees, for example, in the range of 45 degrees to 90 degrees, and the angle γ₂ is in the range of 60 degrees <γ₂ < 90 degrees.

The opening angle Σ of the clamping opening 540 at least satisfies 60 degrees < Σ < 120 degrees. The sum of the opening angle Σ and the angles γ₁ and γ₂ is 180 degrees. The axial span M2 of the clamping opening 540 is in the range of 10 < M2 < 30 mm. M2 refers to the distance between the ends of the upper and lower arms.

Referring to FIG. 48a to FIG. 48d and FIG. 53, taking the upper arm 541 as an example, the upper arm 541 includes a first strut 546 and a second strut 547. The two struts are symmetrically arranged and have main extensions. This extension can extend in an approximately straight line. The extensions of the two struts form an angle θ therebetween, and the angle θ is in the range of 30 degrees to 180 degrees. The structure of the lower arm 542 is similar to that of the upper arm 541.

The anchor arms 500 on the outer frame 400 function to engage with the surrounding tissue such as the native valve leaflets and annulus. The inner frame 300 is connected to the outer frame 400 to prevent displacement.

In some embodiments, an axial limiting structure is provided between the inner frame 300 and the outer frame 400. The function and specific structure of the axial limiting structure refer to the aforementioned embodiments and will not be described here again.

In one embodiment, the inner frame 300 and the outer frame are formed in one piece or separate pieces that are fixedly connected with each other. The inflow sides 910 and/or the outflow sides 920 of the inner frame 300 and the outer frame 400 are provided with fixing ears 460 that are fixedly connected to each other. The inflow sides 910 of the inner frame 300 and/or the outer frame 400 are provided with a connecting ear(s) 420 for engaging with the interventional assembly. The fixation method for the separate pieces and the specific structures of the connecting ear 420 and the fixing ear 460 refer to the aforementioned embodiments.

In one embodiment, both the inner frame 300 and the outer frame 400 have mesh structures. The inner frame 300 and the outer frame 400 each include one or more circles of cells 440 in the axial direction, and the quantity of cells 440 in each circle is in the range of 9 to 12. For details, reference may be made to the aforementioned embodiments.

Referring to FIGS. 48a to 48d, in one embodiment, the outer frame 400 only includes one circle of cells and the circle of cells 440 has a semi-closed structure that is open toward the outflow side.

Each cell 440 includes two connecting struts 545 on two sides in the circumferential direction of the outer frame. Each connecting strut extends roughly in the axial direction of the outer frame. Each connecting strut is shared by two adjacent cells. In general, the outer frame 400 includes a plurality of connecting struts 545 distributed at intervals. The connecting struts 545 are connected with upper arms 541 adjacent to the inflow side 910 for connection of the connecting struts 545. The inflow side of the cells 440 can be regarded as being closed by the upper arms 541. The ends of the connecting struts 545 adjacent to the outflow side 920 are independent of each other to form an open structure. The projection lengths of different connecting struts 545 on the axis of the outer frame are the same or different. For example, the projection length L8 is in the range of 15 mm to 25 mm. The longer connecting strut can extend further toward the outflow side than the other connecting struts, to serve as a connecting ear, if necessary.

The main body of the inner frame 300 has a straight cylindrical shape, and the cells of the inner frame 300 are provided with fixing ears 460 extending outward which also facilitate maintaining the radial gap between the first circumferential surface and the second circumferential surface. The connecting strut 545 is provided with a connection portion 320 that is connected and fixed with the corresponding fixing ear 460.

In this embodiment, the outer frame 400 includes a circle of cells, and the cell includes two connecting struts 545 and an upper arm 541. The ends of the two connecting struts 545 are connected to the two struts of the upper arm 541 at the fixed ends. The lower arms 542 are connected and fixed on the connecting struts 545.

If the corresponding angle γ₁ and the angle γ₂ of the upper arm 541 and the lower arm 542 are relatively large, the upper arm 541 and the lower arm 542 would primarily extend in the radial direction, which may easily scratch the tissue.

In order to solve the above problem, in one embodiment, the ends of the upper arm 541 and the lower arm 542 extend toward the inflow side 910 and the outflow side 920 respectively, to avoid scratching the surrounding tissue. Referring to FIGS. 48a to 48d and FIG. 54, for example, the upper arm 541 has a first section 543 that primarily extends radially outward and a second section 544 that primarily extends axially, and the end of the second section 544 is directed toward the inflow side 910. The second section 544 generally extends in a straight line, and the straight line and the axial direction form an angle δ which is in the range of 15 degrees to 25 degrees.

As another example, as shown in FIGS. 49a to 51d and 55, the second section 544 extends inward, with an obvious turning point 549 between the second section 544 and the first section 543. The turning point is the outermost end point in the radial direction. The first section 543 is smoothly transitioned to the second section 544 at the turning point to prevent the turning point from scratching the surrounding tissue. Some or all of the second sections 544 are provided with connecting ears 420 at the ends thereof for easy assembly.

The end of the lower arm 542 further bends and extends toward the outflow side 920. The lower arm 542 may have a similar structure to the upper arm 541, except that its end is directed toward the outflow side 920. As shown in FIG. 55, taking the upper arm 541 as an example, the upper arm 541 has a turning point 549 which is curved sharply. The line connecting the turning point 549 to the end and the axial direction form an angle µ, and the angle µ is in the range of 0 to 30 degrees. It can be concluded that two sides of the turning point 549 correspond to the first section 543 and the second section 544 respectively.

To allow the first section 543 and the second section 544 of the upper arm 541 to extend differently, referring to FIGS. 49a to 49d, in one embodiment, a connecting strut 545 is connected between the upper arm 541 and the lower arm 542. The connecting strut 545 can be understood as a strut of the frame. The connecting strut 545 extends radially outward toward the inflow side and serves as the first section 543, so that the second section 544 can extend radially inward. The two ends of the first strut 546 and the second strut 547 are gathered together or bent to connect with the connecting strut 545, and the bending angle is relatively great, close to 90 degrees. The connecting strut 545 is provided with an eyelet 548 for sewing fabric.

In the frame shown in FIGS. 51a to 51d, the upper arm 541 and the lower arm 542 are each V-shaped. As for the lower arm 542, the V shape thereof can be regarded as an open structure. Referring to FIG. 56a and 56b, in another embodiment, the lower arm 542 includes two V-shaped struts and connecting struts 554 that connect the two corresponding struts of the two V-shaped struts respectively. The two V-shaped struts and two connecting struts 554 enclose a closed space 553, that is, the lower arm itself encloses the closed area. The two V-shaped struts are defined as the first layer 551 and the second layer 552 in sequence from the outflow side 920 to the inflow side 910 in the axial direction. The first layer 551 extends radially outward relative to the second layer 552. A common connecting strut 554 is shared between adjacent lower arms 542. The end of the connecting strut 554 connected to the second layer 552 is configured to connect with the corresponding connecting strut 545 on the outer frame 400.

In this embodiment, all the lower arms are distributed in the circumferential direction and connected to form an annular structure, and the annular structure and the outer frame are formed in separate pieces and fixedly connected to each other.

As an alternative to the upper arm 541 and the lower arm 542 using the V-shaped strut structure composed of two struts, in another embodiment, for example, as shown in FIG. 50a to FIG. 50d, the lower arm 542 uses a single strut structure.

In this embodiment, the upper arm 541 is V-shaped. The vertex of the V-shape is the free end 510a, and the two sides of the V shape are two fixed ends 520a. The lower arm 542 uses a single strut structure, wherein one end of the single strut is a fixed end 520b which is connected to the outer frame 400 adjacent to one of the fixed ends 520a of the upper arm 541. The other end of the single strut is a free end 510b. Preferably, the free end 510b of the lower arm 542 is provided with an eyelet, which can be used to sew the cushioning fabric 650.

In one aspect, the anchor arms 500 arranged in the pair as mentioned above can be understood as including a V-shaped upper arm 541 and two corresponding single-strut lower arms 542, and two adjacent upper arms 541 share one lower arm 542. In another aspect, it can be understood as that one V-shaped upper arm 541 corresponds to a single-strut lower arm 542.

The outer frame in this embodiment can use the outer frame structure shown in FIG. 48a to FIG. 48d, wherein each connecting strut 545 is connected to a lower arm 542, and the lower arm 542 extends radially outward from the fixed end toward the outflow side 920. In order to avoid scratching the surrounding tissue, the part of the lower arm 542 close to the free end is basically parallel to the axial direction of the outer frame and generally directed toward the outflow side.

In another embodiment, referring to FIGS. 51a to 51d, the axial projection length of the section of the anchor arm 500 adjacent to the inflow side 910 is L7, and L7 is in the range of 3 mm to 12 mm. It can be seen that the section of the anchor arm 500 adjacent to the inflow side 910 is the upper arm 541, and the axial projection lengths L7 of the upper arms can be the same or different. The upper arm 541 with a relatively long projection length L7 also serves as the connecting ear 420.

The release timings of the fixed ends 520 and the free ends 510 of the upper arm 541 and the lower arm 542 are reversed. For example, the inflow side 910 is released first and the outflow side 920 is released later, in which case the free end of the upper arm 541 is released first and the fixed end thereof is released later, and then the fixed end of the lower arm 542 is released first and the free end thereof is released later.

Based on the anchor arms formed as a separate annular structure, the present disclosure further discloses a prosthetic heart valve stent for atrioventricular valve, which has opposite inflow and outflow sides depending on the direction of blood flow. The prosthetic heart valve stent includes an inner frame 300 and an outer frame 400 located around the inner frame 300. Both the inner frame and the outer frame have radially deformable and cylindrical mesh structures, and the inner frame 300 encloses and defines the blood flow channel.

The prosthetic heart valve stent further includes anchor arms 500 distributed in the circumferential direction for positioning and engaging with the surrounding tissue. The anchor arm 500 has a fixed end 520 connected to the inner frame or the outer frame and an opposing free end 510. All anchor arms 500 are connected to each other at the fixed ends 520 thereof to form an annular structure, and are connected to the inner frame 300 or the outer frame 400 through the annular structure.

As shown in FIG. 56c, the anchor arms 500 include pairs of upper arms 541 and lower arms 542 relatively arranged in the axial direction, and the pairs of upper arms 541 and lower arms 542 are distributed in the circumferential direction. All the upper arms 541 and lower arms 542 are sequentially connected in the circumferential direction into one piece, i.e., forming the annular structure. The specific structures of the upper arm 541 and the lower arm 542 can refer to other embodiments of the present disclosure.

Depending on the arrangement of the anchor arms, an embodiment of the present disclosure further provides a prosthetic heart valve stent for atrioventricular valve, including an outer frame 400 and an inner frame 300 surrounded by the outer frame 400. The inner frame 300 and the outer frame 400 both have radially deformable and cylindrical mesh structures. The inner frame 300 encloses and defines the blood flow channel. The prosthetic heart valve stent has an inflow side 910 and an opposing outflow side 920 depending on the blood flow direction. The prosthetic heart valve stent further includes anchor arms 500. One end of the anchor arm is connected to the outflow side 920 of the outer frame 400 and the other end extends outside the outer frame 400 toward the inflow side 910 for positioning and engaging with the surrounding tissue.

Referring to FIGS. 57a to 57d, the anchor arm 500 is connected to the outer frame 400, and generally extends toward the inflow side 910 and has a hook shape for hooking the native valve leaflet. Different from the aforementioned embodiments, the anchor arm 500 is configured to abut the native valve leaflet so that the outer frame 400 acts on the surrounding tissue to prevent the outer frame 400 from being displaced under the action of blood flow. The inner frame 300 is engaged with the outer frame 400 and is indirectly prevented from being displaced. Similarly, the anchor arm 500 also extends radially outward to facilitate the hooking.

The inflow side 910 of the outer frame 400 has positioning structures 410 extending radially outward.

The positioning structures 410 are radially thicker than the remaining part of the outer frame 400 and can be positioned against the native valve annulus in the axial direction of the interventional device. The positioning structures 410 and the anchor arms 500 on the outflow side 920 realize the engagement between two sides of the outer frame and the surrounding tissue, thereby improving the positioning stability of the outer frame 400. The specific structure and form of the positioning structure 410 may refer to the aforementioned embodiments.

The engagement between the inner frame 300 and the outer frame 400 can refer to the aforementioned embodiments. For example, as shown in FIG. 57a to FIG. 57d, the inner frame 300 and the outer frame 400 are formed in separate pieces and are fixedly connected to each other. The inner frame 300 and the outer frame 400 are provided with fixing ears 460 at the outflow side 920 that are fixedly connected to each other. The projection lengths of the fixing ears 460 in the axial direction are different.

In one embodiment, the anchor arm 500 has a single-strut structure or a multi-strut structure. Referring to FIGS. 57a to 64d, the anchor arm 500 has a multi-strut structure. The fixed end of the anchor arm 500 can be connected to the connection of adjacent cells, or the end of the cell close to the outflow side 920. In the compressed state, the anchor arm 500 contacts the radially outer side of the inner frame.

Here, the positioning structure is a V-shaped strut. The anchor arm 500 includes a plurality of struts in V shape. The two struts of the anchor arm 500 correspond to the two arms of the positioning structure 410 in the axial direction and are connected through the connecting struts 545. A fixing ear 460 is provided at the connection of the adjacent anchor arms 500 and the connecting strut 545, or the connection of the adjacent anchor arms 500 and the connecting strut 545 further extends toward the outflow side 920 to form a connection portion 320. Preferably, with reference to FIG. 60d, the connection portion 320 is further lengthened.

As shown in FIGS. 58a to 60d, the angle between the two struts of the anchor arm 500 increases accordingly. That is, the angle θ of the lower arm 542 increases, but is still within the abovementioned range. Therefore, the tip is more rounded, reducing the risk of scratching the surrounding tissue. In addition, the anchor arm 500 further extends radially and outward, that is, the radial size of the tip of the anchor arm 500 is further increased, so that the anchor arm can engage with the native leaflet better. For other configurations of the anchor arm 500, refer to the aforementioned embodiments.

In another embodiment, the connecting strut 545 is provided with an eyelet 548 adjacent the inflow side 910 for sewing the fabric.

In one embodiment, both the inner frame 300 and the outer frame 400 have mesh structures. The inner frame 300 and the outer frame 400 include one or more circles of cells in the axial direction, and the quantity of cells in each circle is in the range of 6 to 12. For details, reference may be made to the aforementioned embodiments. For example, in FIG. 57d, the inner frame 300 includes three circles of cells, and the quantity of cells in each circle is 12. The outer frame 400 includes a circle of cells, and the quantity of cells in the circle is 12.

As another example in FIG. 61a to FIG. 61d, the inner frame 300 includes three circles of cells, and the quantity of cells in each circle is 12. The outer frame 400 includes a circle of cells, and the quantity of cells in each circle is 6, so that the cells of the outer frame 400 have strong deformation ability. That is, due to the reduction in quantity of the cells, the distance between the struts of the cell is greater. Therefore, with the same thickness of the strut, the cell is easier to deform and adapt to the surrounding tissue.

Referring to FIGS. 57a to 57d, a circle of cells 440 on the outflow side 920 of the outer frame 400 has a semi-enclosed structure.

In combination with the above disclosure, the semi-enclosed structure consists of cells adjacent to the outflow side of the outer frame 400, each cell including two connecting struts 545 and a positioning structure 410. The two connecting struts 545 are separate from each other and do not cross each other.

Referring to FIGS. 60a to 60d, in other embodiments, the positioning structure 410 extends radially inward (referring to the first section 543 and the second section 544 mentioned in the aforementioned embodiment). As shown in FIG. 62a to FIG. 62d, the radially inwardly bending degree of the second section 544 decreases, that is, the angle µ as shown in FIG. 55 is reduced. For example, the angle µ is in the range of 0 degrees to 15 degrees. Therefore, the turning point 549 is curved smoothly. In this embodiment, part of the second sections 544 also has connecting ears 420 in T shape.

In other embodiments, as shown in FIGS. 63a to 63d, the struts of some cells of the inner frame 300 and the outer frame 400 on the inflow side further extend toward the inflow side 910 and form connecting ears 420. The connecting ear 420 exceeds other cells.

In combination with the above embodiments, an embodiment of the present disclosure further provides a prosthetic heart valve for atrioventricular valve, which can be implanted into the mitral valve or tricuspid valve to replace the diseased native valve. In the prosthetic heart valve, the inner frame 300 is configured to connect valve leaflets for controlling the opening and closing of the blood flow channel, while the outer frame 400 is configured to abut the inner edge of the native valve annulus and the inner side of the native valve leaflets when correctly implanted in the human body, and provide the necessary radial support.

Referring to FIGS. 64a to 64d, taking the stent shown in FIGS. 63a to 63d as an example, a prosthetic heart valve for atrioventricular valve is provided. In this embodiment, the inner frame 300 is provided with leaflets 610 for controlling the opening degree of the blood flow channel 301. The radial inner side of the inner frame 300 is sewn with a first covering film 630, the radial inner side of the outer frame 400 is sewn with a second covering film 640, and a sealing film 620 is arranged in the radial gap on the inflow side between the inner frame 300 and the outer frame 400 in a sealed connection.

On the inflow side 910, the inner frame 300 exceeds the sealing film 620 by a certain distance. In the case where the distance is short, the sealing film 620 can be connected and transitioned to the first covering film 630. The sealing film 620 and the first covering film 630 can be formed in one piece, or in separate pieces that are fixedly connected with each other.

The inflow side of the inner frame 300 is provided with connecting ears 420. The connecting ear 420 exceeds the first covering film 630 in the axial direction to facilitate connection with the interventional assembly. Similarly, the inflow side of the outer frame 400 is also provided with connecting ears 420 which exceed the sealing film 620. The sealing connection is provided so that blood cannot pass through the gap between the inner frame 300 and the outer frame 400. In this embodiment, the free end of the anchor arm 500 is sewn with a cushioning fabric 650 to avoid scratching the surrounding tissue.

One embodiment of the present disclosure discloses a loading method for an interventional device, for fixing the interventional device according to the above embodiment to the interventional assembly. The loading method includes:
arranging the interventional device in an expanded state around an outer periphery of the support tube of the interventional assembly;
compressing the interventional device radially around the outer periphery of the support tube; and
driving the inner core tube and outer sheath to move until the interventional device is covered.

The interventional device is the interventional device according to the aforementioned embodiment. The compression of the interventional device is performed by using an auxiliary tool to radially compress the outer frame 400. During the compression of the outer frame 400, the inner frame 300 is also compressed accordingly. The connecting ears 420 on the inner frame 300 and/or the outer frame 400 are engaged with the interventional assembly referring to the aforementioned embodiments.

The distal end of the outer sheath is provided with a diameter-increased portion serving as a first loading section, and the opening of the first loading section faces the distal end.

The distal end of the inner core tube is provided with a second loading section, and the opening of the second loading section faces the proximal end. When the inner core tube and the outer sheath are driven to move, they can either synchronously move in reverse directions, or move one after the other in any order, until the first loading section and the second loading section are connected to each other and surround the interventional device. The contact portions of the first loading section and the second loading section can axially abut against each other, or slightly overlap with each other in the radial direction.

An embodiment of the present disclosure discloses a release method for an interventional device. The interventional device according to the above embodiment is pre-fixed on the interventional assembly. The release method includes:
driving one of the inner core tube or the outer sheath to move so that at least part of the anchor arms is exposed and released;
adjusting the anchor arms to the proper position to engage with the surrounding tissue, such as extending to the outside of the native valve leaflets; and
driving the other of the inner core tube and the outer sheath to move, so that the interventional device is completely exposed and switches to the released state, that is, the inner frame 300 and the outer frame 400 are both released inside the native valve leaflets.

The interventional device may include in the axial direction:
a first part, in the compressed state, the axial position of the anchor arms correspond to the first part; and
a second part, i.e., the remaining part of the interventional device except the first part.

Depending on the axial position of the anchor arms, one of the first loading section 215 and the second loading section 216 is mainly used to radially restrain the anchor arms 500, i.e., the first part, which loading section is released first during the release process. That is, the anchor arms are released first to facilitate positioning in the human body.

The other of the first loading section 215 and the second loading section 216 is mainly used to radially restrain the second part, which loading section is released later during the release process. That is, after the anchor arms are positioned in place, the remaining part of the interventional device is completely released, thereby completing the deployment.

The release method further includes delivering the interventional device to a predetermined position, and during the delivery process, using a bending component to perform bending in two directions to adapt to the interventional path. The two directions each correspond to a plane in space, and the two planes are oblique or perpendicular to each other.

The release process will be explained in detail with reference to FIGS. 65 to 68, in which the annulus line 560 shown is used as a reference line for explaining the release position of the interventional device.

As shown in FIG. 65, the interventional device 900 in the compressed state is covered by the first loading section 215 and the second loading section 216 and is delivered into the human body until the first loading section 215 exceeds the annulus line 560. The interventional device 900 is then released. The release process of the interventional device 900 can be divided into two stages. In the first stage (as shown in FIG. 66), relative to the interventional device, the first loading section 215 moves axially to release the restraint on the anchor arms 500. Specifically, the anchor arms 500 switch from the state of contacting the radially outer side of the inner frame 300 to the state of extending outward, while the second loading section 216 still restrains at least part of the inner frame and the outer frame. Now the free ends of the anchor arms 500 extend outward. This state is a semi-released state to facilitate further adjustment of the spatial position of the interventional device.

In order to facilitate control when adjusting the anchor arms and avoid uncertainty caused by changes in the overall posture of the interventional assembly, preferably, the delivery tube 210 is kept in the bent state, and then withdrawn as a whole (as mentioned above) to adjust the anchor arms. Specifically, the transmission member 140 can move with the second support body 120 and slide in the axial direction relative to the first support body 110, so that the delivery tube 210 can move as a whole. Now, at least the bending component 220 remains stationary so that the distal end thereof is directed toward a certain direction. The distal end of the delivery tube 210 extending beyond the bending component 220 can move as a whole in this direction. For example, as shown in FIGS. 6 and 7, the interventional device 900 is retracted and the anchor arms 500 are positioned in place.

The interventional device is the interventional device according to the aforementioned embodiment. When the interventional device is partially exposed, the position of the exposed part relative to the surrounding tissue can be adjusted. If the release position is inappropriate, one of the inner core tube and the outer sheath is driven to the original position to recover the exposed part, the interventional device is disengaged from the surrounding tissue, the release position is readjusted and then the interventional device is released again. These operations can be repeated until the interventional device is positioned at the optimal release position.

After the interventional device is positioned at the optimal release position, in the second stage (as shown in FIG. 68), relative to the interventional device, the second loading section 216 is driven to move axially to release the restraint on the remaining part of the inner frame and outer frame, thereby completely releasing the interventional device. The interventional device is then fully expanded to the expanded state.

After the interventional device is released, the inner core tube is withdrawn so that the second loading section 216 is positioned against the chamfered structure at the distal end of the mounting head 214 for position correction so that the openings of the first loading section 215 and the second loading section 216 are aligned with each other (as shown in FIGS. 69 and 70). If the openings of the first loading section 215 and the second loading section 216 are misaligned as shown in FIG. 71, the withdrawal resistance will be too great, and even the edge of the second loading section 216 may scratch the surrounding tissue.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same drawing, it can be considered that the drawing also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. A control handle for operating an interventional assembly, the interventional assembly comprising a delivery tube for loading an interventional device and a bending component for changing an orientation of a distal end of the delivery tube, the control handle having an axial direction in space, two ends of the control handle in the axial direction being distal and proximal ends respectively, wherein the control handle comprises:
two support bodies arranged sequentially in the axial direction, the two support bodies comprising a first support body and a second support body located at a proximal end of the first support body, and the two support bodies being slidably engaged with each other in the axial direction;
a plurality of mounting seats installed on the corresponding support bodies fixedly or movably, wherein a mounting seat(s) on one support body is configured to connect the bending component, and a mounting seat(s) on the other support body is configured to connect the delivery tube; and
a plurality of driving members movably installed on the corresponding support bodies, which are engaged with and configured to drive the corresponding mounting seats on the support bodies.

2. The control handle for operating an interventional assembly of claim 1, wherein a third support body is movably connected to a distal end of the first support body, and the first support body and the third support body are movably connected to each other in a slide fit in the axial direction of the control handle or by a detachable connection.

3. The control handle for operating an interventional assembly of claim 2, wherein the mounting seats on the support bodies are configured as a fixed seat(s) and a movable seat(s) depending on movement relationships of the mounting seats relative to the support bodies, and all movable seats on the support bodies are respectively provided with corresponding driving members.

4. The control handle for operating an interventional assembly of claim 3, wherein the mounting seats on the support bodies comprise at least one fixed seat and at least one movable seat.

5. The control handle for operating an interventional assembly of claim 1, wherein the bending component comprises a tube(s) or a pull wire(s).

6. The control handle for operating an interventional assembly of claim 1, wherein one or more groups of bending components are provided, with each group consisting of two bending components, and wherein distal ends of the two bending components are relatively fixed, and proximal ends of the two bending components are respectively coupled with corresponding mounting seats and movable relative to each other.

7. The control handle for operating an interventional assembly of claim 6, wherein two groups of bending components are provided, with each group consisting of two bending components, and wherein the bending components in different groups are inserted one in the other, and distal ends thereof are movable relative to each other.

8. The control handle for operating an interventional assembly of claim 2, wherein two groups of bending components are provided, with one group connected to the first support body, and the other group connected to the third support body.

9. The control handle for operating an interventional assembly of claim 1, wherein the bending component comprises two bending tubes arranged sequentially from outside to inside, the mounting seats on the first support body comprises a fixed seat and a movable seat, and wherein distal ends of the two bending tubes are relatively fixed, and proximal ends thereof are respectively connected to the fixed seat and the movable seat.

10. The control handle for operating an interventional assembly of claim 2, wherein the bending component comprises a pull wire and three bending tubes arranged sequentially from outside to inside,
the mounting seats on the first support body comprises a fixed seat and a movable seat, wherein one of two inner bending tubes of the three bending tubes is connected to the fixed seat and the other is connected to the movable seat; and
the mounting seats on the third support body comprises a fixed seat and a movable seat, wherein an outermost bending tube of the three bending tubes is connected to the fixed seat, and the pull wire is connected to the movable seat.

11. The control handle for operating an interventional assembly of claim 1, wherein the bending component comprises four bending tubes arranged sequentially from outside to inside, with two outer bending tubes defined as one group and two inner bending tubes defined as another group, the mounting seats on the first support body comprise two fixed seats and two movable seats, and wherein one of the two bending tubes in the same group is connected to a corresponding fixed seat and the other is connected to a corresponding movable seat.

12. The control handle for operating an interventional assembly of claim 1, wherein the bending component comprises an outer bending tube, a middle bending tube and an inner bending tube arranged sequentially from outside to inside, which are relatively fixed adjacent to a distal portion of the bending component, and proximal ends of which are respectively coupled to the corresponding mounting seats and movable relative to each other;
the mounting seats on the first support body comprise a first fixed seat, a first movable seat and a second movable seat arranged from the distal end to the proximal end, which are respectively connected to the outer bending tube, the middle bending tube and the inner bending tube.

13. The control handle for operating an interventional assembly of claim 1, wherein the delivery tube comprises an outer sheath, a support tube and an inner core tube arranged sequentially from outside to inside, proximal ends of which are respectively coupled with the corresponding mounting seats and movable relative to each other;
the mounting seats on the second support body comprise a third movable seat, a second fixed seat and a fourth movable seat arranged from the distal end to the proximal end, which are respectively connected to the outer sheath, the support tube and the inner core tube.

14. The control handle for operating an interventional assembly of claim 13, wherein a transmission member is connected to the third movable seat, the transmission member extends distally until exceeding the first support body, the exceeding part is provided with a coupling seat, and a proximal end of the outer sheath is fixed to the coupling seat.

15. The control handle for operating an interventional assembly of claim 14, wherein a connection slot is opened on the transmission member, and at least a part of the third movable seat passes through the connection slot so that the transmission member is allowed to move with the second support body.

16. The control handle for operating an interventional assembly of claim 13, wherein a third support body is movably connected to a distal end of the first support body;
in the axial direction of the control handle, a coupling area is remained between the first support body and the third support body, a transmission member is connected to the third movable seat, the transmission member extends distally beyond the first support body until the coupling area, a distal end of the transmission member is provided with a coupling seat located in the coupling area, and a proximal end of the outer sheath is fixed to the coupling seat.

17. The control handle for operating an interventional assembly of claim 14, 15 or 16, wherein outer peripheries of the support bodies are fixed with respective housings, and the transmission member extends in a radial gap between the support bodies and the housings.

18. The control handle for operating an interventional assembly of claim 17, wherein the transmission member extends on two sides of the support bodies opposite to each other in a radial direction.

19. The control handle for operating an interventional assembly of claim 1, wherein a transmission member is connected to a mounting seat on the second support body, the transmission member extends distally until exceeding the first support body, and the exceeding part is provided with a coupling seat for connecting the delivery tube.

20. The control handle for operating an interventional assembly of claim 1, wherein the driving members are cylindrical and are rotatably arranged around outer peripheries of the corresponding support bodies, the driving members are threadedly engaged with the corresponding mounting seats, and the support bodies are provided with slide grooves for guiding the corresponding mounting seats to move linearly.

21. The control handle for operating an interventional assembly of claim 20, wherein at least parts of the mounting seats are exposed to the corresponding support bodies, the exposed parts are provided with male threads, and inner peripheries of the driving members are provided with female threads engaging with the corresponding male threads.

22. The control handle for operating an interventional assembly of claim 20, wherein a rack and gear transmission mechanism(s) is arranged between the driving members and the mounting seats.

23. The control handle for operating an interventional assembly of claim 1, wherein a connecting sleeve is rotatably mounted on the proximal end of the first support body, and a distal end of the second support body extends into the connecting sleeve and is threadedly engaged with the connecting sleeve.

24. The control handle for operating an interventional assembly of claim 23, wherein a distal periphery of the second support body is provided with transmission teeth, and the transmission teeth are threadedly engaged with a female thread of the connecting sleeve.

25. The control handle for operating an interventional assembly of claim 24, wherein a limiting mechanism is provided between the first support body and the second support body to prevent them from rotating relative to each other.

26. The control handle for operating an interventional assembly of claim 25, wherein the limiting mechanism is connected to one of the first support body and the second support body, and extends to and acts on the other.

27. The control handle for operating an interventional assembly of claim 26, wherein both the first support body and the second support body are slidably engaged with the limiting mechanism.

28. The control handle for operating an interventional assembly of claim 25, wherein the limiting mechanism is the transmission member, and the transmission member and the first support body are prevented from rotating relative to each other by:
a transmission groove being provided on the transmission member, the first support body being partially or entirely located in the transmission groove, and in a radial direction of the first support body, groove walls of the transmission groove clamping the first support body from two opposite sides;
the transmission member and the second support body are prevented from rotating relative to each other by:
the second support body being provided with a third movable seat, the transmission member being provided with a connection slot, at least a part of the third movable seat passing through the connection slot, and in a circumferential direction of the second support body, two opposite side groove walls of the connection slot abutting against the third movable seat.

29. The control handle for operating an interventional assembly of claim 1, further comprising a synchronization mechanism acting between two adjacent driving members, wherein the synchronization mechanism comprises:
first synchronization teeth on the driving members; and
a synchronization lock ring being movable in the axial direction of the control handle, the synchronization lock ring being provided with second synchronization teeth, and the synchronization lock ring having relative synchronization and release positions in the axial direction of the control handle;
wherein at the synchronization position, the second synchronization teeth mesh with the first synchronization teeth on two driving members to link the two driving members; and
at the release position, the second synchronizing teeth mesh with a first synchronizing tooth on at most one driving member to release the linkage.

30. The control handle for operating an interventional assembly of claim 29, wherein the first synchronization teeth are distributed on outer peripheries of the corresponding driving members.

31. The control handle for operating an interventional assembly of claim 30, wherein the synchronization lock ring is slidably arranged around one of the driving members, with a guide mechanism provided therebetween for guiding the synchronization lock ring to move in the axial direction of the control handle.

32. The control handle for operating an interventional assembly of claim 1, further comprising an anti-rotation mechanism, wherein the anti-rotation mechanism comprises:
a first latching tooth provided on a corresponding driving member; and
an anti-rotation lock ring being movable in the axial direction of the control handle, the anti-rotation lock ring being provided with second latching teeth, and the anti-rotation lock ring having relative locked and unlocked positions in the axial direction of the control handle;
wherein at the locked position, the first latching tooth is engaged with the second latching teeth to prevent the corresponding driving member from rotating; and
at the unlocked position, the first latching tooth is disengaged from the second latching teeth to allow the corresponding driving member to rotate.

33. The control handle for operating an interventional assembly of claim 32, wherein the first latching tooth is distributed on an inner periphery of the corresponding driving member.

34. The control handle for operating an interventional assembly of claim 33, wherein the anti-rotation lock ring is slidably arranged around a corresponding support body, with a guide mechanism provided therebetween for guiding the anti-rotation lock ring to move in the axial direction of the control handle.

35. The control handle for operating an interventional assembly of claim 1, wherein each mounting seat comprises a sealing member connected to a proximal end of the delivery tube or the bending component, and a housing that engages with a corresponding support body, and wherein the sealing member is disposed inside the housing.

36. The control handle for operating an interventional assembly of claim 35, wherein the housing comprises a cover plate and a hollow body, and an outer periphery of the hollow body is provided with a male thread that is engaged with a female thread of a corresponding driving member.

37. The control handle for operating an interventional assembly of claim 36, wherein a limiting mechanism is provided between the cover plate and the hollow body to prevent them from moving relative to each other, and the limiting mechanism comprises a positioning block provided on one of the cover plate and the hollow body and a positioning groove provided on the other and matching the positioning block.

38. The control handle for operating an interventional assembly of claim 37, wherein the cover plate is provided with a mounting base for installing the sealing member, the hollow body is provided with a mounting groove for accommodating the sealing member, and in an assembled state, the mounting base and the mounting groove clamp and fix the sealing member, and prevent the sealing member from moving in its own axial and radial directions.

39. The control handle for operating an interventional assembly of claim 38, wherein a middle part of the sealing member is provided with an annular step, the mounting base and the mounting groove have an annular protrusion that matches the annular step in the assembled state, and a side wall of the annular protrusion abuts against the sealing member.

40. The control handle for operating an interventional assembly of claim 35, wherein the sealing member comprises a connecting tube for connecting the delivery tube or the bending component, and sealing plugs respectively provided at two ends of the connecting tube for sealing.

41. A control handle for operating an interventional assembly, the interventional assembly being configured to control an interventional device, the control handle having an axial direction in space, two ends of the control handle in the axial direction being distal and proximal ends respectively, wherein in the axial direction, the control handle comprises a distal part, a main part and a proximal part arranged sequentially from the distal end to the proximal end;
components of the interventional assembly are divided into three groups which are respectively connected to and controlled by corresponding parts of the control handle; and
two adjacent parts of the control handle are configured to be coupled to each other through a locking mechanism, and to drive the connected components of the interventional assembly to move relative to each other after being decoupled.

42. A delivery system, comprising an interventional assembly and the control handle for operating the interventional assembly of any one of claims 1 to 41.

43. The delivery system of claim 42, wherein the interventional assembly comprises a plurality of tubes distributed sequentially from outside to inside, and wherein one of two tubes is an outer tube and the other is an inner tube, the outer tube is connected to the second support body, and the inner tube is connected to the first support body.

44. The delivery system of claim 42, wherein the interventional assembly comprises at least three tubes distributed sequentially from outside to inside, and wherein outermost and innermost tubes are both connected to the second support body, and at least one middle tube is connected to the first support body.

45. The delivery system of claim 42, wherein the interventional assembly comprises a plurality of tubes distributed sequentially from outside to inside, at least one tube of the delivery tube is located outside the bending component, and a proximal end of the delivery tube is located at a distal end of the first support body and is connected to the second support body after bypassing the first support body through a transmission member.

46. The delivery system of claim 42, wherein the interventional assembly comprises six tubes distributed sequentially from outside to inside: an outer sheath, an outer bending tube, a middle bending tube, an inner bending tube, a support tube and an inner core tube; the delivery tube comprises the outer sheath, the support tube and the inner core tube; the bending component comprises the outer bending tube, the middle bending tube and the inner bending tube; and wherein the bending component is connected to the first support body, and the delivery tube is connected to the second support body.

47. The delivery system of claim 42, wherein the interventional assembly comprises an outer sheath, a first bending tube, a second bending tube, a third bending tube, a fourth bending tube, a support tube and an inner core tube distributed sequentially from outside to inside; the delivery tube comprises the outer sheath, the support tube and the inner core tube; the bending component comprises the first bending tube, the second bending tube, the third bending tube and the fourth bending tube; and wherein the bending component is connected to the first support body, and the delivery tube is connected to the second support body.

48. The delivery system of claim 42, wherein the interventional assembly comprises an outer sheath, a first bending mechanism, a second bending mechanism, a support tube and an inner core tube distributed sequentially from outside to inside; the delivery tube comprises the outer sheath, the support tube and the inner core tube; the bending component comprises the bending mechanisms; and wherein the bending component is connected to the first support body, and the delivery tube is connected to the second support body.

49. The delivery system of claim 42, wherein a third support body is movably connected to a distal end of the first support body;
the interventional assembly comprises a third bending mechanism, an outer sheath, a fourth bending mechanism, a support tube and an inner core tube distributed sequentially from outside to inside; the delivery tube comprises the outer sheath, the support tube and the inner core tube; the bending component comprises the bending mechanisms; and wherein the third bending mechanism is connected to the third support body, the fourth bending mechanism is connected to the first support body, and the delivery tube is connected to the second support body.

50. The delivery system of claim 48 or 49, wherein each bending mechanism comprises:
two bending tubes inserted one in the other, or
a bending tube and a pull wire, the pull wire being located inside or outside the corresponding bending tube.

51. The delivery system of claim 50, wherein distal ends of the bending mechanisms are movable relative to each other, and distal ends of the bending mechanisms and the delivery tube are movable relative to each other.

52. The delivery system of claim 46, 47 or 48, wherein the support tube is provided with a mounting head for engaging with the interventional device, and the mounting head is provided with a chamfered structure at a distal edge thereof.

53. The delivery system of claim 49, wherein a distal end of the outer sheath is provided with a first loading section with an increased diameter;
a distal end of the inner core tube exceeds the distal end of the outer sheath, and the exceeding part is provided with a second loading section with an increased diameter; the first loading section and the second loading section are opened towards each other and contact with each other to form a loading space for accommodating the interventional device.

54. A prosthetic heart valve stent for atrioventricular valve, which has opposite inflow and outflow sides depending on a direction of blood flow, wherein the prosthetic heart valve stent comprises an inner frame and an outer frame located around the inner frame, the inner frame and the outer frame both have radially deformable and cylindrical mesh structures, and the inner frame encloses and defines a blood flow channel;
the inner frame or the outer frame is further connected with anchor arms, and an opening is formed by the anchor arms or between the anchor arms and the outer frame to accommodate a surrounding tissue for positioning and engaging with the surrounding tissue.

55. The prosthetic heart valve stent for atrioventricular valve of claim 54, wherein the opening is opened radially outward or toward the inflow side.

56. A prosthetic heart valve stent for atrioventricular valve, which has opposite inflow and outflow sides depending on a direction of blood flow, wherein the prosthetic heart valve stent comprises an inner frame and an outer frame located around the inner frame, the inner frame and the outer frame both have radially deformable and cylindrical mesh structures, and the inner frame encloses and defines a blood flow channel;
the prosthetic heart valve stent further comprises anchor arms for positioning and engaging with a surrounding tissue, each anchor arm has a fixed end connected to the inner frame or the outer frame and an opposing free end, the anchor arms are distributed in a circumference direction, all the anchor arms are connected to each other at the fixed ends thereof to form an annular structure as a whole and are connected to the inner frame or the outer frame through the annular structure.

57. A prosthetic heart valve stent for atrioventricular valve, comprising an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further comprises anchor arms, one end of each anchor arm is connected to the inner frame, the other end extends outward and exceeds the outer frame, and the exceeding part beyond the outer frame is bent toward the inflow side for positioning and engaging with a surrounding tissue.

58. The prosthetic heart valve stent for atrioventricular valve of claim 57, wherein each anchor arm extends outward and exceeds the outer frame by extending outward through a structural gap of the outer frame, and/or by extending outward from the outflow side of the outer frame and bypassing the outer frame.

59. The prosthetic heart valve stent for atrioventricular valve of claim 57, wherein the outflow side of the outer frame is provided with a plurality of semi-closed structural gaps which are opened toward the outflow side, and each anchor arm extends outward and exceeds the outer frame through a corresponding semi-closed structural gap.

60. A prosthetic heart valve stent for atrioventricular valve, comprising an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further comprises anchor arms which are arranged in pairs and are connected with the outer frame, the anchor arms in the same pair are arranged in an axial direction of the outer frame, and a clamping opening is defined between the anchor arms in the same pair that faces radially outward for positioning and engaging with a surrounding tissue.

61. The prosthetic heart valve stent for atrioventricular valve of claim 60, wherein one of the anchor arms in the same pair on the inflow side is an upper arm, the other on the outflow side is a lower arm, and the upper arm and the lower arm each have a single strut or multi-strut structure.

62. The prosthetic heart valve stent for atrioventricular valve of claim 61, wherein the inflow side of the outer frame has positioning structures extending radially outward, and each positioning structure also serves as the upper arm.

63. The prosthetic heart valve stent for atrioventricular valve of claim 61, wherein each anchor arm has a fixed end connected to the inner frame or the outer frame and an opposing free end, and the fixed ends of the upper arm and the lower arm are adjacent to or axially spaced apart from each other.

64. The prosthetic heart valve stent for atrioventricular valve of claim 60, wherein the clamping opening has an opening angle Σ at least in a range of 60 degrees < Σ < 120 degrees.

65. The prosthetic heart valve stent for atrioventricular valve of claim 60, wherein the clamping opening has an axial span M2 in a range of 10 < M2 < 30 mm.

66. A prosthetic heart valve stent for atrioventricular valve, comprising an outer frame and an inner frame surrounded by the outer frame, wherein the inner frame and the outer frame both have radially deformable and cylindrical mesh structures, the inner frame encloses and defines a blood flow channel, and the prosthetic heart valve stent has opposite inflow and outflow sides depending on a direction of blood flow; and wherein the prosthetic heart valve stent further comprises anchor arms, one end of each anchor arm is connected to the outflow side of the outer frame, and the other end extends outside the outer frame and toward the inflow side for positioning and engaging with a surrounding tissue.

67. A prosthetic heart valve stent for atrioventricular valve, having opposite inflow and outflow sides depending on a direction of blood flow, wherein the prosthetic heart valve stent comprises an inner frame and an outer frame located around the inner frame, the inner frame and the outer frame both have radially deformable and cylindrical mesh structures, and the inner frame and the outer frame are formed in separate pieces which are connected to each other at a connection portion(s), and the connection portion(s) is flexible.

68. The prosthetic heart valve stent for atrioventricular valve of claim 67, wherein the inner frame or the outer frame is further connected with anchor arms for positioning and engaging with a surrounding tissue.

69. A prosthetic heart valve stent for atrioventricular valve, having opposite inflow and outflow sides depending on a direction of blood flow, wherein the prosthetic heart valve stent comprises an inner frame and an outer frame located around the inner frame, the inner frame and the outer frame both have radially deformable and cylindrical mesh structures, the inner frame encloses and defines a blood flow channel, and the outer frame is configured to support an inner side of a native valve annulus and native valve leaflets; and
wherein a radial spacing is defined between the inner frame and the outer frame which are connected to each other through a fixing ear(s) spanning the radial spacing.

70. The prosthetic heart valve stent for atrioventricular valve of claim 69, wherein the inner frame or the outer frame is further connected with anchor arms for positioning and engaging with a surrounding tissue.

71. The prosthetic heart valve stent for atrioventricular valve of any one of claims 54 to 66, or claim 68 or claim 70, wherein the anchor arms are fixed to the inner frame or the outer frame by a binding wire(s).

72. The prosthetic heart valve stent for atrioventricular valve of any one of claims 54 to 66, or claim 68 or claim 70, wherein each anchor arm has an open structure or encloses a closed space.

73. The prosthetic heart valve stent for atrioventricular valve of any one of claims 54 to 66, or claim 68 or claim 70, wherein each anchor arm is provided with one or more eyelets.

74. The prosthetic heart valve stent for atrioventricular valve of claim 73, wherein at least one eyelet is provided at an end of each anchor arm.

75. The prosthetic heart valve stent for atrioventricular valve of claim 73, wherein a periphery of the eyelet has a closed or semi-open structure.

76. The prosthetic heart valve stent for atrioventricular valve of any one of claims 54 to 66, or claim 68 or claim 70, wherein each anchor arm has a single-strut structure.

77. The prosthetic heart valve stent for atrioventricular valve of any one of claims 54 to 66, or claim 68 or claim 70, wherein each anchor arm has fixed ends connected to the inner frame or the outer frame and an opposing free end, each anchor arm has a multi-strut structure which comprises at least two struts, and fixed ends of the struts diverge from each other and free ends thereof converge and fix each other.

78. The prosthetic heart valve stent for atrioventricular valve of any one of claims 54 to 66, or claim 68 or claim 70, wherein each anchor arm has a V shape and a vertex of the V shape is a free end.

79. The prosthetic heart valve stent for atrioventricular valve of any one of claims 54 to 66, or claim 68 or claim 70, wherein in a compressed state, the anchor arms are located on an axial side of the inner frame or the outer frame.

80. The prosthetic heart valve stent for atrioventricular valve of any one of claims 54 to 66, or claim 68 or claim 70, wherein during a switching process from a compressed state to a released state, a release timing of the inflow side and the outflow side is T, and a release timing of fixed ends and free ends of the anchor arms is T or -T.

81. The prosthetic heart valve stent for atrioventricular valve of any one of claims 54 to 66, or claim 68 or claim 70, wherein in a compressed state, the anchor arms contact a radial side of the inner frame or the outer frame.

82. The prosthetic heart valve stent for atrioventricular valve of any one of claims 54 to 70, wherein the inflow side of the outer frame has positioning structures extending radially outward.

83. The prosthetic heart valve stent for atrioventricular valve of claim 82, wherein an inflow edge of each positioning structure extends radially inward.

84. The prosthetic heart valve stent for atrioventricular valve of claim 82, wherein an inflow edge of each positioning structure has a connecting ear for engaging with an interventional assembly.

85. The prosthetic heart valve stent for atrioventricular valve of claim 84, wherein an angle between the connecting ear and an axis of the outer frame is α, and the angle α satisfies 0 degree ≤ α < 30 degrees.

86. The prosthetic heart valve stent for atrioventricular valve of claim 84, wherein the connecting ear has a length of L9, and L9 satisfies 3.5 mm < L9 < 6.5 mm.

87. The prosthetic heart valve stent for atrioventricular valve of claim 82, wherein the positioning structures comprise a plurality of V-shaped struts arranged in a circumferential direction of the outer frame, a vertex of each V-shaped strut faces the inflow side, and two arms of each V-shaped strut are connected to a remaining part of the outer frame.

88. The prosthetic heart valve stent for atrioventricular valve of claim 87, wherein the V-shaped struts has a quantity in a range of 6 to 12.

89. The prosthetic heart valve stent for atrioventricular valve of claim 87, wherein adjacent V-shaped struts are connected to each other in the circumferential direction of the outer frame.

90. The prosthetic heart valve stent for atrioventricular valve of claim 87, wherein in the circumferential direction of the outer frame, adjacent V-shaped struts are arranged at intervals, and wherein each interval has a circumferential width of M1 with a corresponding central angle of β, and the central angle β is in a range of 30 degrees to 60 degrees.

91. The prosthetic heart valve stent for atrioventricular valve of claim 90, wherein a sum of central angles of all M1 in the circumferential direction of the outer frame is 360 degrees.

92. The prosthetic heart valve stent for atrioventricular valve of claim 87, wherein in the circumferential direction of the outer frame, a release opening is defined between adjacent V-shaped struts for releasing connection therebetween, and wherein in a compressed state, the outer frame has a length of L1, the release opening has a length of L2, and L1 : L2 = X, wherein X is in a range of 2.0 to 3.0.

93. The prosthetic heart valve stent for atrioventricular valve of claim 87, wherein in the circumferential direction of the outer frame, a release opening is defined between adjacent V-shaped struts for releasing connection therebetween, and wherein in a released state, the outer frame has a length of L3, the release opening has a length of L4, and L3 : L4 = Y, wherein Y is in a range of 1.5 to 2.5.

94. The prosthetic heart valve stent for atrioventricular valve of any one of claims 54 to 70, wherein a connection portion(s) of the inner frame and the outer frame is located on the outflow side.

95. The prosthetic heart valve stent for atrioventricular valve of any one of claims 54 to 70, wherein the outer frame has a radial stiffness less than that of the inner frame.

96. The prosthetic heart valve stent for atrioventricular valve of any one of claims 54 to 70, wherein the inner frame and the outer frame are bound and fixed by a flexible element(s).

97. The prosthetic heart valve stent for atrioventricular valve of claim 96, wherein the inner frame and the outer frame are fixed with a binding wire(s).

98. The prosthetic heart valve stent for atrioventricular valve of any one of claims 54 to 70, wherein the inner frame and the outer frame are fixed to each other with a plurality of fixing ears distributed in a circumferential direction and formed by at least one of:
the fixing ears and the inner frame being formed in one piece and the fixing ears being bent radially outward until being connected to the outer frame;
the fixing ears and the outer frame being formed in one piece and the fixing ears being bent radially inward until being connected to the inner frame; and
the fixing ears being divided into two groups which are formed in separate pieces with the inner frame and the outer frame respectively and are bent radially toward each other and connected to each other.

99. The prosthetic heart valve stent for atrioventricular valve of claim 98, wherein each fixing ear is rod-shaped.

100. The prosthetic heart valve stent for atrioventricular valve of claim 99, wherein an end of each fixing ear is provided with a threading hole.

101. The prosthetic heart valve stent for atrioventricular valve of any one of claims 54 to 70, wherein the outflow side of the outer frame is radially flared.

102. The prosthetic heart valve stent for atrioventricular valve of any one of claims 54 to 70, wherein the outflow side of the outer frame is axially aligned with the outflow side of the inner frame.

103. An interventional device, comprising:
the prosthetic heart valve stent for atrioventricular valve of any one of claims 54 to 102;
leaflets configured in the inner frame for control an opening degree of a blood flow channel; and
a sealing film connected in a radial gap between the inner and outer frames.

104. The interventional device of claim 103, wherein the sealing film is located on the inflow side.

105. The interventional device of claim 104, wherein the sealing film is loosely connected in the radial gap between the inner frame and the outer frame.

106. The interventional device of claim 104, wherein the radial gap between the inner frame and the outer frame is open toward the outflow side.

107. The interventional device of claim 104, further comprising a first covering film connected to an inner side and/or an outer side of the inner frame.

108. The interventional device of claim 107, further comprising a second covering film connected to an inner side and/or an outer side of the outer frame.

109. The interventional device of claim 108, wherein at least one of the first covering film and the second covering film is connected to the sealing film.

110. A loading method for an interventional device, for fixing the interventional device of any one of claims 103 to 109 to an interventional assembly, the interventional assembly comprising an outer sheath, a support tube and an inner core tube arranged sequentially from outside to inside and slidably engaged with each other, the loading method comprising:
arranging the interventional device in an expanded state around an outer periphery of the support tube of the interventional assembly;
compressing the interventional device radially around the outer periphery of the support tube; and
driving the inner core tube and outer sheath to move until the interventional device is covered.

111. A release method for an interventional device, wherein the interventional device is the interventional device of any one of claims 103 to 109 and is pre-fixed on an interventional assembly, the interventional assembly comprises an outer sheath, a support tube and an inner core tube arranged sequentially from outside to inside and slidably engaged with each other, and the release method comprises:
driving one of the inner core tube or the outer sheath to move so that at least part of anchor arms of the interventional device is exposed and released;
adjusting the anchor arms to a proper position to engage with a surrounding tissue; and
driving the other of the inner core tube and the outer sheath to move, so that the interventional device is completely exposed and switches to a released state.
